# EUROPEAN PATENT APPLICATION

(11) **EP 3 858 439 A1**
(43) Date of publication of application: **04.08.2021**
(21) Application number: 20382068.3
(22) Date of filing: 03.02.2020
(51) Int. Cl.: A61P 35/04, A61K 31/4468, A61K 31/4523, C07D 205/06, C07D 211/58, C07D 401/06

(54) **AMIDE DERIVATIVES HAVING MULTIMODAL ACTIVITY AGAINST PAIN**

(71) Applicant: ESTEVE PHARMACEUTICALS, S.A., 08038 Barcelona (ES)
(72) Inventor: ALMANSA-ROSALES, Carmen, 08022 Barcelona (ES); VIRGILI-BERNADÓ, Marina, 08028 Barcelona (ES); ALONSO-XALMA, Mónica, 08028 Barcelona (ES); OSORIO-PLANES, Laura, 08028 Barcelona (ES)

(57) **Abstract**

The present invention relates to new compounds that show pharmacological activity towards the subunit α2δ of voltage-gated calcium channels (VGCC), especially the α2δ-1 subunit of voltage-gated calcium channels or dual activity towards the subunit α2δ of voltage-gated calcium channels (VGCC), especially the α2δ-1 subunit of voltage-gated calcium channels, and the µ-opiod receptor (MOR or mu-opioid). The invention is also related to the process for the preparation of said compounds as well as to compositions comprising them, and to their use as medicaments.

## Description

### FIELD OF THE INVENTION

The present invention relates to new compounds that show pharmacological activity towards the subunit α2δ of voltage-gated calcium channels (VGCC), especially the α2δ-1 subunit of voltage-gated calcium channels or dual activity towards the subunit α2δ of voltage-gated calcium channels (VGCC), especially the α2δ-1 subunit of voltage-gated calcium channels, and the µ-opiod receptor (MOR or mu-opioid receptor).
More particularly, the present invention relates to amide derivatives having this pharmacological activity, to processes of preparation of such compounds, to pharmaceutical compositions comprising them, and to their use in therapy, in particular for the treatment of pain and pain related conditions.

### BACKGROUND OF THE INVENTION

The adequate management of pain constitutes an important challenge, since currently available treatments provide in many cases only modest improvements, leaving many patients unrelieved (Turk, D.C., Wilson, H.D., Cahana, A.; 2011; Lancet; 377; 2226-2235). Pain affects a big portion of the population with an estimated prevalence of 20 % and its incidence, particularly in the case of chronic pain, is increasing due to the population ageing. Additionally, pain is clearly related to comorbidities, such as depression, anxiety and insomnia, which lead to important productivity losses and socio-economical burden (Goldberg, D.S., McGee, S.J.; 2011; BMC Public Health; 11; 770). Existing pain therapies include non-steroidal anti-inflammatory drugs (NSAIDs), opioid agonists, calcium channel blockers and antidepressants, but they are much less than optimal regarding their safety ratio. All of them show limited efficacy and a range of secondary effects that preclude their use, especially in chronic settings.

Voltage-gated calcium channels (VGCC) are required for many key functions in the body. Different subtypes of voltage-gated calcium channels have been described (Zamponi et al., Pharmacol. Rev. 2015 67:821-70). The VGCC are assembled through interactions of different subunits, namely α₁ (Caᵥα₁), β (Caᵥβ) α₂δ (Caᵥα₂δ) and γ (Caᵥγ). The α₁ subunits are the key porous forming units of the channel complex, being responsible for the Ca²⁺ conduction and generation of Ca²⁺ influx. The α₂δ, β, and γ subunits are auxiliary, although very important for the regulation of the channel, since they increase the expression of the α₁ subunits in the plasma membrane as well as modulate their function, resulting in functional diversity in different cell types. Based on their physiological and pharmacological properties, VGCC can be subdivided into low voltage-activated T-type (Caᵥ3.1, Caᵥ3.2, and Caᵥ3.3), and high voltage-activated L- (Caᵥ1.1 through Caᵥ1.4), N-(Caᵥ2.2), P/Q-(Caᵥ2.1), and R-(Caᵥ2.3) types, depending on the channel forming Cava subunits. All of these five subclasses are found in the central and peripheral nervous systems. Regulation of intracellular calcium through activation of these VGCC plays obligatory roles in: 1) neurotransmitter release, 2) membrane depolarization and hyperpolarization, 3) enzyme activation and inactivation, and 4) gene regulation (Perret and Luo, Neurotherapeutics. 2009 6:679-92; Zamponi *et al*., 2015 *supra*; Neumaier et al., Prog. Neurobiol. 2015 129:1-36.). A large body of data has clearly indicated that VGCC are implicated in mediating various disease states including pain processing. Drugs interacting with the different calcium channel subtypes and subunits have been developed. Current therapeutic agents include drugs targeting L-type Caᵥ1.2 calcium channels, particularly 1,4-dihydropyridines, which are widely used in the treatment of hypertension. T-type (Caᵥ3) channels are the target of ethosuximide, widely used in absence epilepsy. Ziconotide, a peptide blocker of N-type (Caᵥ2.2) calcium channels, has been approved as a treatment of intractable pain. (Perret and Luo, 2009, *supra*; Vink and Alewood, Br J Pharmacol. 2012 167:970-89.).

The Caᵥ1 and Caᵥ2 subfamilies contain an auxiliary α₂δ subunit, which is the therapeutic target of the gabapentinoid drugs of value in certain epilepsies and chronic neuropathic pain. To date, there are four known α₂δ subunits, each encoded by a unique gene and all possessing splice variants. Each α₂δ protein is encoded by a single messenger RNA and is posttranslationally cleaved and then linked by disulfide bonds. Four genes encoding α₂δ subunits have now been cloned. α₂δ-1 was initially cloned from skeletal muscle and shows a fairly ubiquitous distribution. The α₂δ-2 and α₂δ-3 subunits were subsequently cloned from brain. The most recently identified subunit, α₂δ-4, is largely nonneuronal. The human α₂δ-4 protein sequence shares 30, 32 and 61% identity with the human α₂δ-1, α₂δ-2 and α₂δ-3 subunits, respectively. The gene structure of all α₂δ subunits is similar. All α₂δ subunits show several splice variants (Davies et al., Trends Pharmacol Sci. 2007 28:220-8.; Dolphin AC, Nat Rev Neurosci. 2012 13:542-55., Biochim Biophys Acta. 2013 1828:1541-9.).

The Caᵥα₂δ-1 subunit may play an important role in neuropathic pain development (Perret and Luo, 2009, *supra;* Vink and Alewood, 2012, *supra).* Biochemical data have indicated a significant Caᵥα₂δ-1, but not Caᵥα₂δ-2, subunit upregulation in the spinal dorsal horn, and DRG (dorsal root ganglia) after nerve injury that correlates with neuropathic pain development. In addition, blocking axonal transport of injury-induced DRG Caᵥα₂δ-1 subunit to the central presynaptic terminals diminishes tactile allodynia in nerve injured animals, suggesting that elevated DRG Caᵥα₂δ-1 subunit contributes to neuropathic allodynia.

The Caᵥα₂δ-1 subunit (and the Caᵥα₂δ-2, but not Caᵥα₂δ-3 and Caᵥα₂δ-4, subunits) is the binding site for gabapentin which has anti-allodynic/ hyperalgesic properties in patients and animal models. Because injury-induced Caᵥα₂δ-1 expression correlates with neuropathic pain development and maintenance, and various calcium channels are known to contribute to spinal synaptic neurotransmission and DRG neuron excitability, injury-induced Caᵥα₂α-1 subunit upregulation may contribute to the initiation and maintenance of neuropathic pain by altering the properties and/or distribution of VGCC in the subpopulation of DRG neurons and their central terminals, therefore modulating excitability and/or synaptic neuroplasticity in the dorsal horn. Intrathecal antisense oligonucleotides against the Caᵥα₂δ-1 subunit can block nerve injury-induced Caᵥα₂δ-1 upregulation and prevent the onset of allodynia and reserve established allodynia.

As mentioned above, the α₂δ subunits of VGCC form the binding site for gabapentin and pregabalin, which are structural derivatives of the inhibitory neurotransmitter GABA although they do not bind to GABAA, GABAB, or benzodiazepine receptors, or alter GABA regulation in animal brain preparations. The binding of gabapentin and pregabalin to the Caᵥα₂δ subunit results in a reduction in the calcium-dependent release of multiple neurotransmitters, leading to efficacy and tolerability for neuropathic pain management. Gabapentinoids may also reduce excitability by inhibiting synaptogenesis (Perret and Luo, 2009, *supra;* Vink and Alewood, 2012, *supra,* Zamponi *et al.,* 2015, *supra).*

Thus, the present invention relates to compounds with inhibitory effect towards α2δ subunits of voltage-gated calcium channels, preferably towards the α2δ-1 subunit of voltage-gated calcium channels.

As mentioned before, there are few available therapeutic classes for the treatment of pain, and opioids are among the most effective, especially when addressing severe pain states. They act through three different types of opioid receptors (mu, kappa and gamma) which are transmembrane G-protein coupled receptors (GPCRs). Still, the main analgesic action is attributed to the activation of the µ-opioid receptor (MOR). However, the general administration of MOR agonists is limited due to their important side effects, such as constipation, respiratory depression, tolerance, emesis and physical dependence [Meldrum, M.L. (Ed.). Opioids and Pain Relief: A Historical Perspective. Progress in Pain Research and Management, Vol 25. IASP Press, Seattle, 2003]. Additionally, MOR agonists are not optimal for the treatment of chronic pain as indicated by the diminished effectiveness of morphine against chronic pain conditions. This is especially proven for the chronic pain conditions of neuropathic or inflammatory origin, in comparison to its high potency against acute pain. The finding that chronic pain can lead to MOR down-regulation may offer a molecular basis for the relative lack of efficacy of morphine in long-term treatment settings [Dickenson, A.H., Suzuki, R. Opioids in neuropathic pain: Clues from animal studies. Eur J Pain 9, 113-6 (2005)]. Moreover, prolonged treatment with morphine may result in tolerance to its analgesic effects, most likely due to treatment-induced MOR down-regulation, internalization and other regulatory mechanisms. As a consequence, long-term treatment can result in substantial increases in dosing in order to maintain a clinically satisfactory pain relief, but the narrow therapeutic window of MOR agonists finally results in unacceptable side effects and poor patient compliance.

Polypharmacology is a phenomenon in which a drug binds multiple rather than a single target with significant affinity. The effect of polypharmacology on therapy can be positive (effective therapy) and/or negative (side effects). Positive and/or negative effects can be caused by binding to the same or different subsets of targets; binding to some targets may have no effect. Multi-component drugs or multi-targeting drugs can overcome toxicity and other side effects associated with high doses of single drugs by countering biological compensation, allowing reduced dosage of each compound or accessing context-specific multitarget mechanisms. Because multitarget mechanisms require their targets to be available for coordinated action, one would expect synergies to occur in a narrower range of cellular phenotypes given differential expression of the drug targets than would the activities of single agents. In fact, it has been experimentally demonstrated that synergistic drug combinations are generally more specific to particular cellular contexts than are single agent activities, such selectivity is achieved through differential expression of the drugs' targets in cell types associated with therapeutic, but not toxic, effects (Lehar et al., Nat Biotechnol 2009; 27: 659-666).

In the case of chronic pain, which is a multifactorial disease, multi-targeting drugs may produce concerted pharmacological intervention of multiple targets and signaling pathways that drive pain. Because they actually make use of biological complexity, multi-targeting (or multi-component drugs) approaches are among the most promising avenues toward treating multifactorial diseases such as pain (Gilron et al., Lancet Neurol. 2013 Nov;12(11):1084-95.). In fact, positive synergistic interaction for several compounds, including analgesics, has been described (Schroder et al., J Pharmacol Exp Ther. 2011; 337:312-20. Erratum in: J Pharmacol Exp Ther. 2012; 342:232; Zhang et al., Cell Death Dis. 2014; 5:e1138.; Gilron *et al.,* 2013, *supra).*

Given the significant differences in pharmacokinetics, metabolisms and bioavailability, reformulation of drug combinations (multi-component drugs) is challenging. Further, two drugs that are generally safe when dosed individually cannot be assumed to be safe in combination. In addition to the possibility of adverse drug-drug interactions, if the theory of network pharmacology indicates that an effect on phenotype may derive from hitting multiple targets, then that combined phenotypic perturbation may be efficacious or deleterious. The major challenge to both drug combination strategies is the regulatory requirement for each individual drug to be shown to be safe as an individual agent and in combination (Hopkins, Nat Chem Biol. 2008; 4:682-90.).

An alternative strategy for multitarget therapy is to design a single compound with selective polypharmacology (multi-targeting drug). It has been shown that many approved drugs act on multiple targets. Dosing with a single compound may have advantages over a drug combination in terms of equitable pharmacokinetics and biodistribution. Indeed, troughs in drug exposure due to incompatible pharmacokinetics between components of a combination therapy may create a low-dose window of opportunity where a reduced selection pressure can lead to drug resistance. In terms of drug registration, approval of a single compound acting on multiple targets faces significantly lower regulatory barriers than approval of a combination of new drugs (Hopkins, 2008, *supra).*

Pain is multimodal in nature, since in nearly all pain states several mediators, signaling pathways and molecular mechanisms are implicated. Consequently, monomodal therapies fail to provide complete pain relief. Currently, combining existing therapies is a common clinical practice and many efforts are directed to assess the best combination of available drugs in clinical studies (Mao, J., Gold, M.S., Backonja, M.; 2011; J. Pain; 12; 157-166).

Thus, there is still a need to find compounds that have an alternative or improved pharmacological activity in the treatment of pain, being both effective and showing the desired selectivity, and having good "drugability" properties, i.e. good pharmaceutical properties related to administration, distribution, metabolism and excretion.

The present application relates to the advantages of having dual activity, for µ-receptor and the α₂δ-1 subunit of voltage-gated calcium channels, in the same molecule to treat chronic pain.

The present invention relates to compounds having a complementary dual mechanism of action (µ-receptor agonist and blocker of the α₂δ subunit, in particular the α₂δ-1 subunit, of voltage-gated calcium channels) which implies a better profile of tolerability than the strong opioids (morphine, oxycodone, fentanyl etc) and/or better efficacy and tolerability than gabapentinoids (pregabalin and gabapentin).

The compounds of the present invention show a primary pharmacological activity towards the α₂δ subunit, in particular the α₂δ-1 subunit, of the voltage-gated calcium channel, and compounds that show dual pharmacological activity towards both the α₂δ subunit, in particular the α₂δ-1 subunit, of the voltage-gated calcium channel and the µ-opioid receptor resulting in an innovative, effective, complementary and alternative solution for the treatment of pain.

In view of the existing results of the currently available therapies and clinical practices, the present invention offers a solution by developing compounds binding to a single target or by combining in a single compound binding to two different targets relevant for the treatment of pain. This was mainly achieved by providing the compounds according to the invention that bind to the α₂δ subunit, in particular the α₂δ-1 subunit, of the voltage-gated calcium channel, or both to the the µ-opioid receptor and to the α₂δ subunit, in particular the α₂δ-1 subunit, of the voltage-gated calcium channel.

### SUMMARY OF THE INVENTION

The present invention discloses novel compounds with pharmacological activity to the α2δ subunit of voltage-gated calcium channels, more specifically to the α2δ-1 subunit, and which in preferred embodiments, have also affinity towards the µ-opioid receptor, thus resulting in a dual activity for treating pain and pain related disorders.

The main aspect of the present invention is related to compounds of general formula (I):

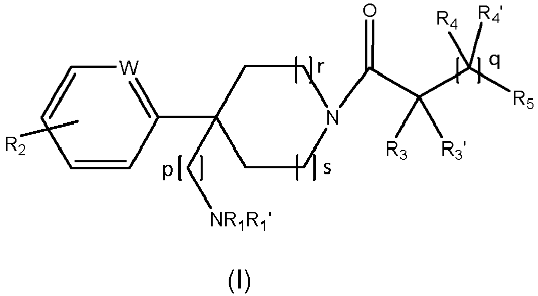

A further aspect of the invention refers to the processes for preparation of compounds of formula (I).

A still further aspect of the invention refers to the use of intermediate compounds for the preparation of a compound of formula (I).

It is also an aspect of the invention a pharmaceutical composition comprising a compound of formula (I).

Finally, it is an aspect of the invention a compound of formula (I) for use in therapy and more particularly for the treatment of pain and pain related conditions.

### DETAILED DESCRIPTION OF THE INVENTION

The invention is directed to a family of compounds, in particular, to amide derivatives which show primary pharmacological activity towards the α₂δ subunit, in particular the α₂δ-1 subunit, of the voltage-gated calcium channel. The invention also refers to compounds having a dual pharmacological activity towards both the α₂δ subunit, in particular the α₂δ-1 subunit, of the voltage-gated calcium channel and the µ-opioid receptor and to their use in the treatment of pain and related disorders.

The applicant has surprisingly found that the problem of providing a new effective and alternative solution for treating pain and pain related disorders can be solved by using an analgesic approach using compounds binding to the α₂δ subunit, in particular the α₂δ-1 subunit, of the voltage-gated calcium channel or a multimodal analgesic approach combining two activities in a single drug (i.e., dual ligands which are bifunctional and bind to µ-opioid receptor and to α₂δ subunit, in particular the α₂δ-1 subunit, of the voltage-gated calcium channel).

As described above, the µ-opioid receptor as well as the α2δ1 subunit modulate intracellular calcium concentration and the activity of voltage-dependent calcium channels. There is also a robust clinical and pre-clinical evidence linking both targets with the treatment of chronic neuropathic pain. Thus, the present invention, also relates to the advantages of having dual activity, for the α₂δ-1 subunit of voltage-gated calcium channels and the µ-opioid receptor, in the same molecule to treat pain. This supports the therapeutic value of a dual agent, whereby the α2δ binding component acts as an intrinsic adjuvant of the MOR binding component.

A dual compound that possesses binding to both the µ-opioid receptor and to the α2δ subunit of the voltage-gated calcium channel shows a highly valuable therapeutic potential by achieving an outstanding analgesia (enhanced in respect to the potency of the opioid component alone) with a reduced side-effect profile (safety margin increased compared to that of the opioid component alone) versus existing opioid therapies.

Advantageously, the dual compounds according to the present invention would in addition show one or more the following functionalities: blockade of the α2δ subunit, in particular the α2δ-1 subunit, of the voltage-gated calcium channel and µ-opioid receptor agonism.

A further advantage of using designed multiple ligands is a lower risk of drug-drug interactions compared to cocktails or multi-component drugs, thus involving simpler pharmacokinetics and less variability among patients. Additionally, this approach may improve patient compliance and broaden the therapeutic application in relation to monomechanistic drugs, by addressing more complex aetiologies.

In a first aspect, the present invention is directed to a compound of formula (I): 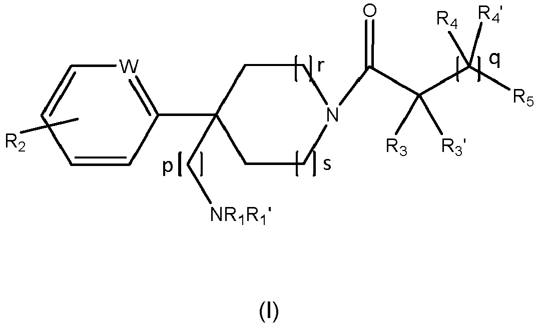 wherein:
W is N or CH;
R₁ and R₁' are independently selected from the group consisting of hydrogen, substituted or unsubstituted C₁₋₆ alkyl, substituted or unsubstituted C₂₋₆alkenyl, and substituted or unsubstituted C₂₋₆ alkynyl;
R₂ is absent or is selected from the group consisting of halogen, substituted or unsubstituted C₁₋₆ alkyl, substituted or unsubstituted C₂₋₆ alkenyl, substituted or unsubstituted C₂₋₆ alkynyl, OR₂', -SR₂', -SOR₂', -SO₂R₂', -CN, -COR₂', -COOR₂',-NR₂'R₂", -CONR₂'R₂" and haloalkoxy;
   wherein R₂' and R₂" are independently selected from the group consisting of hydrogen and substituted or unsubstituted C₁₋₆ alkyl;
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and (CR₆R₆')ₐ-NR₇R₇'; wherein one of R₃ or R₄ is (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
R₅ is selected from the group consisting of substituted or unsubstituted aryl, substituted or unsubstituted heterocyclyl and substituted or unsubstituted C₄₋₁₀-cycloalkyl;
p is 0, 1 or 2;
q is 0, 1 or 2;
r is 0, 1 or 2; and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The compounds of the invention represented by the above described formula (I) may include enantiomers depending on the presence of chiral centres or isomers depending on the presence of multiple bonds. The single isomers, enantiomers or diastereoisomers and mixtures thereof fall within the scope of the present invention.

Thus, in another embodiment, these compounds according to the invention are optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt or solvate thereof.

For the sake of clarity, the expression "a compound according to formula (I), wherein R₁, R₁', R₂, R₃, R₃', R₄, R₄', R₅, W, p, q, r and s are as defined herein in the detailed description" would (just like the expression "a compound of formula (I) as defined in any one of claims 1 to 8 found in the claims) refer to "a compound according to formula (I)", wherein the definitions of the respective substituents R₁ etc. (also from the cited claims) are applied.

For clarity purposes, all groups and definitions described in the present description and referring to compounds of formula (I), also apply to all intermediates of synthesis.

In the context of this invention, alkyl is understood as meaning a straight or branched hydrocarbon chain radical containing no unsaturation, and which is attached to the rest of the molecule by a single bond. It may be unsubstituted or substituted once or several times. It encompasses e.g. -CH₃ and -CH₂-CH₃. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl and C₁₋₆-alkyl represents C1-, C2-, C3-, C4-, C5- or C6-alkyl. Examples of alkyl radicals include among others methyl, ethyl, propyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl. Preferably alkyl is understood in the context of this invention C₁₋₆-alkyl like methyl, ethyl, propyl, butyl, pentyl, or hexyl; and more preferably is C₁₋₃-alkyl like methyl, ethyl and propyl.

Alkenyl is understood as meaning straight or branched hydrocarbon chain radical containing at least two carbon atoms and at least one unsaturation, and which is attached to the rest of the molecule by a single bond. It may be unsubstituted or substituted once or several times. It encompasses groups like e.g. -CH=CH-CH₃. The alkenyl radicals are preferably vinyl (ethenyl), allyl (2-propenyl). Preferably in the context of this invention alkenyl is C₂₋₆-alkenyl like ethylene, propylene, butylene, pentylene, or hexylene; or is C₂₋₄-alkenyl, like ethylene, propylene, or butylenes.

Alkynyl is understood as meaning a straight or branched hydrocarbon chain radical containing at least two carbon atoms and at least one carbon-carbon triple bond, and which is attached to the rest of the molecule by a single bond. It may be unsubstituted or substituted once or several times. It encompasses groups like e.g. -C≡C-CH₃ (1-propynyl). Preferably alkynyl in the context of this invention is C₂₋₆-alkynyl like ethyne, propyne, butyene, pentyne, or hexyne; or is C₂₋₄-alkynyl like ethyne, propyne or butyene.

In connection with alkyl (also in alkyl-heterocyclyl, alkyl-cycloalkyl or alkyl-aryl), alkenyl, alkynyl and O-alkyl - unless defined otherwise - the term substituted in the context of this invention is understood as meaning replacement of at least one hydrogen radical on a carbon atom by halogen, -OR', -SR', -SOR', -SO₂R', -OR', -CN, -COR', -COOR', -NR'R", -CONR'R", haloalkyl, haloalkoxy or -OC₁₋₆ alkyl wherein each of the R' and R" groups is independently selected from the group consisting of hydrogen, OH, NO₂, NH₂, SH, CN, halogen, -COH, -COalkyl, -COOH and C₁₋₆ alkyl.

More particularly, the alkyl, alkenyl or alkynyl as defined in R₁-R₇' if substituted, is substituted with one or more substituent/s selected from -OR', halogen, -CN, haloalkyl, haloalkoxy and -NR'R"; wherein R' and R" are independently selected from hydrogen, unsubstituted C₁₋₆ alkyl, unsubstituted C₂₋₆ alkenyl, and unsubstituted C₂₋₆ alkynyl.

More than one replacement on the same molecule and also on the same carbon atom is possible with the same or different substituents. This includes for example 3 hydrogens being replaced on the same C atom, as in the case of CF₃, or at different places of the same molecule, as in the case of e.g. -CH(OH)-CH=CH-CHCl₂.

In the context of this invention haloalkyl is understood as meaning an alkyl being substituted once or several times by a halogen (selected from F, CI, Br, I). It encompasses e.g. -CH₂Cl, -CH₂F, -CHCl₂, -CHF₂, -CCl₃, -CF₃ and -CH₂-CHCl₂. Preferably haloalkyl is understood in the context of this invention as halogen-substituted C₁₋₄-alkyl representing halogen substituted C1-, C2-, C3- or C4-alkyl. The halogen-substituted alkyl radicals are thus preferably methyl, ethyl, propyl, and butyl. Preferred examples include -CH₂Cl, -CH₂F, -CH₂-CH₂F, -CH₂-CHF₂, -CHCl₂, -CHF₂, and -CF₃.

In the context of this invention haloalkoxy is understood as meaning an -O-alkyl being substituted once or several times by a halogen (selected from F, CI, Br, I). It encompasses e.g. -OCH₂Cl, -OCH₂F, -OCHCl₂, -OCHF₂, -OCCl₃, -OCF₃ and -OCH₂-CHCl₂. Preferably haloalkoxy is understood in the context of this invention as halogen-substituted -OC₁₋₄-alkyl representing halogen substituted C1-, C2-, C3- or C4-alkoxy. The halogen-substituted O-alkyl radicals are thus preferably O-methyl, O-ethyl, O-propyl, and O-butyl. Preferred examples include -OCH₂Cl, -OCH₂F, -OCHCl₂, -OCHF₂, and-OCF₃.

In the context of this invention cycloalkyl is understood as meaning saturated and unsaturated (but not aromatic) cyclic hydrocarbons (without a heteroatom in the ring), which can be unsubstituted or once or several times substituted. Preferred cycloalkyls are C₃₋₄-cycloalkyl representing C3- or C4-cycloalkyl, C₃₋₅-cycloalkyl representing C3-, C4- or C5-cycloalkyl, C₃₋₆-cycloalkyl representing C3-, C4-, C5- or C6-cycloalkyl, C₃₋₇-cycloalkyl representing C3-, C4-, C5-, C6- or C7-cycloalkyl, C₃₋₈-cycloalkyl representing C3-, C4-, C5-, C6-, C7- or C8-cycloalkyl, C₄₋₅-cycloalkyl representing C4- or C5-cycloalkyl, C₄₋₆-cycloalkyl representing C4-, C5- or C6-cycloalkyl, C₄₋₇-cycloalkyl representing C4-, C5-, C6- or C7-cycloalkyl, C₅₋₆-cycloalkyl representing C5- or C6-cycloalkyl and C₅₋₇-cycloalkyl representing C5-, C6- or C7-cycloalkyl. Examples are cyclopropyl, 2-methylcyclopropyl, cyclopropyl methyl, cyclobutyl, cyclopentyl, cyclopentylmethyl, cyclohexyl, cycloheptyl, cyclooctyl, and also adamantyl. Preferably in the context of this invention cycloalkyl is C₃₋₈-cycloalkyl like cyclopropyl, cyclobutyl, cyclopentyl, cyclohexyl, cycloheptyl, or cyclooctyl; or is C₃₋₇-cycloalkyl like cyclopropyl, cyclobutyl, cyclopentyl, cyclohexyl, or cycloheptyl; or is C₃₋₆-cycloalkyl like cyclopropyl, cyclobutyl, cyclopentyl or cyclohexyl, especially cyclopentyl or cyclohexyl.

Aryl is understood as meaning 6 to 18 membered mono or polycyclic ring systems with at least one aromatic ring but without heteroatoms even in only one of the rings. Examples are phenyl, naphthyl, fluoranthenyl, fluorenyl, tetralinyl or indanyl, 9*H*-fluorenyl or anthracenyl radicals, which can be unsubstituted or once or several times substituted. Most preferably aryl is understood in the context of this invention as phenyl, naphthyl or anthracenyl, more preferably the aryl is phenyl.

A heterocyclyl radical or group (also called heterocyclyl hereinafter) is understood as meaning 5 to 18 membered mono or polycyclic heterocyclic ring systems, with at least one saturated or unsaturated ring which contains one or more heteroatoms selected from the group consisting of nitrogen, oxygen and/or sulfur in the ring. A heterocyclic group can also be substituted once or several times.

Examples include non-aromatic heterocyclyls such as tetrahydropyran, oxazepane, morpholine, piperidine, pyrrolidine as well as heteroaryls such as furan, benzofuran, thiophene, benzothiophene, pyrrole, pyridine, pyrimidine, pyrazine, quinoline, isoquinoline, phthalazine, thiazole, benzothiazole, indole, benzotriazole, carbazole and quinazoline.

Subgroups inside the heterocyclyls as understood herein include heteroaryls and non-aromatic heterocyclyls.
- the heteroaryl (being equivalent to heteroaromatic radicals or aromatic heterocyclyls) is an aromatic 5 to 18 membered mono or polycyclic heterocyclic ring system of one or more rings of which at least one aromatic ring contains one or more heteroatoms from the group consisting of nitrogen, oxygen and/or sulfur in the ring; preferably is a 5 to 18 membered mono or polycyclic aromatic heterocyclic ring system of one or two rings of which at least one aromatic ring contains one or more heteroatoms selected from the group consisting of nitrogen, oxygen and/or sulfur in the ring, more preferably it is selected from furan, benzofuran, thiophene, benzothiophene, pyrrole, pyridine, pyrimidine, pyrazine, quinoline, isoquinoline, phthalazine, benzothiazole, indole, benzotriazole, carbazole, quinazoline, thiazole, imidazole, pyrazole, oxazole, thiophene and benzimidazole;
- the non-aromatic heterocyclyl is a 5 to 18 membered mono or polycyclic heterocyclic ring system of one or more rings of which at least one ring - with this (or these) ring(s) then not being aromatic - contains one or more heteroatoms from the group consisting of nitrogen, oxygen and/or sulfur in the ring; preferably it is a 5 to 18 membered mono or polycyclic heterocyclic ring system of one or two rings of which one or both rings - with this one or two rings then not being aromatic - contain/s one or more heteroatoms selected from the group consisting of nitrogen, oxygen and/or sulfur in the ring, more preferably it is selected from oxazepane, pyrrolidine, piperidine, piperazine, tetrahydropyran, morpholine, indoline, oxopyrrolidine, benzodioxane, especially is piperazine, benzodioxane, morpholine, tetrahydropyran, piperidine, oxopyrrolidine and pyrrolidine.

Preferably, in the context of this invention heterocyclyl is defined as a 5 to 18 membered mono or polycyclic ring system of one or more saturated or unsaturated rings of which at least one ring contains one or more heteroatoms selected from the group consisting of nitrogen, oxygen and/or sulfur in the ring. Preferably it is a 5 to 18 membered mono or polycyclic heterocyclic ring system of one or two saturated or unsaturated rings of which at least one ring contains one or more heteroatoms selected from the group consisting of nitrogen, oxygen and/or sulfur in the ring. More preferably, it is a 5 to 10 membered mono or bicyclic heterocyclyl ring system containing one nitrogen atom and optionally a second heteroatom selected from nitrogen and oxygen. In another preferred embodiment of the invention, said heterocyclyl is a substituted mono or bicyclic heterocyclyl ring system.

Preferred examples of heterocyclyls include oxazepane, pyrrolidine, imidazole, oxadiazole, tetrazole, pyridine, pyrimidine, piperidine, piperazine, benzofuran, benzimidazole, indazole, benzodiazole, thiazole, benzothiazole, tetrahydropyran, morpholine, indoline, furan, triazole, isoxazole, pyrazole, thiophene, benzothiophene, pyrrole, pyrazine, pyrrolo[2,3b]pyridine, quinoline, isoquinoline, tetrahydroisoquinoline, phthalazine, benzo-1,2,5-thiadiazole, indole, benzotriazole, benzoxazole, oxopyrrolidine, pyrimidine, benzodioxolane, benzodioxane, carbazole and quinazoline, especially is pyridine, piperazine, pyrazine, indazole, benzodioxane, thiazole, benzothiazole, morpholine, tetrahydropyran, pyrazole, imidazole, piperidine, thiophene, indole, benzimidazole, pyrrolo[2,3b]pyridine, benzoxazole, oxopyrrolidine, pyrimidine, oxazepane and pyrrolidine. In the context of this invention oxopyrrolidine is understood as meaning pyrrolidin-2-one.

In connection with aromatic heterocyclyls (heteroaryls), non-aromatic heterocyclyls, aryls and cycloalkyls, when a ring system falls within two or more of the above cycle definitions simultaneously, then the ring system is defined first as an aromatic heterocyclyl (heteroaryl) if at least one aromatic ring contains a heteroatom. If no aromatic ring contains a heteroatom, then the ring system is defined as a non-aromatic heterocyclyl if at least one non-aromatic ring contains a heteroatom. If no non-aromatic ring contains a heteroatom, then the ring system is defined as an aryl if it contains at least one aryl cycle. If no aryl is present, then the ring system is defined as a cycloalkyl if at least one non-aromatic cyclic hydrocarbon is present.

In the context of this invention alkyl-aryl or aryl-alkyl is understood as meaning an aryl group (see above) being connected to another atom through a C₁₋₆-alkyl (see above) which may be branched or linear and is unsubstituted or substituted once or several times as described before. Preferably aryl-alkyl is understood as meaning an aryl group (see above) being connected to another atom through 1 to 4 (-CH₂-) groups.

In the context of this invention alkyl-heterocyclyl is understood as meaning an heterocyclyl group (see above) being connected to another atom through a C₁₋₆-alkyl (see above) which may be branched or linear and is unsubstituted or substituted once or several times as described before. Preferably alkyl-heterocyclyl is understood as meaning an heterocyclyl group (see above) being connected to another atom through 1 to 4 (-CH₂-) groups.

In the context of this invention alkyl-cycloalkyl is understood as meaning a cycloalkyl group (see above) being connected to another atom through a C₁₋₆-alkyl (see above) which may be branched or linear and is unsubstituted or substituted once or several times as described before. Preferably alkylcycloalkyl is understood as meaning a cycloalkyl group (see above) being connected to another atom through 1 to 4 (-CH₂-) groups.

Preferably, the aryl is a monocyclic aryl. More preferably the aryl is a 6 or 7 membered monocyclic aryl. Even more preferably the aryl is a 6 membered monocyclic aryl, preferably phenyl.

Preferably, the heteroaryl is a monocyclic heteroaryl. More preferably the heteroaryl is a 5, 6 or 7 membered monocyclic heteroaryl. Even more preferably the heteroaryl is a 5 or 6 membered monocyclic heteroaryl.

Preferably, the non-aromatic heterocyclyl is a monocyclic non-aromatic heterocyclyl. More preferably the non-aromatic heterocyclyl is a 4, 5, 6 or 7 membered monocyclic non-aromatic heterocyclyl. Even more preferably the non-aromatic heterocyclyl is a 5 or 6 membered monocyclic non-aromatic heterocyclyl. In another preferred embodiment, said non-aromatic heterocyclyl is a bicyclic non-aromatic heterocyclyl.

Preferably, the cycloalkyl is a monocyclic cycloalkyl. More preferably the cycloalkyl is a 3, 4, 5, 6, 7 or 8 membered monocyclic cycloalkyl. Even more preferably the cycloalkyl is a 3, 4, 5 or 6 membered monocyclic cycloalkyl.

A ring system is a system consisting of at least one ring of connected atoms but including also systems in which two or more rings of connected atoms are joined with "joined" meaning that the respective rings are sharing one (like a spiro structure), two or more atoms being a member or members of both joined rings.

The term "leaving group" means a molecular fragment that departs with a pair of electrons in heterolytic bond cleavage. Leaving groups can be anions or neutral molecules. Common anionic leaving groups are halides such as Cl-, Br-, and I-, and sulfonate esters, such as tosylate (TsO-), mesylate, nosylate or triflate.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes via ionic interactions. The definition particularly includes physiologically acceptable salts, this term must be understood as equivalent to "pharmacologically acceptable salts".

The term "physiologically acceptable salt" means in the context of this invention any salt that is physiologically tolerated (most of the time meaning not being toxic- especially lacking toxicity caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts.

Physiologically acceptable salts can also be formed with anions or acids and in the context of this invention is understood as meaning salts of at least one of the compounds used according to the invention as the cation with at least one anion which are physiologically tolerated - especially if used on humans and/or mammals. By this it is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be present in crystalline form or in the form of free compounds like a free base or acid.

Any compound that is a solvate of a compound according to the invention like a compound according to formula (I) defined above is understood to be also covered by the scope of the invention. Methods of solvation are generally known within the art. Suitable solvates are pharmaceutically acceptable solvates. The term "solvate" according to this invention is to be understood as meaning any form of the active compound according to the invention in which this compound has attached to it via noncovalent binding another molecule (most likely a polar solvent). Especially preferred examples include hydrates and alcoholates, like methanolates or ethanolates.

Any compound that is a prodrug of a compound according to the invention like a compound according to formula (I) defined above is understood to be also covered by the scope of the invention. The term "prodrug" is used in its broadest sense and encompasses those derivatives that are converted *in vivo* to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Any compound that is a *N*-oxide of a compound according to the invention like a compound according to formula (I) defined above is understood to be also covered by the scope of the invention.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or of a nitrogen by ¹⁵N-enriched nitrogen are within the scope of this invention.

The compounds of formula (I) as well as their salts or solvates are preferably in pharmaceutically acceptable or substantially pure form. By pharmaceutically acceptable pure form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts. This applies also to its solvates or prodrugs.

All the groups above mentioned that can be substituted or unsubstituted may be substituted - unless defined otherwise - at one or more available positions by one or more suitable groups such as OR', =O, SR', SOR', SO₂R', OSO₂R', OSO₃R', NO₂, NHR', N(R')₂, =N-R', N(R')COR', N(COR')₂, N(R')SO₂R', N(R')C(=NR')N(R')R', N₃, CN, halogen, COR', COOR', OCOR', OCOOR', OCONHR', OCON(R')₂, CONHR', CON(R')₂, CON(R')OR', CON(R')SO₂R', PO(OR')₂, PO(OR')R', PO(OR')(N(R')R'), C₁₋₁₂ alkyl, C₃₋₁₀ cycloalkyl, C₂₋₁₂ alkenyl, C₂₋₁₂ alkynyl, aryl, and heterocyclic group, wherein each of the R' groups is independently selected from the group consisting of hydrogen, OH, NO₂, NH₂, SH, CN, halogen, COH, COalkyl, COOH, C₁₋₁₂ alkyl, C₃₋₁₀cycloalkyl, C₂₋₁₂ alkenyl, C₂₋₁₂ alkynyl, aryl and heterocyclic group. Where such groups are themselves substituted, the substituents may be chosen from the foregoing list.

In a particular embodiment of the invention, the compound of formula (I) according to the invention is a compound wherein W is CH;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.
In a particular embodiment of the invention, the compound of formula (I) according to the invention is a compound wherein W is N;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₁ and R₁' are independently selected from the group consisting of hydrogen and substituted or unsubstituted C₁₋₆ alkyl, preferably unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₁ and R₁' are independently selected from the group consisting of hydrogen and unsubstituted C₁₋₆ alkyl, preferably, unsubstituted C₁₋₃ alkyl, more preferably methyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₂ is absent or is selected from the group consisting of halogen, substituted or unsubstituted C₁₋₆ alkyl, OR₂', -SR₂', -SOR₂',-SO₂R₂', -CN, -COR₂', -COOR₂', -NR₂'R₂", -CONR₂'R₂" and haloalkoxy;
wherein R₂' and R₂" are independently selected from the group consisting of hydrogen and substituted or unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₂ is absent or is selected from the group consisting of halogen, substituted or unsubstituted C₁₋₆ alkyl, and OR₂';
wherein R₂' is selected from the group consisting of hydrogen and substituted or unsubstituted C₁₋₆ alkyl, preferably unsubstituted C₁₋₆ alkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a more particular embodiment of the invention, the compound of formula (I) according to the invention is a compound wherein R₂ is absent or is selected from the group consisting of halogen and OR₂';
wherein R₂' is selected from the group consisting of hydrogen and unsubstituted C₁₋₆ alkyl, preferably unsubstituted C₁₋₆ alkyl, more preferably methyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₃, R₃', R₄ and R₄' are independently selected from the group consisting of hydrogen, substituted or unsubstituted C₁₋₆ alkyl, and (CR₆R₆')ₐ-NR₇R₇', wherein one of R₃ or R₄ is (CR₆R₆')ₐ-NR₇R₇';
wherein
R₆ and R₆' are independently selected from the group consisting of hydrogen, substituted or unsubstituted C₁₋₆ alkyl; and
R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₃, R₃', R₄ and R₄' are independently selected from the group consisting of hydrogen, substituted or unsubstituted C₁₋₆ alkyl, preferably unsubstituted C₁₋₆ alkyl, and (CR₆R₆')ₐ-NR₇R₇', wherein one of R₃ or R₄ is (CR₆R₆')ₐ-NR₇R₇';
wherein
R₆ and R₆' are independently selected from the group consisting of hydrogen and unsubstituted C₁₋₆ alkyl; and
R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₃ is (CR₆R₆')ₐ-NR₇R₇' and R₃', R₄ or R₇ forms a cycle with R₅;
wherein
R₆ and R₆' are independently selected from the group consisting of hydrogen and unsubstituted C₁₋₆ alkyl; and
R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₃ is (CR₆R₆')ₐ-NR₇R₇' and R₃' forms a cycle with R₅;
wherein
R₆ and R₆' are independently selected from the group consisting of hydrogen and unsubstituted C₁₋₆ alkyl; and
R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₃ is (CR₆R₆')ₐ-NR₇R₇' and R₇ forms a cycle with R₅;
wherein
R₆ and R₆' are independently selected from the group consisting of hydrogen and unsubstituted C₁₋₆ alkyl; and
R₇ is unsubstituted C₁₋₆ alkyl and R₇' is selected from the group consisting of hydrogen and substituted or unsubstituted C₁₋₆ alkyl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₃ is (CR₆R₆')ₐ-NR₇R₇' and R₄ forms a cycle with R₅;
wherein
R₆ and R₆' are independently selected from the group consisting of hydrogen and unsubstituted C₁₋₆ alkyl; and
R₇ and R₇' are independently selected from the group consisting of hydrogen, substituted or unsubstituted C₁₋₆ alkyl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a more particular embodiment of the invention, the compound of formula (I) according to the invention is a compound wherein R₃ is (CR₆R₆')ₐ-NR₇R₇' and R₇ forms a cycle with R₄;
wherein
R₆ and R₆' are independently selected from the group consisting of hydrogen and unsubstituted C₁₋₆ alkyl; and
R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a more particular embodiment of the invention, the compound of formula (I) according to the invention is a compound wherein R₃ is (CR₆R₆')ₐ-NR₇R₇' and R₇ forms a cycle with R₄;
wherein
R₆ and R₆' are independently selected from the group consisting of hydrogen and unsubstituted C₁₋₆ alkyl; and
R₇ and R₇' are independently selected from the group consisting of hydrogen, unsubstituted C₁₋₆ alkyl, unsubstituted aryl, and unsubstituted C₁₋₆ alkyl-aryl, preferably C₁₋₃ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a more particular embodiment of the invention, the compound of formula (I) according to the invention is a compound wherein R₃ is (CR₆R₆')ₐ-NR₇R₇' and R₆ forms a cycle with R₄, wherein said cycle is a substituted cycloalkyl being substituted by -NR₇R₇';
wherein
R₆ and R₆' are independently selected from the group consisting of hydrogen and unsubstituted C₁₋₆ alkyl; and
R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl; and
a is 0, 1 or 2;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment of the invention, the compound of formula (I) according to the invention is a compound wherein R₅ is selected from the group consisting of substituted or unsubstituted aryl, substituted or unsubstituted heterocyclyl and substituted or unsubstituted C₄₋₁₀-cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a more particular embodiment of the invention, the compound of formula (I) according to the invention is a compound wherein R₅ is selected from the group consisting of substituted or unsubstituted aryl, preferably a 6 or 7 membered monocyclic aryl, more preferably phenyl, substituted or unsubstituted heterocyclyl and substituted or unsubstituted C₄₋₁₀-cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In an even more particular embodiment of the invention, the compound of formula (I) according to the invention is a compound wherein R₅ is selected from the group consisting of substituted or unsubstituted aryl, preferably a 6 or 7 membered monocyclic aryl, more preferably phenyl, substituted or unsubstituted heterocyclyl and substituted or unsubstituted C₄₋₁₀-cycloalkyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In the context of the invention, when the aryl, heterocyclyl or substituted or C₄₋₁₀-cycloalkyl in R₅ is substituted, the substituents are selected from the group consisting of halogen, unsubstituted C₁₋₆ alkyl, -OR', -SR', -SOR', -SO₂R', -CN, -COR', -COOR',-NR'R", -CONR'R", haloalkyl, and haloalkoxy, wherein each of the R' and R" groups is independently selected from the group consisting of hydrogen, OH and unsubstituted C₁₋₆ alkyl.

In a particular embodiment of the invention, when the aryl in R₅ is substituted, the substituents are selected from the group consisting of halogen, unsubstituted C₁₋₆ alkyl, -OR', -CN, -COR', and -COOR', wherein R' is selected from the group consisting of hydrogen, OH and unsubstituted C₁₋₆ alkyl, preferably methyl.

For the sake of clarity, in the context of the present invention, it should be understood that naturally, if q is 0, R₅ is still present in general Markush Formula (I). In this regard, it should also be understood, that only the groups R₄ and, R₄' are included into the brackets and thus, q means the number of times that R₄ and R₄' are repeated.

Also, it should be understood that each R₄ and R₄' are independently selected from the group consisting of hydrogen, halogen, substituted or unsubstituted C₁₋₆ alkyl, substituted or unsubstituted C₂₋₆ alkenyl, and substituted or unsubstituted C₂₋₆ alkynyl in each repetition, i.e. R₄ and/or R₄' might still be equal of different in each repetition.

In a particular embodiment of the invention, the compound according to formula (I) is a compound wherein q is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a particular embodiment of the invention, the compound according to formula (I) is a compound wherein p is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a particular embodiment of the invention, the compound according to formula (I) is a compound wherein r is 0, 1 or 2; preferably, r is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a particular embodiment of the invention, the compound according to formula (I) is a compound wherein s is 0, 1 or 2; preferably, s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention, the compound according to formula (I) is a compound, wherein in R₁, R₁', R₂, R₃, R₃', R₄ and R₄' as defined in any of the embodiments of the present invention,
the C₁₋₆ alkyl is preferably selected from methyl, ethyl, propyl, butyl, pentyl, hexyl, isopropyl, and 2-methylpropyl;
and/or
the C₂₋₆ -alkenyl is preferably selected from ethylene, propylene, butylene, pentylene, hexylene, isopropylene, and isobutylene;
and/or
the C₂₋₆ -alkynyl is preferably selected from ethyne, propyne, butyne, pentyne, hexyne, isopropyne, and isobutyne;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the invention according to formula (I) the compound is a compound, wherein in R₅ as defined in any of the embodiments of the present invention,
the cycloalkyl is C₄₋₁₀ cycloalkyl like for example cyclobutyl, cyclopentyl, cyclohexyl, cycloheptyl or cyclooctyl;
and/or
the heterocyclyl is a heterocyclic ring system of one or more saturated or unsaturated rings of which at least one ring contains one or more heteroatoms from the group consisting of nitrogen, oxygen and/or sulfur in the ring; preferably is a heterocyclic ring system of one or two saturated or unsaturated rings of which at least one ring contains one or more heteroatoms from the group consisting of nitrogen, oxygen and/or sulfur in the ring, more preferably is selected from oxazepan, pyrrolidine, imidazole, oxadiazole, tetrazole, pyridine, pyrimidine, piperidine, piperazine, benzofuran, benzimidazole, indazole, benzothiazole, benzodiazole, thiazole, benzothiazole, tetrahydropyrane, morpholine, indoline, furan, triazole, isoxazole, pyrazole, thiophene, benzothiophene, pyrrole, pyrazine, pyrrolo[2,3b]pyridine, quinoline, isoquinoline, phthalazine, benzo-1,2,5-thiadiazole, indole, benzotriazole, benzoxazole oxopyrrolidine, pyrimidine, benzodioxolane, benzodioxane, carbazole, and quinazoline; more preferably is piperazine;
and/or
the aryl is selected from phenyl, naphtyl, or anthracenyl; preferably is phenyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 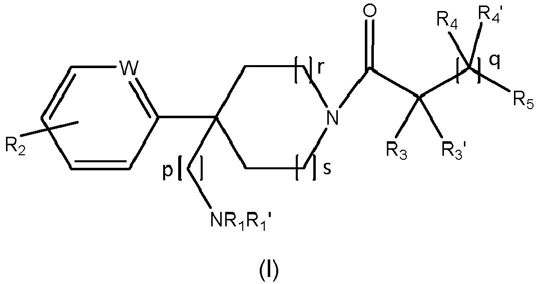 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl, substituted or unsubstituted C₂₋₆ alkenyl, substituted or unsubstituted C₂₋₆ alkynyl, OR₂', -SR₂', -SOR₂', -SO₂R₂', -CN, -COR₂', -COOR₂',-NR₂'R₂", -CONR₂'R₂" and haloalkoxy;
   wherein R₂' and R₂" are independently selected from the group consisting of hydrogen, substituted or unsubstituted C₁₋₆ alkyl;
and/or
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted (CR₆R₆')ₐ-NR₇R₇'; wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆alkenyl, and substituted or unsubstituted C₂₋₆ alkynyl; and
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
and/or
R₅ is selected from the group consisting of substituted or unsubstituted aryl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 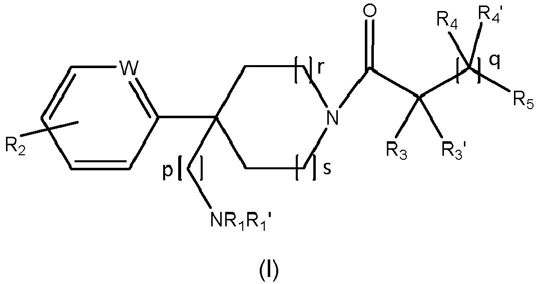 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl, OR₂', -SR₂', -SOR₂', -SO₂R₂', -CN, -COR₂', -COOR₂',-NR₂'R₂", -CONR₂'R₂" and haloalkoxy;
   wherein R₂' and R₂" are independently selected from the group consisting of hydrogen and substituted or unsubstituted C₁₋₆ alkyl;
and/or
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted (CR₆R₆')ₐ-NR₇R₇'; wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆alkenyl, and substituted or unsubstituted C₂₋₆alkynyl; and
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
and/or
R₅ is selected from the group consisting of substituted or unsubstituted aryl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 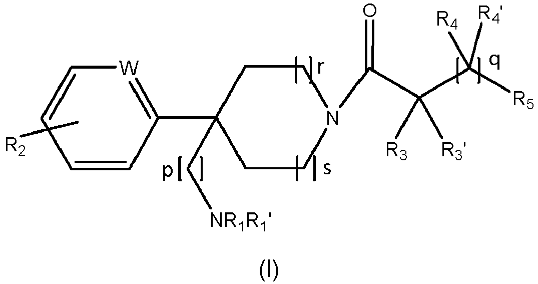 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted (CR₆R₆')ₐ-NR₇R₇'; wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆alkenyl, and substituted or unsubstituted C₂₋₆ alkynyl; and
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
and/or
R₅ is selected from the group consisting of substituted or unsubstituted aryl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 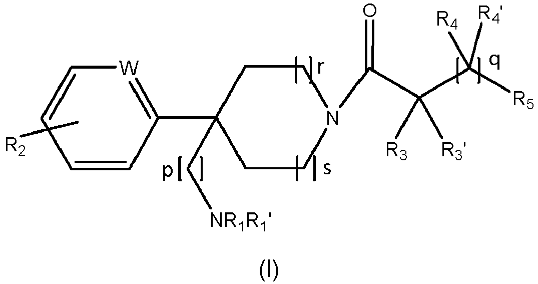 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃, R₃', R₄ and R₄' are independently selected from the group consisting of hydrogen, substituted or unsubstituted C₁₋₆ alkyl, and substituted or unsubstituted (CR₆R₆')ₐ-NR₇R₇', wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and/or
R₅ is selected from the group consisting of substituted or unsubstituted aryl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 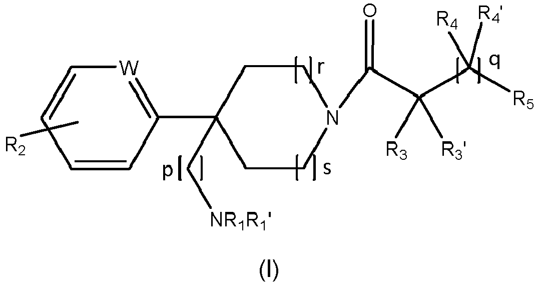 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃, R₃', R₄ and R₄' are independently selected from the group consisting of hydrogen, substituted or unsubstituted C₁₋₆ alkyl, preferably unsubstituted C₁₋₆ alkyl, and substituted or unsubstituted (CR₆R₆')ₐ-NR₇R₇', wherein one of R₃ or R₄ is (CR₆R₆')ₐ-NR₇R₇';
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and/or
R₅ is selected from the group consisting of substituted or unsubstituted aryl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 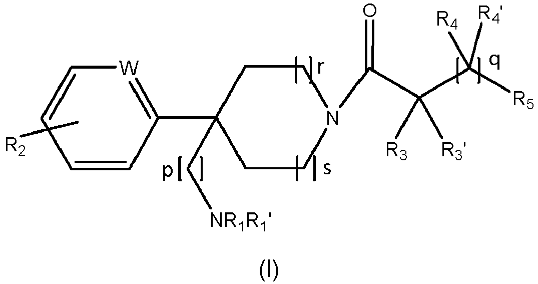 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and
   R₃', R₄ or R₇ forms a cycle with R₅; or
   R₇ forms a cycle with R₄; or
   R₆ forms a cycle with R₄, wherein said cycle is a substituted cycloalkyl being substituted by -NR₇R₇';
   wherein
      R₆ and R₆' are independently selected from the group consisting of hydrogen and unsubstituted C₁₋₆ alkyl; and
      R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
      a is 0, 1 or 2;
and/or
R₅ is selected from the group consisting of substituted or unsubstituted aryl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 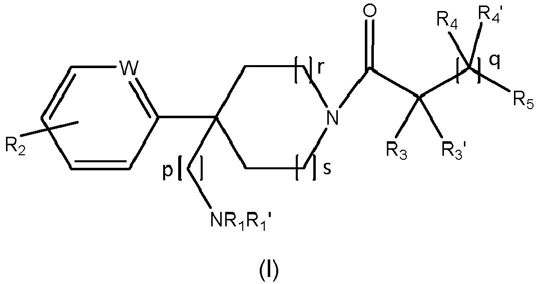 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and
   R₃', R₄ or R₇ forms a cycle with R₅; or
   R₇ forms a cycle with R₄; or
   R₆ forms a cycle with R₄, wherein said cycle is a substituted cycloalkyl being substituted by -NR₇R₇';
   wherein
      R₆ and R₆' are independently selected from the group consisting of hydrogen and unsubstituted C₁₋₆ alkyl; and
      R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
      a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0, 1 or 2;
and/or
q is 0, 1 or 2;
and/or
r is 0, 1 or 2;
and/or
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 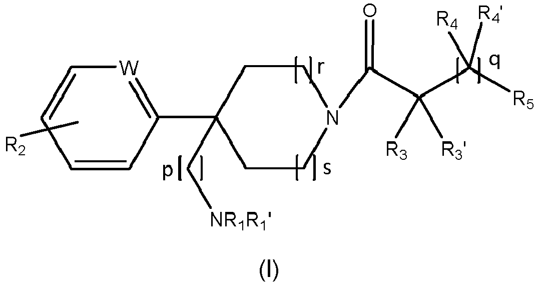 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and
   R₃', R₄ or R₇ forms a cycle with R₅; or
   R₇ forms a cycle with R₄; or
   R₆ forms a cycle with R₄, wherein said cycle is a substituted cycloalkyl being substituted by -NR₇R₇';
   wherein
      R₆ and R₆' are independently selected from the group consisting of hydrogen and unsubstituted C₁₋₆ alkyl; and
      R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
      a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 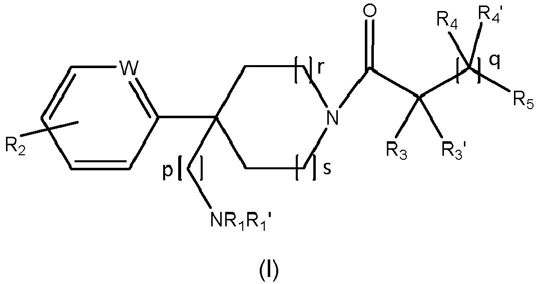 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and R₃', R₄ or R₇ forms a cycle with R₅;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 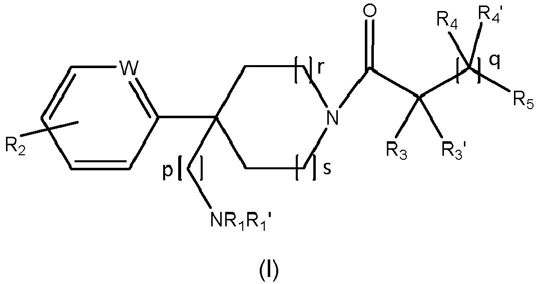 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and R₃' forms a cycle with R₅;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 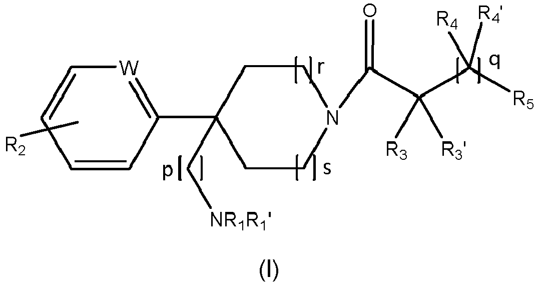 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and R₇ forms a cycle with R₅;
   wherein
   R₆ and R₆' are independently selected from the group consisting of hydrogen and unsubstituted C₁₋₆ alkyl; and
   R₇ is unsubstituted C₁₋₆ alkyl and R₇' is selected from the group consisting of hydrogen and substituted or unsubstituted C₁₋₆ alkyl; and
   a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 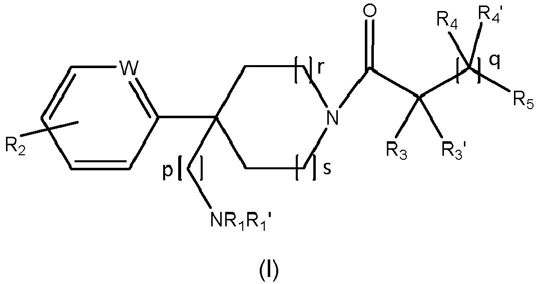 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and R₄ forms a cycle with R₅;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl; and
   a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 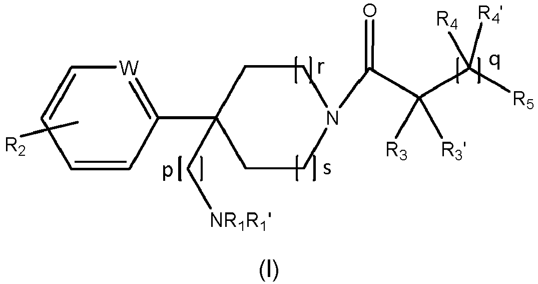 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and R₇ forms a cycle with R₄;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl, preferably C₁₋₃ alkyl-aryl; and
   a is 0, 1 or 2;
and/or
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 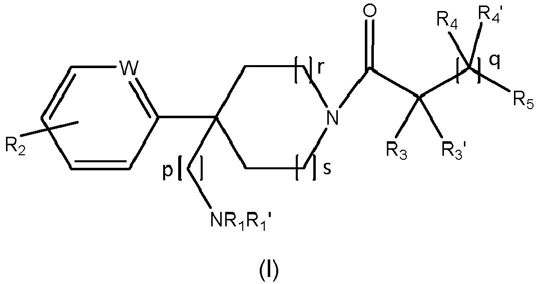 wherein
W is N or CH;
and/or
R₁ and R₁' are independently selected from the group consisting of hydrogen and substituted or unsubstituted C₁₋₆ alkyl, preferably unsubstituted C₁₋₆ alkyl, more preferably, methyl;
and/or
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and/or
R₃ is (CR₆R₆')ₐ-NR₇R₇' and R₆ forms a cycle with R₄, wherein said cycle is a substituted cycloalkyl being substituted by -NR₇R₇';
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl; and a is 0, 1 or 2;
and/or
R₅ is selected from the group consisting of substituted or unsubstituted aryl, preferably a monocyclic aryl, preferably phenyl, substituted or unsubstituted heterocyclyl and substituted or unsubstituted C₄₋₁₀-cycloalkyl;
and/or
p is 0 or 1;
and/or
q is 0 or 1;
and/or
r is 0 or 1;
and/or
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 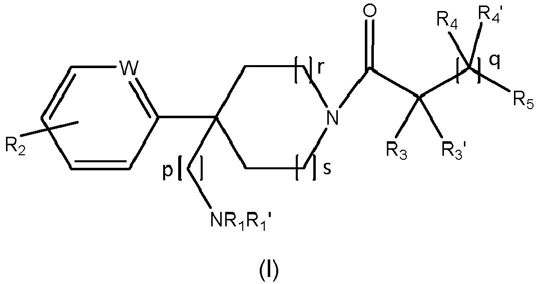 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl, substituted or unsubstituted C₂₋₆ alkenyl, substituted or unsubstituted C₂₋₆alkynyl, OR₂', -SR₂', -SOR₂', -SO₂R₂', -CN, -COR₂', -COOR₂',-NR₂'R₂", -CONR₂'R₂" and haloalkoxy;
   wherein R₂' and R₂" are independently selected from the group consisting of hydrogen, substituted or unsubstituted C₁₋₆ alkyl;
and
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted (CR₆R₆')ₐ-NR₇R₇'; wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆alkenyl, and substituted or unsubstituted C₂₋₆ alkynyl; and
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
and
R₅ is selected from the group consisting of substituted or unsubstituted aryl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 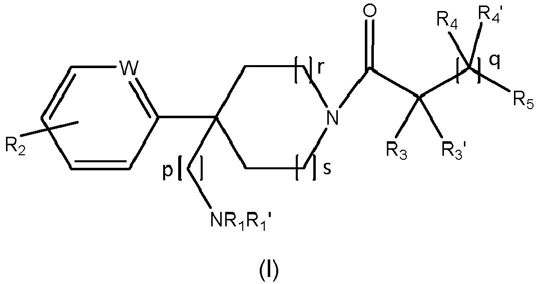 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl, OR₂', -SR₂', -SOR₂', -SO₂R₂', -CN, -COR₂', -COOR₂',-NR₂'R₂", -CONR₂'R₂" and haloalkoxy;
   wherein R₂'and R₂" are independently selected from the group consisting of hydrogen and substituted or unsubstituted C₁₋₆ alkyl;
and
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted (CR₆R₆')ₐ-NR₇R₇'; wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆alkenyl, and substituted or unsubstituted C₂₋₆alkynyl; and
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
and
R₅ is selected from the group consisting of substituted or unsubstituted aryl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 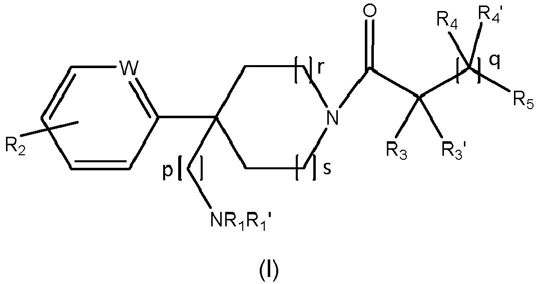 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and substituted or unsubstituted (CR₆R₆')ₐ-NR₇R₇'; wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl; and
   R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkyl-aryl; and
   a is 0, 1 or 2;
   alternatively,
      R₃', R₄ or R₇ forms a cycle with R₅; or
      R₇ forms a cycle with R₄; or
      R₆ forms a cycle with R₄, wherein said cycle is a substituted cycloalkyl being substituted by -NR₇R₇';
and
R₅ is selected from the group consisting of substituted or unsubstituted aryl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 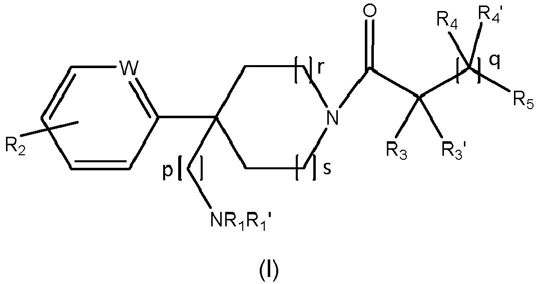 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃, R₃', R₄ and R₄' are independently selected from the group consisting of hydrogen, substituted or unsubstituted C₁₋₆ alkyl, and substituted or unsubstituted (CR₆R₆')ₐ-NR₇R₇', wherein one of R₃ or R₄ is substituted or unsubstituted (CR₆R₆')ₐ-NR₇R₇';
   wherein
   R₆ and R₆' are independently selected from the group consisting of hydrogen, substituted or unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and
R₅ is selected from the group consisting of substituted or unsubstituted aryl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 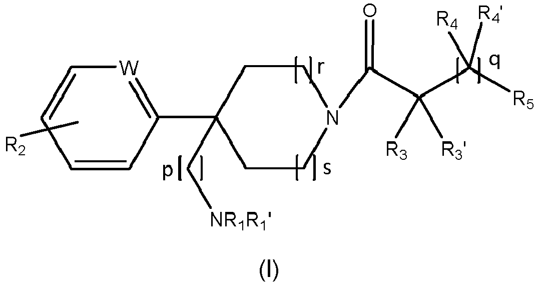 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃, R₃', R₄ and R₄' are independently selected from the group consisting of hydrogen, substituted or unsubstituted C₁₋₆ alkyl, preferably unsubstituted C₁₋₆ alkyl, and substituted or unsubstituted (CR₆R₆')ₐ-NR₇R₇', wherein one of R₃ or R₄ is (CR₆R₆')ₐ-NR₇R₇';
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and
R₅ is selected from the group consisting of substituted or unsubstituted aryl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 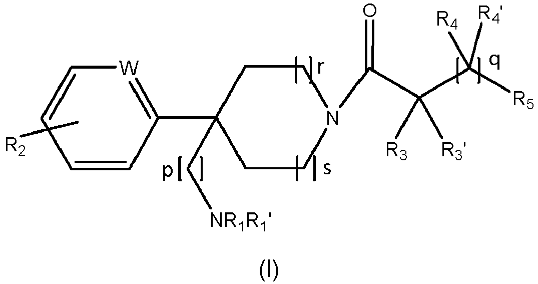 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and
   R₃', R₄ or R₇ forms a cycle with R₅; or
   R₇ forms a cycle with R₄; or
   R₆ forms a cycle with R₄, wherein said cycle is a substituted cycloalkyl being substituted by -NR₇R₇';
   wherein
      R₆ and R₆' are independently selected from the group consisting of hydrogen and unsubstituted C₁₋₆ alkyl; and
      R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
      a is 0, 1 or 2;
and
R₅ is selected from the group consisting of substituted or unsubstituted aryl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 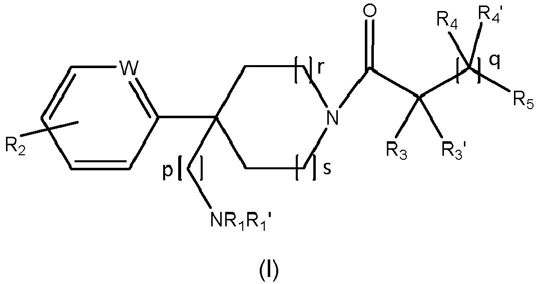 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and
   R₃', R₄ or R₇ forms a cycle with R₅; or
   R₇ forms a cycle with R₄; or
   R₆ forms a cycle with R₄, wherein said cycle is a substituted cycloalkyl being substituted by -NR₇R₇';
   wherein
      R₆ and R₆' are independently selected from the group consisting of hydrogen and unsubstituted C₁₋₆ alkyl; and
      R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
      a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0, 1 or 2;
and
q is 0, 1 or 2;
and
r is 0, 1 or 2;
and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 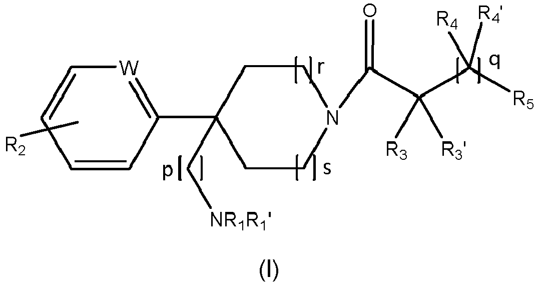 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and
   R₃', R₄ or R₇ forms a cycle with R₅; or
   R₇ forms a cycle with R₄; or
   R₆ forms a cycle with R₄, wherein said cycle is a substituted cycloalkyl being substituted by -NR₇R₇';
   wherein
      R₆ and R₆' are independently selected from the group consisting of hydrogen and unsubstituted C₁₋₆ alkyl; and
      R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
      a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 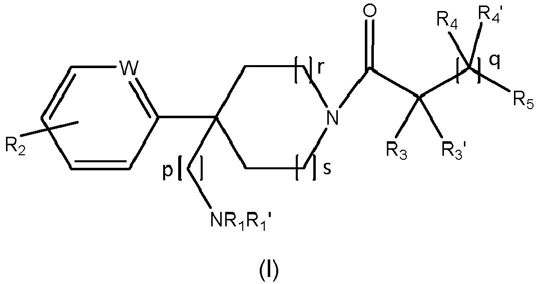 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and R₃', R₄ or R₇ forms a cycle with R₅;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 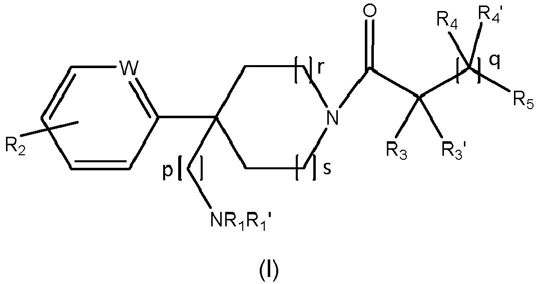 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and R₃' forms a cycle with R₅;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl and substituted or unsubstituted C₁₋₆ alkyl-aryl; and
   a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 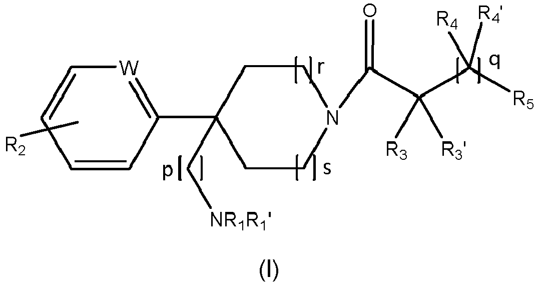 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and R₇ forms a cycle with R₅;
   wherein
   R₆ and R₆' are independently selected from the group consisting of hydrogen and unsubstituted C₁₋₆ alkyl; and
   R₇ is unsubstituted C₁₋₆ alkyl and R₇' is selected from the group consisting of hydrogen and substituted or unsubstituted C₁₋₆ alkyl; and
   a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 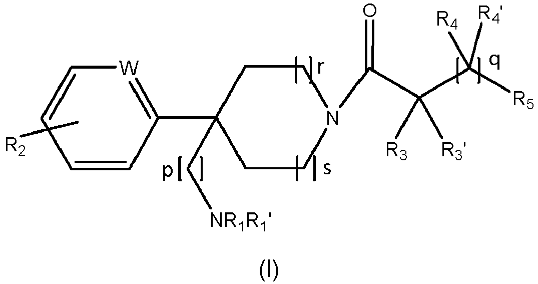 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and R₄ forms a cycle with R₅;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl; and
   a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 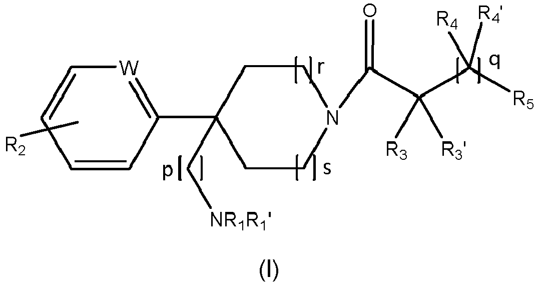 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and R₇ forms a cycle with R₄;
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl, preferably C₁₋₃ alkyl-aryl; and
   a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another particular embodiment, the present invention is directed to a compound of formula (I): 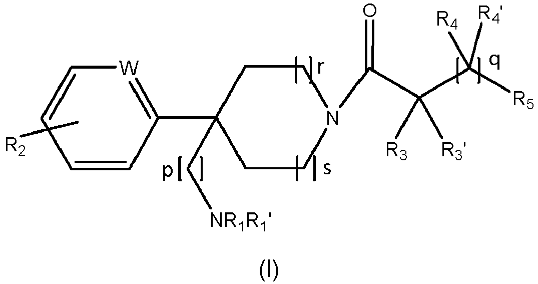 wherein
W is N or CH;
and
R₁ and R₁' are independently selected from the group consisting of hydrogen and substituted or unsubstituted C₁₋₆ alkyl, preferably unsubstituted C₁₋₆ alkyl, more preferably, methyl;
and
R₂ is absent or is selected from the group consisting of halogen, substituted or unsubstituted C₁₋₆ alkyl and OR₂';
   wherein R₂' is selected from the group consisting of hydrogen and substituted or unsubstituted C₁₋₆ alkyl, preferably unsubstituted C₁₋₆ alkyl;
and
R₃ is (CR₆R₆')ₐ-NR₇R₇' and R₆ forms a cycle with R₄, wherein said cycle is a substituted cycloalkyl being substituted by -NR₇R₇';
   wherein
   R₆ and R₆' are independently selected from the group consisting of hydrogen and unsubstituted C₁₋₆ alkyl; and
   R₇ and R₇' are independently selected from the group consisting of hydrogen, substituted or unsubstituted C₁₋₆ alkyl, substituted or unsubstituted aryl, and substituted or unsubstituted C₁₋₆ alkyl-aryl; and
   a is 0, 1 or 2;
and
R₅ is selected from the group consisting of substituted or unsubstituted aryl, preferably a 6 or 7 membered monocyclic aryl, preferably phenyl, substituted or unsubstituted heterocyclyl and substituted or unsubstituted C₄₋₁₀-cycloalkyl;
and
p is 0 or 1;
and
q is 0 or 1;
and
r is 0 or 1;
and
s is 0 or 1;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In a preferred further embodiment, the compound of formula (I) is selected from:

| | |
|---|---|
| [1] | (2*S*,3*R*)-1-(4-(dimethylamino)-4-phenylpiperidin-1-yl)-2-((methylamino)methyl)-3-phenylbutan-1-one dihydrochloride; |
| [2] | (2*S*,3*R*)-1-(4-(dimethylamino)-4-(4-fluorophenyl)piperidin-1-yl)-2-((methylamino)methyl)-3-phenylbutan-1-one; |
| [3] | (2*S*,3*R*)-1-(4-(dimethylamino)-4-(3-fluorophenyl)piperidin-1-yl)-2-((methylamino)methyl)-3-phenylbutan-1-one; |
| [4] | (2*S*,3*R*)-1-(4-(methylamino)-4-phenylpiperidin-1-yl)-2-((methylamino)methyl)-3-phenylbutan-1-one; |
| [5] | (*S*)-2-(aminomethyl)-1-(4-(dimethylamino)-4-phenylpiperidin-1-yl)-3-methyl-3-phenylbutan-1-one; |
| [6] | (2S,3R)-2-(aminomethyl)-1-(4-(methylamino)-4-phenylpiperidin-1-yl)-3-phenylpentan-1-one dihydrochloride; |
| [7] | (2*S*,3*R*)-2-(aminomethyl)-1-(4-(dimethylamino)-4-phenylpiperidin-1-yl)-3-phenylbutan-1-one dihydrochloride; |
| [8] | (2*S*,3*R*)-2-(aminomethyl)-1-(4-(dimethylamino)-4-phenylpiperidin-1-yl)-3-phenylpentan-1-one dihydrochloride; |
| [9] | (2*S*,3*R*)-2-(aminomethyl)-1-(4-(methylamino)-4-phenylpiperidin-1-yl)-3-phenylbutan-1-one; |
| [10] | (2*S*,3*R*)-2-(aminomethyl)-1-(3-(dimethylamino)-3-phenylazetidin-1-yl)-3-phenylpentan-1-one; |
| [11] | (2*S*,3*R*)-2-(aminomethyl)-3-(3-fluorophenyl)-1-(4-(methylamino)-4-phenylpiperidin-1-yl)pentan-1-one; |
| [12] | (2S,3R)-1-(4-(dimethylamino)-4-(4-methoxyphenyl)piperidin-1-yl)-2-((methylamino)methyl)-3-phenylbutan-1-one; |
| [13] | (2*S*,3*R*)-1-(4-(dimethylamino)-4-phenylpiperidin-1-yl)-2-((methylamino)methyl)-3-phenylpentan-1-one dihydrochloride; |
| [14] | (2*S*,3*R*)-1-(4-(dimethylamino)-4-phenylpiperidin-1-yl)-3-(2-fluorophenyl)-2-((methylamino)methyl)butan-1-one dihydrochloride; |
| [15] | (2*S*,3*R*)-1-(4-(dimethylamino)-4-phenylpiperidin-1-yl)-3-(3-fluorophenyl)-2-((methylamino)methyl)butan-1-one; |
| [16] | (2*S*,3*R*)-1-(4-(dimethylamino)-4-(3-hydroxyphenyl)piperidin-1-yl)-2-((methylamino)methyl)-3-phenylbutan-1-one dihydrochloride; |
| [17] | (2*S*,3*R*)-1-(4-(methylamino)-4-phenylpiperidin-1-yl)-2-((methylamino)methyl)-3-phenylpentan-1-one dihydrochloride; |
| [18] | (2*S*,3*R*)-1-(4-((dimethylamino)methyl)-4-phenylpiperidin-1-yl)-2-((methylamino)methyl)-3-phenylbutan-1-one; |
| [19] | (2*S*,3*R*)-1-(3-(dimethylamino)-3-phenylazetidin-1-yl)-2-((methylamino)methyl)-3-phenylpentan-1-one dihydrochloride; |
| [20] | (2*S*,3*R*)-3-(3-fluorophenyl)-1-(4-(methylamino)-4-phenylpiperidin-1-yl)-2-((methylamino)methyl)pentan-1-one; |
| [21] | (2*S*,3*R*)-1-(4-(dimethylamino)-4-phenylpiperidin-1-yl)-3-(3-fluorophenyl)-2-((methylamino)methyl)pentan-1-one; |
| [22] | (2*S*,3*R*)-1-(4-(4-methoxyphenyl)-4-(methylamino)piperidin-1-yl)-2-((methylamino)methyl)-3-phenylpentan-1-one dihydrochloride; |
| [23] | (2*S*,3*R*)-1-(3-(methylamino)-3-phenylazetidin-1-yl)-2-((methylamino)methyl)-3-phenylpentan-1-one dihydrochloride; |
| [24] | (*S*)-2-amino-1-(4-(dimethylamino)-4-phenylpiperidin-1-yl)-3-phenylpropan-1-one dihydrochloride; |
| [25] | (*S*)-1-(4-(dimethylamino)-4-phenylpiperidin-1-yl)-2-(methylamino)-3-phenylpropan-1-one dihydrochloride; |
| [26] | 1-(4-(dimethylamino)-4-phenylpiperidin-1-yl)-4-(methylamino)-2-phenylbutan-1-one; |
| [27] | 3-amino-1-(4-(dimethylamino)-4-phenylpiperidin-1-yl)-3-methyl-2-phenylbutan-1-one dihydrochloride; |
| [28] | 3-amino-2-benzyl-1-(4-(dimethylamino)-4-phenylpiperidin-1-yl)propan-1-one dihydrochloride; |
| [29] | 1-(4-(dimethylamino)-4-phenylpiperidin-1-yl)-3-(methylamino)-2-phenylpropan-1-one; |
| [30] | 2-benzyl-1-(4-(dimethylamino)-4-phenylpiperidin-1-yl)-3-(ethylamino)propan-1-one dihydrochloride; |
| [31] | 2-benzyl-1-(4-(dimethylamino)-4-phenylpiperidin-1-yl)-4-(methylamino)butan-1-one dihydrochloride; |
| [32] | 1-(4-(dimethylamino)-4-phenylpiperidin-1-yl)-2-methyl-3-(methylamino)-2-phenylpropan-1-one dihydrochloride; |
| [33] | 3-amino-1-(4-(dimethylamino)-4-phenylpiperidin-1-yl)-2-methyl-2-phenylpropan-1-one dihydrochloride; |
| [34] | 2-benzyl-1-(4-(dimethylamino)-4-(3-hydroxyphenyl)piperidin-1-yl)-3-(methylamino)propan-1-one dihydrochloride; |
| [35] | (4-(dimethylamino)-4-(3-hydroxyphenyl)piperidin-1-yl)(1,2,3,4-tetrahydroisoquinolin-1-yl)methanone dihydrochloride; |
| [36] | 2-benzyl-1-(4-(dimethylamino)-4-(pyridin-2-yl)piperidin-1-yl)-3-(methylamino)propan-1-one dihydrochloride; |
| [37] | 2-benzyl-1-(4-((dimethylamino)methyl)-4-phenylpiperidin-1-yl)-3-(methylamino)propan-1-one dihydrochloride; |
| [38] | (4-(dimethylamino)-4-(3-hydroxyphenyl)piperidin-1-yl)(1,2,3,4-tetrahydroisoquinolin-4-yl)methanone; |
| [39] | 1-(4-(dimethylamino)-4-phenylpiperidin-1-yl)-2-(3-methoxybenzyl)-3-(methylamino)propan-1-one dihydrochloride; |
| [40] | 3-(3-(4-(dimethylamino)-4-phenylpiperidin-1-yl)-2-((methylamino)methyl)-3-oxopropyl)benzonitrile dihydrochloride; |
| [41] | 2-benzyl-1-(4-(dimethylamino)-4-(3-methoxyphenyl)piperidin-1-yl)-3-(methylamino)propan-1-one dihydrochloride; |
| [42] | 3-amino-2-benzyl-1-(4-(dimethylamino)-4-phenylpiperidin-1-yl)-2-methylpropan-1-one dihydrochloride; |
| [43] | 2-benzyl-1-(4-(dimethylamino)-4-phenylpiperidin-1-yl)-2-methyl-3-(methylamino)propan-1-one; |
| [44] | 2-benzyl-1-(4-(3-hydroxyphenyl)-4-(methylamino)piperidin-1-yl)-3-(methylamino)propan-1-one dihydrochloride; |
| [45] | (4-(dimethylamino)-4-phenylpiperidin-1-yl)(2-((methylamino)methyl)-2,3-dihydro-1 *H*-inden-2-yl)methanone dihydrochloride; |
| [46] | (*R*)-(4-(dimethylamino)-4-phenylpiperidin-1-yl)(1,2,3,4-tetrahydroisoquinolin-3-yl)methanone dihydrochloride; |
| [47] | (S)-(4-(dimethylamino)-4-phenylpiperidin-1-yl)(1,2,3,4-tetrahydroisoquinolin-3-yl)methanone dihydrochloride; |
| [48] | (2*S*,3*S*)-3-amino-2-benzyl-1-(4-(dimethylamino)-4-phenylpiperidin-1-yl)butan-1-one dihydrochloride; |
| [49] | (2*R*,3*R*)-3-amino-2-benzyl-1-(4-(dimethylamino)-4-phenylpiperidin-1-yl)butan-1-one dihydrochloride; |
| [50] | 1-(4-(dimethylamino)-4-phenylpiperidin-1-yl)-2-((methylamino)methyl)-4-phenylbutan-1-one dihydrochloride; |
| [51] | 1-(4-(dimethylamino)-4-phenylpiperidin-1-yl)-2-(4-fluorobenzyl)-3-(methylamino)propan-1-one; |
| [52] | 1-(4-(dimethylamino)-4-phenylpiperidin-1-yl)-2-(3-fluorobenzyl)-3-(methylamino)propan-1-one; |
| [53] | 2-benzyl-3-(methylamino)-1-(4-(methylamino)-4-phenylpiperidin-1-yl)propan-1-one; |
| [54] | 1-(4-(dimethylamino)-4-phenylpiperidin-1-yl)-2-(4-methoxybenzyl)-3-(methylamino)propan-1-one; |
| [55] | 2-(3-(4-(dimethylamino)-4-phenylpiperidin-1-yl)-2-((methylamino)methyl)-3-oxopropyl)benzonitrile dihydrochloride; |
| [56] | 4-(3-(4-(dimethylamino)-4-phenylpiperidin-1-yl)-2-((methylamino)methyl)-3-oxopropyl)benzonitrile dihydrochloride; |
| [57] | 1-(4-(dimethylamino)-4-phenylpiperidin-1-yl)-2-(2-fluorobenzyl)-3-(methylamino)propan-1-one dihydrochloride; |
| [58] | *trans*-(4-(dimethylamino)-4-phenylpiperidin-1-yl)(4-phenylpyrrolidin-3-yl)methanone dihydrochloride; |
| [59] | *cis*-(4-(dimethylamino)-4-phenylpiperidin-1-yl)(4-phenylpyrrolidin-3-yl)methanone dihydrochloride; |
| [60] | *trans*-(4-benzylpyrrolidin-3-yl)(4-(dimethylamino)-4-phenylpiperidin-1-yl)methanone; |
| [61] | (4-(dimethylamino)-4-phenylpiperidin-1-yl)(2,3,4,5-tetrahydro-1*H-*benzo[c]azepin-4-yl)methanone dihydrochloride; |
| [62] | *trans*/*trans*-(2-amino-5-phenylcyclopentyl)(4-(dimethylamino)-4-phenylpiperidin-1-yl)methanone; |
| [63] | ((1*RS*,2*SR*,5*SR*)-2-amino-5-phenylcyclopentyl)(4-(dimethylamino)-4-phenylpiperidin-1-yl)methanone; |
| [64+ | *trans*/*trans*-(4-(dimethylamino)-4-phenylpiperidin-1-yl)(2-(methylamino)-5-phe nylcyclopentyl) meth anon e dihydrochloride; |
| [65] | (2*S*,3*R*)-1-(4-(4-fluorophenyl)-4-(methylamino)piperidin-1-yl)-2-((methylamino)methyl)-3-phenylpentan-1-one; |
| [66] | (2*S*,3*R*)-2-(aminomethyl)-3-(3-chlorophenyl)-1-(4-(dimethylamino)-4-phenylpiperidin-1-yl)pentan-1-one; |
| [67] | (2*S*,3*R*)-3-(3-chlorophenyl)-1-(4-(dimethylamino)-4-phenylpiperidin-1-yl)-2-((methylamino)methyl)pentan-1-one; |
| [68] | 3-amino-2-(2,3-dihydro-1 *H*-inden-1-yl)-1-(4-(dimethylamino)-4-phenylpiperidin-1-yl)propan-1-one; |
| [69] | (2*S*,3*R*)-3-(3-chlorophenyl)-1-(4-(4-fluorophenyl)-4-(methylamino)piperidin-1-yl)-2-((methylamino)methyl)pentan-1-one; |
| [70] | 2-benzyl-1-(4-(dimethylamino)-4-phenylpiperidin-1-yl)-3-(methylamino)propan-1-one; |
| [71] | 2-Benzyl-1-(4-(dimethylamino)-4-phenylpiperidin-1-yl)-3-(phenethylamino)propan-1-one; |
| [72] | 2-benzyl-1-(4-(dimethylamino)-4-phenylpiperidin-1-yl)-3-(isopropylamino)propan-1-one; |
| [73] | 2-benzyl-3-(dimethylamino)-1-(4-(dimethylamino)-4-phenylpiperidin-1-yl)propan-1-one; |
| [74] | *trans*-(1,4-dibenzylpyrrolidin-3-yl)(4-(dimethylamino)-4-phenylpiperidin-1-yl)methanone; |
| [75] | 3-Amino-1-(4-(dimethylamino)-4-phenylpiperidin-1-yl)-2-(2-methylbenzyl)propan-1-one dihydrochloride; |
| [76] | 2-Benzyl-3-(benzylamino)-1-(4-(dimethylamino)-4-phenylpiperidin-1-yl)propan-1-one dihydrochloride; |
| [77] | (*RS*)-3-amino-1-(4-(dimethylamino)-4-phenylpiperidin-1-yl)-2-((*SR*)-1,2,3,4-tetrahydronaphthalen-1-yl)propan-1-one; |
| [78] | (*RS*)-3-amino-1-(4-(dimethylamino)-4-phenylpiperidin-1-yl)-2-((*RS*)-1,2,3,4-tetrahydronaphthalen-1-yl)propan-1-one; |
| [79] | (*S*)-2-benzyl-1-(4-(dimethylamino)-4-phenylpiperidin-1-yl)-3-(methylamino)propan-1-one dihydrochloride; |
| [80] | (*R*)-2-benzyl-1-(4-(dimethylamino)-4-phenylpiperidin-1-yl)-3-(methylamino)propan-1-one dihydrochloride; |
| [81] | (*R*)-3-amino-2-((*S*)-2,3-dihydro-1*H*-inden-1-yl)-1-(4-(dimethylamino)-4-phenylpiperidin-1-yl)propan-1-one; |
| [82] | (*S*)-3-amino-2-((*R*)-2,3-dihydro-1*H*-inden-1-yl)-1-(4-(dimethylamino)-4-phenylpiperidin-1-yl)propan-1-one; |
| [83] | (*R*)-3-amino-2-((*R*)-2,3-dihydro-1*H*-inden-1-yl)-1-(4-(dimethylamino)-4-phenylpiperidin-1-yl)propan-1-one; |
| [84] | (*S*)-3-amino-2-((*S*)-2,3-dihydro-1*H*-inden-1-yl)-1-(4-(dimethylamino)-4-phenylpiperidin-1-yl)propan-1-one; |
| [85] | (2*S*,3*S*)-1-(4-(dimethylamino)-4-phenylpiperidin-1-yl)-2-((methylamino)methyl)-3-phenylbutan-1-one; |
| [86] | (2*R*,3*R*)-1-(4-(dimethylamino)-4-phenylpiperidin-1-yl)-2-((methylamino)methyl)-3-phenylbutan-1-one; and |
| [87] | (2*R*,3*S*)-1-(4-(dimethylamino)-4-phenylpiperidin-1-yl)-2-((methylamino)methyl)-3-phenylbutan-1-one. |

optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another aspect, the invention refers to a process for the preparation of a compound of formula (I) as defined above.

The obtained reaction products may, if desired, be purified by conventional methods, such as crystallization and chromatography. Where the processes described below for the preparation of compounds of the invention give rise to mixtures of stereoisomers, these isomers may be separated by conventional techniques such as preparative chromatography. The compounds may be prepared in racemic form, or individual enantiomers may be prepared either by enantiospecific synthesis or by resolution.

One preferred pharmaceutically acceptable form of a compound of the invention is the crystalline form, including such form in pharmaceutical composition. In the case of salts and also solvates of the compounds of the invention the additional ionic and solvent moieties must also be non-toxic. The compounds of the invention may present different polymorphic forms, it is intended that the invention encompasses all such forms.

The compounds of formula (I) can be obtained by following the method described below. As it will be obvious to one skilled in the art, the exact method used to prepare a given compound may vary depending on its chemical structure.

The compounds of formula (I) may be prepared by a one step process as described in Scheme 1. 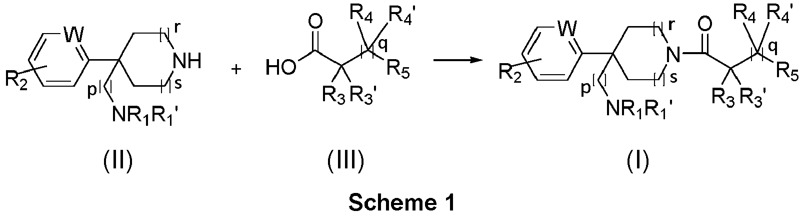 wherein R₁, R₁', R₂, R₃, R₃', R₄, R₄', R₅, W, p, q, r and s have the same meaning as indicated above for a compound of formula (I).

In this sense, in another aspect, the invention refers to a process for the preparation of a compound of formula (I): 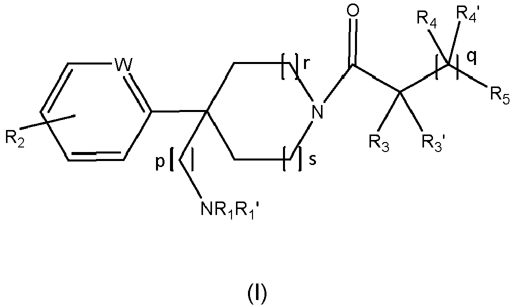 said process comprising:
treating a compound of formula (II): 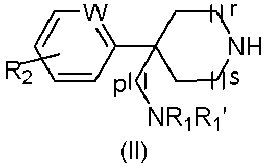 with a compound of formula (III): 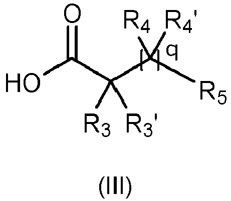 wherein R₁, R₁', R₂, R₃, R₃', R₄, R₄', R₅, W, p, q, r and s are as defined above for a compound of formula (I).

In a particular embodiment, the preparation of a compound of formula (I) from a compound of formula (II) and an acid compound of formula (III) can be carried out under conventional amidation conditions, preferably using a suitable coupling reagent such as *N*-[(dimethylamino)-1*H*-1,2,3-triazolo-[4,5-*b*]pyridin-1-ylmethylene]-*N-*methylmethanaminium hexafluorophosphate *N*-oxide (HATU), *N*-(3-dimethylaminopropyl)-*N'*-ethylcarbodiimide (EDC), *N,N,N',N'*-tetramethyl-*O*-(*1H-*benzotriazol-1-yl)uronium hexafluorophosphate (HBTU), (benzotriazol-1-yloxy)tripyrrolidinophosphonium hexafluorophosphate (PyBOP), dicyclohexylcarbodiimide (DCC), or propylphosphonic anhydride (T3P), optionally in the presence of 1-hydroxybenzotriazole, optionally in the presence of an organic base such as *N,N*-diisopropylethylamine, *N*-methylmorpholine or triethylamine, in a suitable solvent such as dimethylformamide or dichloromethane, and at a suitable temperature, preferably at room temperature. Alternatively, the amidation can be performed in two steps by first converting an acid of formula (III) into its corresponding acyl halide or mixed anhydride following standard conditions described in the literature, and then reacting it with a compound of formula (II) in a suitable solvent, such as dichloromethane, tetrahydrofuran, ethyl acetate or ethyl acetate-water mixtures; in the presence of an organic base such as triethylamine or *N,N*-diisopropylethylamine or an inorganic base such as K₂CO₃; and at a suitable temperature, preferably comprised between 0 °C and the reflux temperature. Additionally, an activating agent such as 4-dimethylaminopyridine can be used.

For the amidation process described above it may be necessary to protect the amino group present in either R₃ or R₄ wherein they represent (CR₆R₆')ₐ-NR₇R₇' with suitable protecting groups, such as for example Boc (*tert*-butoxycarbonyl), Cbz (benzyloxycarbonyl) or benzyl. The procedures for the introduction and removal of these protecting groups are well known in the art and can be found thoroughly described in the literature. As a way of example, for Cbz or benzyl as protecting group, the deprotection reaction is preferably carried out by hydrogenation under hydrogen atmosphere and metal catalysis, preferably by the use of palladium or palladium hydroxide over charcoal as catalyst in a suitable solvent such as methanol or ethanol, optionally in the presence of an acid such as acetic acid or hydrochloric acid. When the protecting group is Boc, the deprotection can be conducted by adding a solution of a strong acid such as HCI, in a suitable solvent such as diethyl ether, 1,4-dioxane or methanol, or with trifluoroacetic acid in dichloromethane.

The compounds of formula (II) and (III) are commercially available or can be synthesized following common procedures described in the literature.

In a particular embodiment, the chiral synthesis of a particular subtype of beta-aminoacids (IIIa) and (IIIb) may be acomplished following a four to five step-process that renders the corresponding *N*-protected precursors (IIIa) and (IIIb), as shown in Scheme 2. 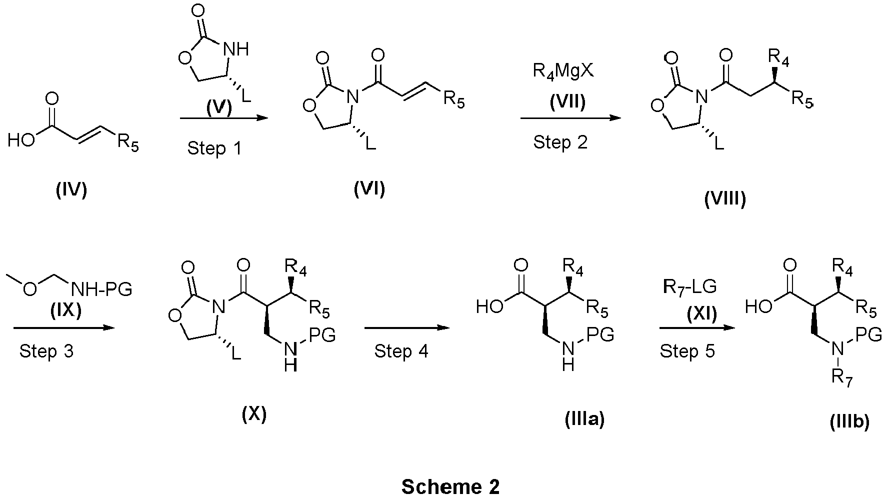 wherein R₄, R₅ and R₇ have the same meanings as defined above for the compound of Formula (I), L represents aryl, aralkyl or alkyl, preferably phenyl, X represents chloro, bromo or iodo, PG represents a suitable amino-protecting group, preferably Cbz or Boc, and LG represents a leaving group such as halogen, mesylate, nosylate, tosylate or triflate.

In another particular embodiment, the invention refers to a process for the manufacture of a compound of formula (VI) 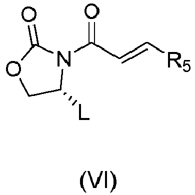 said process comprising:
treating a compound of formula (IV): 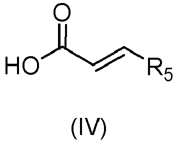 with a compound of formula (V): 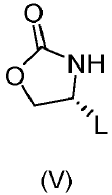 wherein R₅ is as defined above for a compound of formula (I) and L represents aryl, aryl-alkyl, preferably phenyl or alkyl.

Starting from an acid of formula (IV), the corresponding acyl chloride is generated *in situ* and it is reacted with a chiral oxazolidin-2-one of formula (V) to render a chiral Michael acceptor of formula (VI). The reaction is carried out in a suitable solvent such as dichloromethane, in the presence of a base such as triethylamine or *N,N-*diisopropylethylamine.

In step 2, a Michael addition using a Grignard reagent of formula (VII) to render a chiral compound of formula (VIII) is performed, in the presence of a copper source such as copper bromide-dimethyl sulfide complex and a Lewis acid such as boron trifluoride diethyl etherate, in a suitable solvent such as tetrahydrofuran.

Thus, in another particular embodiment, the invention refers to a process for the manufacture of a compound of formula (VIII): 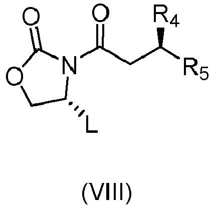 said process comprising:
treating a compound of formula (VI): 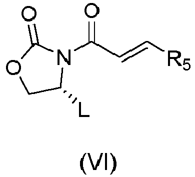 with a compound of formula (VII):

R₄MgX (VII)

wherein R₄ and R₅ have the same meaning as defined above for the compound of Formula (I), L represents aryl, aryl-alkyl, preferably phenyl or alkyl and X represents chloro, bromo or iodo.

In step 3, treatment of a compound of formula (VIII) with an electrophilic compound of formula (IX) in the presence of a Lewis acid such as titanium (IV) chloride and a suitable base such as triethylamine, in a suitable solvent such as dichloromethane, yielded a chiral compound of formula (X).

Thus, in another particular embodiment, the invention refers to a process for the manufacture of a compound of formula (X): 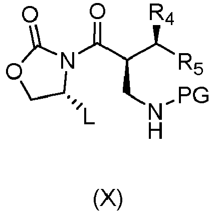 said process comprising:
treating a compound of formula (VIII): 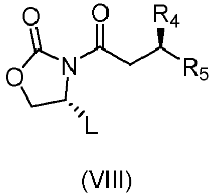 with a compound of formula (IX): 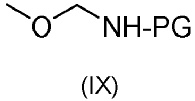 wherein R₄ and R₅ have the same meanings as defined above for the compound of Formula (I), L represents aryl, aryl-alkyl, preferably phenyl or alkyl, and PG represents a suitable amino-protecting group, preferably Cbz or Boc.

Finally, removal of the chiral auxiliary using lithium peroxide (generated *in situ* from lithium hydroxide and hydrogen peroxide) in aqueous tetrahydrofuran rendered a *N-*protected chiral acid of formula (IIIa)

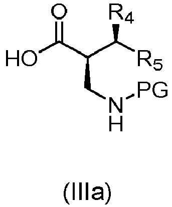

This compound (IIIa) can be further derivatized to a *N*-protected chiral acid of formula (IIIb): 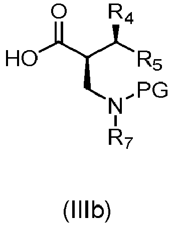 by reaction with an alkylating agent of formula (XI), in the presence of a base such as sodium hydride, in a suitable solvent such as tetrahydrofuran or dimethylformamide.

Thus, in another particular embodiment, the invention refers to a process for the manufacture of a compound of formula (IIIb): 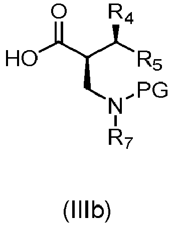 said process comprising:
treating a compound of formula (IIIa) 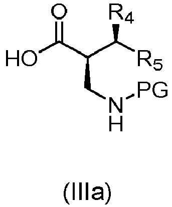 with a compound of formula (XI):

R₇-LG (XI)

wherein R₄, R₅ and R₇ have the same meanings as defined above for the compound of Formula (I), PG represents a suitable amino-protecting group, preferably Cbz or Boc, and LG represents a leaving group such as halogen, mesylate, nosylate, tosylate or triflate.

The compounds of formula (IV), (V), (VII), (IX) and (XI) are commercially available or can be synthesized following common procedures described in the literature.

Moreover, certain compounds of the present invention can also be obtained starting from other compounds of formula (I) by appropriate conversion reactions of functional groups, in one or several steps, using well-known reactions in organic chemistry under standard experimental conditions. For example, starting from a compound of formula (I) wherein R₇ or R_{7'} in (CR₆R₆')ₐ-NR₇R₇' are independently hydrogen, either one or both of R₇ or R_{7'} can be transformed into an alkyl or alkyl-aryl group under standard reductive amination or alkylation conditions.

The reductive amination reaction is carried out in the presence of a suitable aldehyde or ketone and a reductive reagent, such as sodium cyanoborohydride, sodium triacetoxyborohydride or sodium borohydride, in a suitable solvent, preferably 1,2-dichloroethane, tetrahydrofuran or methanol, optionally in the presence of an acid (preferably acetic acid) or a base (preferably *N,N*-diisopropylethylamine). Alternatively, the alkylation reaction is carried out in the presence of an alkylating agent, in a suitable solvent, such as dimethylformamide, dimethylsulfoxide, ethanol or acetonitrile, preferably dimethylformamide; in the presence of a base such as sodium hydride, potassium tert-butoxide, potassium carbonate, *N,N*-diisopropylethylamine or triethylamine, preferably sodium hydride; at a suitable temperature, comprised between room temperature and the reflux temperature; and preferably the alkylation reaction is carried out with the amino group in (CR₆R₆')ₐ-NR₇R₇' protected as Boc or Cbz.

In addition, a compound of formula (I) can be obtained in enantiopure form by resolution of a racemic compound of formula (I) or a diastereomeric mixture, either by chiral preparative HPLC or by crystallization of a diastereomeric salt or co-crystal. Alternatively, the resolution step can be carried out at a previous stage, using any suitable intermediate.

In another aspect, the invention refers to the use of a compound selected from 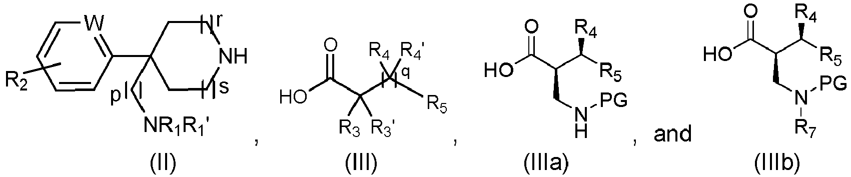 for the manufacture of a compound of formula (I), wherein R₁, R₁', R₂, R₃, R₃', R₄, R₄', R₅, W, p, q, r and s have the same meanings as defined above for the compound of Formula (I), and PG represents a suitable amino-protecting group, preferably Cbz or Boc.

Turning to another aspect, the invention also relates to the therapeutic use of the compounds of general formula (I). As mentioned above, compounds of general formula (I) show a strong affinity to the subunit α2δ and more preferably to the α2δ-1 subunit of voltage-gated calcium channels. In a more preferred embodiment of the invention compounds of general formula (I) show a strong affinity both to the subunit α2δ and more preferably to the α2δ-1 subunit of voltage-gated calcium channels as well as to the µ-receptor and can behave as agonists, antagonists, inverse agonists, partial antagonists or partial agonists thereof. Therefore, compounds of general formula (I) are useful as medicaments.

Another aspect of the invention refers to a pharmaceutical composition which comprises a compound according to the invention as described above according to formula (I) or a pharmaceutically acceptable salt thereof, prodrug, solvate or steroisomer thereof, and a pharmaceutically acceptable carrier, adjuvant or vehicle. The present invention thus provides pharmaceutical compositions comprising a compound of this invention, or a pharmaceutically acceptable salt, prodrug, solvate or stereoisomers thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to a compound of formula (I) as described above, or a pharmaceutical acceptable salt or isomer thereof for use as a medicament.

Another aspect of the invention refers to a compound of formula (I), or a pharmaceutically acceptable salt or isomer thereof, for use in the treatment or prophylaxis of pain. Preferably, the pain is medium to severe pain, visceral pain, chronic pain, cancer pain, migraine, inflammatory pain, acute pain or neuropathic pain, including central neuropathic pain and peripheral neuropathic pain, allodynia or hyperalgesia. This may include mechanical allodynia or thermal hyperalgesia.

Another aspect of the invention refers to the use of a compound of the invention in the manufacture of a medicament for the treatment or prophylaxis of pain. In a preferred embodiment the pain is selected from medium to severe pain, visceral pain, chronic pain, cancer pain, migraine, inflammatory pain, acute pain or neuropathic pain, including central neuropathic pain and peripheral neuropathic pain, allodynia or hyperalgesia, also preferably including mechanical allodynia or thermal hyperalgesia.

Another aspect of this invention relates to a method of treating or preventing pain which method comprises administering to a patient in need of such a treatment or prevention a therapeutically effective amount of a compound as above defined or a pharmaceutical composition thereof. Among the pain syndromes that can be treated or prevented are medium to severe pain, visceral pain, chronic pain, cancer pain, migraine, inflammatory pain, acute pain or neuropathic pain, including central neuropathic pain and peripheral neuropathic pain, allodynia or hyperalgesia, whereas this could also include mechanical allodynia or thermal hyperalgesia.

The present invention is illustrated below with the aid of examples. These illustrations are given solely by way of example and do not limit the general spirit of the present invention.

### EXAMPLES

The following abbreviations are used in the examples:
ACN: acetonitrile
Anh.: anhydrous
aq.: aqueous
Boc: *tert*-butoxycarbonyl
CH: cyclohexane
conc.: concentrated
DBU: 1,8-Diazabicyclo[5.4.0]undec-7-ene
DCE: dichloroethane
DCM: dichloromethane
DIPEA: *N,N*-diisopropylethylamine
DMF: *N,N*-dimethylformamide
EtOAc: ethyl acetate
EtOH: ethanol
EX: example
h: hour/s
HATU: *O*-(7-azabenzotriazol-1-yl)-*N,N,N',N'*-tetramethyluronium hexafluorophosphate
HPLC: high performance liquid chromatography
INT: intermediate
*i*-PrOH: isopropanol
LDA: lithium diisopropylamide
MeOH: methanol
MS: mass spectrometry
Min: minutes
Quant: quantitative
Ret.: retention
r.t.: room temperature
Sat: saturated
Sol.: solution
TEA: triethylamine
THF: tetrahydrofuran
wt: weight

The following methods were used to determine the HPLC-MS spectra:

### Method A

Column: Kinetex EVO 50 x 4.6 mm, 2.6 µm
Temperature: 40 °C
Flow: 1.5 mL/min
Gradient: NH₄HCO₃ pH 8 : ACN (95:5)---0.5min---(95:5)---6.5min---(0:100)---2min-(0:100)
Sample dissolved approx. 1 mg/mL in NH₄HCO₃ pH 8/ ACN

### Method B

Column: Aquity UPLC BEH C18 2.1 x 50 mm, 1.7 µm
Temperature: ambient
Flow: 0.61 mL/min
Gradient: NH₄HCO₃ 10 mM pH 10.6 : ACN (98:2)---0.3 min---(98:2)---2.7 min---(0:100)---2min---(0:100)
Sample dissolved approx. 1 mg/mL in NH₄HCO₃ pH 10.6/ ACN

### Synthesis of Intermediates

### Intermediate 1A: (2S,3R)-2-((((Benzyloxy)carbonyl)amino)methyl)-3-phenylbutanoic acid

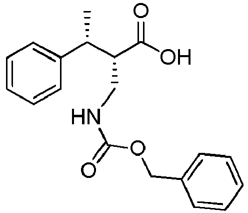

**Step 1. (*R*)-3-Cinnamoyl-4-phenyloxazolidin-2-one:** To a solution of cinnamic acid (12.0 g, 81.0 mmol) in DCM (50 mL), cooled at 0 °C under a N₂ atmosphere, oxalyl chloride (13.9 mL, 162 mmol) was added dropwise. The resulting solution was then stirred at r.t. for 4 h and finally it was concentrated to dryness to render cinnamoyl chloride (quant. yield assumed). To a stirred solution of (R)-4-phenyloxazolidin-2-one (13.2 g, 81.0 mmol) and TEA (16.9 mL, 121 mmol) in DCM (50 mL), cooled at 0 °C, a solution of the previously prepared cinnamoyl chloride (81.0 mmol) in DCM (8 mL) was added dropwise. The mixture was stirred at 0 °C for 1 h and then left to warm to r.t. overnight. NH₄Cl sat. sol. was then added and it was extracted with DCM. The combined organic phases were dried over Na₂SO₄, filtered and concentrated to dryness. The resulting crude solid was slurried in hot EtOAc (5 volumes), then it was allowed to cool down to r.t. The solids were collected by filtration and dried under vacuum to give the title compound (19.12 g, 80% yield).
**Step 2. (*R*)-4-Phenyl-3-((*R*)-3-phenylbutanoyl)oxazolidin-2-one:** To a suspension of CuBr·Me₂S complex (8.41 g, 40.9 mmol) in dry THF (46 mL), cooled at -40 °C under a N₂ atmosphere, methylmagnesium bromide (1.0 M in THF, 82.0 mL, 82.0 mmol) was added dropwise and the resulting yellow-green mixture was stirred for 25 min at this temperature. Then, BF₃·Et₂O (5.05 mL, 40.9 mmol) was added dropwise and the mixture was stirred for further 25 min. A solution of the product obtained in Step 1 (8.00 g, 27.3 mmol) in dry THF (46 mL) was added and the resulting heterogeneous mixture was allowed to warm to -20°C over 3.5 h. NH₄Cl sat. sol. was then added and it was extracted with EtOAc. The combined organic phases were dried over Na₂SO₄, filtered and concentrated to dryness. The resulting crude solid was recrystallized from hot *i*-PrOH (10 volumes) to give the title compound (7.80 g, 92% yield).
**Step 3. Benzyl ((2*S*,3*R*)-2-((*R*)-2-oxo-4-phenyloxazolidine-3-carbonyl)-3-phenylbutyl)carbamate:** To a solution of the product obtained in Step 2 (1.83 g, 5.92 mmol) in anh. DCM (30 mL), cooled to -15 °C under a N₂ atmosphere, titanium(IV) chloride (0.72 mL, 6.51 mmol) was added dropwise. Then, TEA (0.91 mL, 6.51 mmol) was added and the resulting dark red solution was stirred at -15 °C for 30 min. In a separate flask, benzyl (methoxymethyl)carbamate (prepared according to Chem. Eur. J. 2012, 6655; 1.27 g, 6.51 mmol) and titanium(IV) chloride (0.72 mL, 6.51 mmol) were added to DCM (16 mL) at 0 °C and the resulting solution was added to the previous mixture via cannula. The reaction mixture was stirred at 0 °C for 4 h. Then, NH₄Cl sat. sol. was added and it was allowed to warm to r.t. The phases were separated and the aqueous phase was extracted with DCM. The combined organic phases were washed with brine, dried over Na₂SO₄, filtered and concentrated to dryness. The residue was purified by flash chromatography, silica gel, gradient CH to EtOAc, to give the title compound (1.88 g, 67% yield).
**Step 4. Title compound:** To a solution of the product obtained in Step 3 (1.56 g, 3.30 mmol) in a mixture of THF (8.25 mL) and water (2 mL), cooled at 0 °C, hydrogen peroxide (30%wt aq. sol, 1.35 mL, 13.2 mmol) and lithium hydroxide monohydrate (0.24 g, 5.78 mmol) were added. The resulting mixture was stirred at 0 °C for 1 h and then it was allowed to warm to r.t. overnight. Na₂S₂O₃ sat. sol. was added and THF was evaporated under vacuo. The residual aqueous phase was extracted with EtOAc and the combined organic phases were evaporated to dryness. The residue was dissolved in DCM and it was extracted with 1 N NaOH. The basic aqueous phase was then acidified with 2.8 N HCI to pH 1 and it was finally extracted with EtOAc. The combined organic phases were dried over Na₂SO₄, filtered and concentrated to dryness. The crude product was used in the next step without further purification (1.12 g, quant yield).

This method was used for the preparation of Intermediates 1B-1F using suitable starting materials:

| **INT** | **Structure** | **Chemical name** |
|---|---|---|
| **1B** | 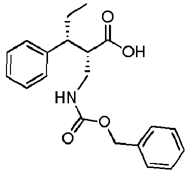 | (2*S*,3*R*)-2-((((benzyloxy)carbonyl)amino)methyl)-3-phenylpentanoic acid |
| **1C** | 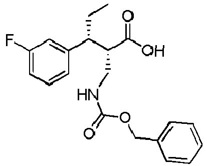 | (2*S*,3*R*)-2-((((benzyloxy)carbonyl)amino)methyl)-3-(3-fluorophenyl)pentanoic acid |
| **1D** | 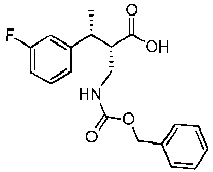 | (2*S*,3*R*)-2-((((benzyloxy)carbonyl)amino)methyl)-3-(3-fluorophenyl)butanoic acid |
| **1E** | 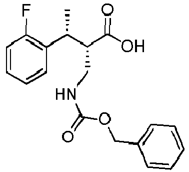 | (2*S*,3*R*)-2-((((benzyloxy)carbonyl)amino)methyl)-3-(2-fluorophenyl)butanoic acid |
| **1F** | 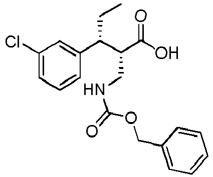 | (2*S*,3*R*)-2-((((benzyloxy)carbonyl)amino)methyl)-3-(3-chlorophenyl)pentanoic acid |

### Intermediate 2A: (2S,3R)-2-(((tert-Butoxycarbonyl)amino)methyl)-3-phenylbutanoic acid

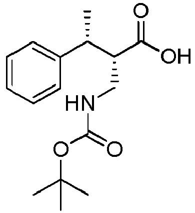

**Step 1. (2S,3R)-2-(Aminomethyl)-3-phenylbutanoic acid:** A pressure flask was charged with a solution of Intermediate 1A (1.92 g, 5.86 mmol) in MeOH (45 mL) and it was purged with N₂. Palladium hydroxide on carbon (20 wt%, wet, 0.19 g) was added and the mixture was stirred under 2 bar of H₂ overnight at 40 °C. The catalyst was filtered off through a pad of Celite that was washed thoroughly with MeOH. The filtrate was evaporated to dryness. The resulting crude product was used in the next step without further purification (1.10 g, 97% yield).
**Step 2. Title compound:** To a solution of the product obtained in Step 1 (0.81 g, 4.20 mmol) in a solution of 1,4-dioxane (15.6 mL) and water (7.80 mL), di-*tert*-butyl dicarbonate (1.37 g, 6.30 mmol) and 6 M NaOH solution (2.10 mL, 12.6 mmol) were added. The resulting mixture was stirred at r.t. overnight. Then, it was cooled to 0 °C, the pH was adjusted to 2 with 1 N HCI and it was extracted with EtOAc. The combined organic phases were washed with brine, dried over Na₂SO₄, filtered and concentrated to dryness. The crude product obtained was used as such without further purification (1.24 g, quant. yield).

This method was used for the preparation of Intermediates 2B-2E using suitable starting materials:

| **INT** | **Structure** | **Chemical name** |
|---|---|---|
| **2B** | 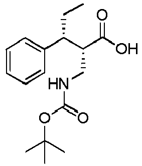 | (2*S*,3*R*)-2-(((*tert-*butoxycarbonyl)amino)methyl)-3-phenylpentanoic acid |
| **2C** | 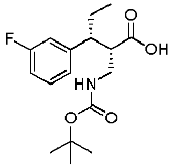 | (2*S*,3*R*)-2-(((*tert-*butoxycarbonyl)amino)methyl)-3-(3-fluorophenyl)pentanoic acid |
| **2D** | 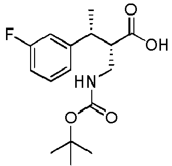 | (2*S*,3*R*)-2-(((*tert-*butoxycarbonyl)amino)methyl)-3-(3-fluorophenyl)butanoic acid |
| **2E** | 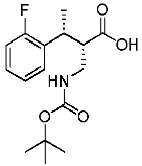 | (2*S*,3*R*)-2-(((*tert-*butoxycarbonyl)amino)methyl)-3-(2-fluorophenyl)butanoic acid |

### Intermediate 2F: (2S,3R)-2-(((tert-Butoxycarbonyl)amino)methyl)-3-(3-chlorophenyl)pentanoic acid

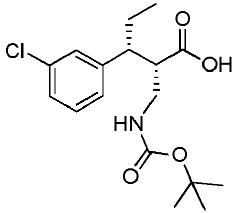

**Step 1. (2S,3R)-2-(Aminomethyl)-3-(3-chlorophenyl)pentanoic acid:** A solution of Intermediate 1F (0.5 g, 1.33 mmol) and iodotrimethylsilane (0.56 mL, 3.96 mmol) in ACN (20 mL) was stirred at r.t. for 30 min. MeOH (5 mL) was added to quench the reaction and it was concentrated to dryness. The resulting crude product was used in the next step without further purification (0.81 g, overweight, 39 wt%; 0.32 g theoretical weight).
**Step 2. Title compound:** Starting from the product obtained in Step 1 (0.81 g, 39 wt%, 1.33 mmol) and following the experimental procedure described for the preparation of Step 2 of Intermediate 2A, the title compound was obtained as a crude product that was used as such without further purification (0.46 g, quant. yield).

### Intermediate 3A: (2S,3R)-2-(((tert-Butoxycarbonyl)(methyl)amino)methyl)-3-phenylbutanoic acid

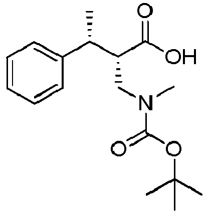

To a solution of Intermediate 2A (0.40 g, 1.36 mmol) in anh. THF (15.5 mL), cooled at 0 °C under a N₂.atmosphere, NaH (0.33 g, 8.18 mmol; 60 wt% dispersion in mineral oil) was added. The heterogeneous mixture was stirred at 0 °C for 30 min and then iodomethane (0.51 mL, 8.18 mmol) was added dropwise. The reaction mixture was allowed to warm to r.t. overnight. Then, it was cooled to 0 °C, 1 N HCI was added until pH was adjusted to 4 and it was extracted with EtOAc. The combined organic phases were washed with brine, dried over Na₂SO₄, filtered and concentrated to dryness. The crude product was used in the next step without further purification (0.41 g, 98% yield).

This method was used for the preparation of Intermediates 3B-3Q using suitable starting materials:

| **INT** | **Structure** | **Chemical name** |
|---|---|---|
| **3B** | 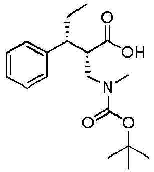 | (2*S*,3*R*)-2-(((*tert-*butoxycarbonyl)(methyl)amino)methyl)-3-phenylpentanoic acid |
| **3C** | 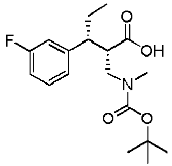 | (2*S*,3*R*)-2-(((*tert-*butoxycarbonyl)(methyl)amino)methyl)-3-(3-fluorophenyl)pentanoic acid |
| **3D** | 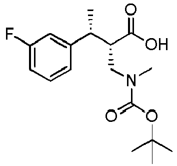 | (2*S*,3*R*)-2-(((*tert-*butoxycarbonyl)(methyl)amino)methyl)-3-(3-fluorophenyl)butanoic acid |
| **3E** | 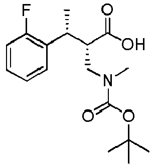 | (2*S*,3*R*)-2-(((*tert-*butoxycarbonyl)(methyl)amino)methyl)-3-(2-fluorophenyl)butanoic acid |
| **3F** | 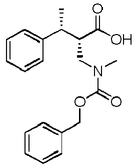 | (2S,3R)-2-((((benzyloxy)carbonyl)(methyl)amino)me thyl)-3-phenylbutanoic acid |
| **3G** | 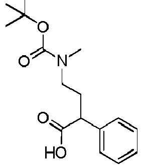 | 4-((*tert*-butoxycarbonyl)(methyl)amino)-2-phenylbutanoic acid |
| **3H** | 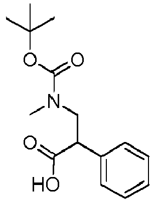 | 3-((*tert*-butoxycarbonyl)(methyl)amino)-2-phenylpropanoic acid |
| **3I** | 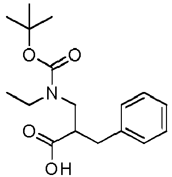 | 2-benzyl-3-((*tert-*butoxycarbonyl)(ethyl)amino)propanoic acid |
| **3J** | 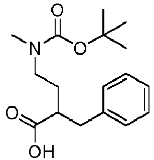 | 2-benzyl-4-((*tert-*butoxycarbonyl)(methyl)amino)butanoic acid |
| **3K** | 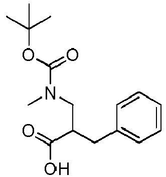 | 2-benzyl-3-((*tert-*butoxycarbonyl)(methyl)amino)propanoic acid |
| **3L** | 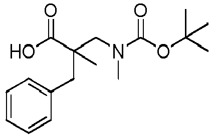 | 2-benzyl-3-((*tert-*butoxycarbonyl)(methyl)amino)-2-methylpropanoic acid |
| **3M** | 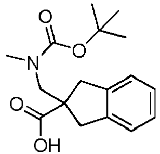 | 2-(((*tert-*butoxycarbonyl)(methyl)amino)methyl)-2,3-dihydro-1 *H*-indene-2-carboxylic acid |
| **3N** | 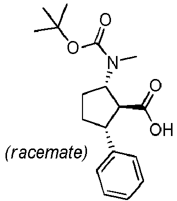 | *trans*/*trans*-2-((*tert-*butoxycarbonyl)(methyl)amino)-5-phenylcyclopentanecarboxylic acid |
| **3O** | 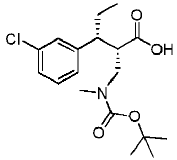 | (2*S*,3*R*)-2-(((*tert-*butoxycarbonyl)(methyl)amino)methyl)-3-(3-chlorophenyl)pentanoic acid |
| **3P** | 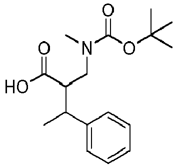 | 2-(((*tert-*butoxycarbonyl)(methyl)amino)methyl)-3-phenylbutanoic acid |
| **3Q** | 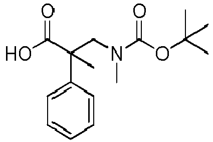 | 3-((*tert*-butoxycarbonyl)(methyl)amino)-2-methyl-2-phenylpropanoic acid |

### Intermediate 4: (S)-2-((((Benzyloxy)carbonyl)amino)methyl)-3-methyl-3-phenylbutanoic acid

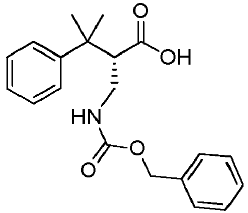

**Step 1. 3-Methyl-3-phenylbutanoic acid:** To a solution of 3-methylbut-2-enoic acid (1.0 g, 10.0 mmol) in anh. benzene (2.5 mL), cooled at 0-5 °C, AlCl₃ (1.60 g, 12.0 mmol) was added portionwise over 1 h under an argon atmosphere. The reaction mixture was stirred for further 20 min at this temperature and afterwards at r.t. overnight. Then, the reaction mixture was poured into ice and it was allowed to stir for 15 min. Benzene was evaporated under vacuo and the resulting solid residue was slurried in a mixture of EtOH-water 1:1. The solids were filtered and dried under vacuum. The crude solid was slurried in water, the pH was adjusted to 3 with 1 N HCI and it was extracted 3 times with Et₂O. The combined organic phases were extracted six times with Na₂CO₃ sat. sol. The basic aqueous phases were combined, the pH was readjusted to 3 with 3 N HCI and it was extracted again with Et₂O. After checking that both ethereal phases contained desired product by HPLC/MS, they were combined, dried over Na₂SO₄, filtered and concentrated to dryness. The crude product obtained was used in the next step without further purification (0.835 g, 47% yield).
**Step 2. (R)-3-(3-Methyl-3-phenylbutanoyl)-4-phenyloxazolidin-2-one:** Following the experimental procedure described in Step 1 of Intermediate 1A, but using the product obtained in Step 1 instead of cinnamic acid as starting material, the title compound was obtained (0.766 g, 50% yield).
**Step 3. Benzyl ((*S*)-3-methyl-2-((*R*)-2-oxo-4-phenyloxazolidine-3-carbonyl)-3-phenylbutyl)carbamate:** Following the experimental procedure described in Step 3 of Intermediate 1A, but using the product obtained in Step 2 as starting material, the title compound was obtained (0.110 g, 36% yield).
**Step 4. Title compound:** Following the experimental procedure described in Step 4 of Intermediate 1A, but using the product obtained in Step 3 as starting material, the title compound was obtained (0.030 g, 39% yield).

### Intermediate 5A: 3-((tert-Butoxycarbonyl)amino)-3-methyl-2-phenylbutanoic acid

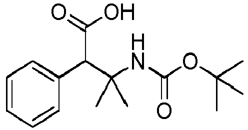

Following the experimental procedure described in Step 2 of Intermediate 2A, starting from 3-amino-3-methyl-2-phenylbutanoic acid hydrochloride (56 mg, 0.24 mmol), the title compound was obtained (28 mg, 39% yield).

### Intermediate 5B: 3-((tert-Butoxycarbonyl)amino)-2-methyl-2-phenylpropanoic acid

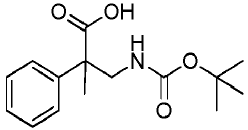

Following the experimental procedure described in Step 2 of Intermediate 2A, starting from 3-amino-2-methyl-2-phenylpropanoic acid hydrochloride (prepared according to WO2010/127329; 270 mg, 1.25 mmol), the title compound was obtained (239 mg, 95% yield).

### Intermediate 6A: 3-((tert-Butoxycarbonyl)(methyl)amino)-2-(3-methoxybenzyl)propanoic acid

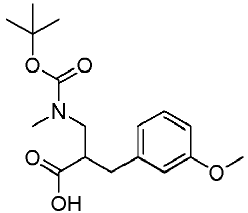

**Step 1. Methyl 3-((*tert*-butoxycarbonyl)(methyl)amino)-2-(3-methoxybenzyl)propanoate:** To a solution of methyl 3-((*tert-*butoxycarbonyl)(methyl)amino)propanoate (0.2 g, 0.92 mmol) in anh. THF (2 mL), cooled at -78 °C under a N₂ atmosphere, LDA (0.73 mL, 1.1 mmol, 1.5 M in THF/Ethylbenzene/Heptane) was added dropwise and the mixture was stirred for 30 min at -78 °C. Then, a solution of 1-(bromomethyl)-3-methoxybenzene (0.13 mL, 0.92 mmol) in anh. THF (2 mL) was added dropwise and the reaction mixture was left to reach r.t. NH₄Cl sat. sol. was added and it was extracted with EtOAc. The combined organic extracts were washed with brine, dried over MgSO₄, filtered and concentrated to dryness. The residue was purified by flash chromatography, silica gel, gradient CH to EtOAc, to give the title compound (0.245 g, 79% yield).
**Step 2. Title compound:** A solution of the product obtained in Step 1 (0.4 g, 1.18 mmol) in THF (12 mL) and 1 M NaOH (12 mL) was heated at 50 °C overnight. The solvent was evaporated and the aqueous phase was washed with Et₂O twice. The basic aqueous phase was acidified to pH 5 with 3 N HCI and it was extracted with DCM. The combined organic phases were washed with brine, dried over MgSO₄, filtered and concentrated to dryness to afford the title compound, which was used without further purification (293 mg, 76% yield).

This method was used for the preparation of Intermediates 6B-6H using suitable starting materials:

| **INT** | **Structure** | **Chemical name** |
|---|---|---|
| **6B** | 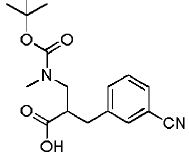 | 3-((*tert-*butoxycarbonyl)(methyl)amino)-2-(3-cyanobenzyl)propanoic acid |
| **6C** | 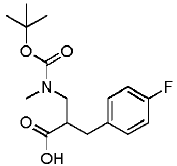 | 3-((*tert-*butoxycarbonyl)(methyl)amino)-2-(4-fluorobenzyl)propanoic acid |
| **6D** | 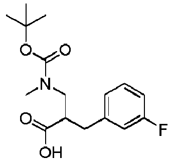 | 3-((*tert-*butoxycarbonyl)(methyl)amino)-2-(3-fluorobenzyl)propanoic acid |
| **6E** | 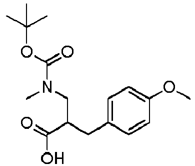 | 3-((*tert-*butoxycarbonyl)(methyl)amino)-2-(4-methoxybenzyl)propanoic acid |
| **6F** | 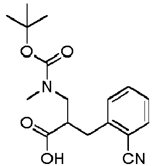 | 3-((*tert-*butoxycarbonyl)(methyl)amino)-2-(2-cyanobenzyl)propanoic acid |
| **6G** | 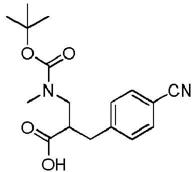 | 3-((*tert-*butoxycarbonyl)(methyl)amino)-2-(4-cyanobenzyl)propanoic acid |
| **6H** | 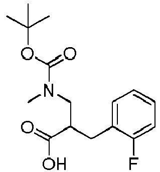 | 3-((*tert-*butoxycarbonyl)(methyl)amino)-2-(2-fluorobenzyl)propanoic acid |

### Intermediate 7: 2-Benzyl-3-((tert-butoxycarbonyl)amino)-2-methylpropanoic acid

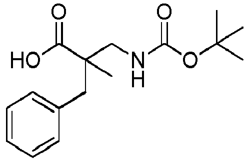

**Step 1. Ethyl 2-cyano-2-methyl-3-phenylpropanoate:** A solution of ethyl 2-cyanopropanoate (1 g, 7.8 mmol), benzyl bromide (0.93 mL, 7.8 mmol) and DBU (1.17 mL, 7.8 mmol) in DMF (10 mL) was stirred at r.t overnight. Water was added to the reaction mixture and it was extracted with EtOAc. The combined organic phases were dried over MgSO₄, filtered and concentrated to dryness to afford the title compound (1.3 g, 77% yield).
**Step 2. Ethyl 2-benzyl-3-((*tert*-butoxycarbonyl)amino)-2-methylpropanoate:** To a solution of the product obtained in Step 1 (1.3 g, 6.0 mmol), cobalt(II) chloride hexahydrate (0.71 g, 3.0 mmol) and di-*tert*-butyl dicarbonate (3.95 g, 18.1 mmol) in MeOH (125 mL), cooled at 0 °C, sodium borohydride (3.19 g, 14 mmol) was added portionwise and the mixture was stirred for 30 min. The solvent was concentrated and the residue was re-dissolved in EtOAc and NaHCO₃ sat. sol. The dark solid formed was filtered through a pad of Celite and discarded. The phases present in the filtrate were separated and the aqueous phase was extracted with EtOAc. The combined organic extracts were washed with brine, dried over MgSO₄, filtered and concentrated to dryness. The residue was purified by flash chromatography, silica gel, gradient CH to EtOAc, to give the title compound (0.68 g, 35% yield).
**Step 3. Title compound:** Following the experimental procedure described in Step 2 of Intermediate 6A, starting from the compound obtained in Step 2 (0.68 g, 2.1 mmol), the title compound was obtained (0.30 g, 50% yield).

### Intermediate 8: 2-(((tert-Butoxycarbonyl)(methyl)amino)methyl)-4-phenylbutanoic acid

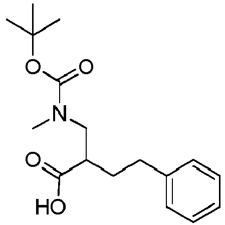

**Step 1. Methyl 2-cyano-4-phenylbutanoate:** To a solution of methyl 2-cyanoacetate (1 g, 10 mmol) and K₂CO₃ (1.4 g, 10 mmol) in DMF (10 mL), (2-bromoethyl)benzene (1.8 g, 10 mmol) was added dropwise and the mixture was stirred at r.t. overnight. Water was added and it was extracted with EtOAc. The combined organic extracts were washed with brine, dried over MgSO₄, filtered and concentrated. The crude product obtained was purified by flash chromatography, C₁₈, gradient NH₄HCO₃ pH 8 to ACN, to give the title compound (0.751 g, 37% yield).
**Step 2. Methyl 2-(((*tert*-butoxycarbonyl)amino)methyl)-4-phenylbutanoate:** Following the experimental procedure described in Step 2 of Intermediate 7, starting from the compound obtained in Step 1, the title compound was obtained (0.754 g, 64% yield).
**Step 3. 2-(((*tert*-Butoxycarbonyl)amino)methyl)-4-phenylbutanoic acid:** Following the experimental procedure described in Step 2 of Intermediate 6A, starting from the compound obtained in Step 2, the title compound was obtained (0.325 g, 45% yield).
**Step 4. Title compound:** Following the experimental procedure described for the preparation of Intermediate 3A, starting from the compound obtained in Step 3, the title compound was obtained (0.265 g, 78% yield).

### Intermediate 9: cis-1-(tert-Butoxycarbonyl)-4-phenylpyrrolidine-3-carboxylic acid

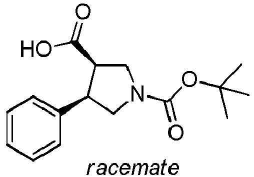

**Step 1. *cis*-1-*tert*-Butyl 3-methyl 4-phenylpyrrolidine-1,3-dicarboxylate:** To a solution of *cis*-methyl 4-phenylpyrrolidine-3-carboxylate (0.5 g, 2 mmol) in EtOAc (5 mL) and 1 M KHCO₃ (3 mL, 3 mmol), cooled at 0 °C, a solution of di-*tert*-butyl dicarbonate (0.45 g, 2 mmol) in EtOAc (4 mL) was added and the mixture was stirred at r.t. overnight. The phases were separated and the aqueous phase was extracted with EtOAc. The combined organic phases were washed with brine, dried over MgSO₄, filtered and concentrated to dryness to afford the title compound, which was used without further purification (0.521 g, 82% yield).
**Step 2. Title compound:** Following the experimental procedure described in Step 2 of Intermediate 6A, starting from the compound obtained in Step 1, the title compound was obtained (0.383 g, 77% yield) as a racemate, relative configuration *cis* as drawn.

### Intermediate 10: trans/trans-2-((tert-Butoxycarbonyl)amino)-5-phenylcyclopentanecarboxylic acid

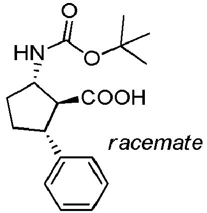

**Step 1. *trans*/*trans*-2-Amino-5-phenylcyclopentanecarboxylic acid:** To a solution of ethyl *trans*/*trans*-2-phenyl-5-(2,2,2-trichloroacetamido)cyclopentanecarboxylate (prepared according to Chemistry - An Asian Journal, 2010, vol. 5, # 5, p. 1112 - 1119; 80 mg, 0.21 mmol) in THF (4.2 mL), cooled at 0 °C, 0.5 M KOH sol. (1.7 mL, 0.84 mmol) was added and it was stirred at r.t. for 2 days. The reaction mixture was acidified to pH 4 with acetic acid. The precipitated solids were filtered, washed with water and dried under vacuum to afford the title compound (as a racemate, relative configuration as drawn) (31 mg, 71% yield).
**Step 2. Title compound:** Following the experimental procedure described in Step 2 of Intermediate 2A, starting from the product obtained in Step 1, the title compound was obtained (as a racemate, relative configuration as drawn) (30 mg, 84% yield).

### Intermediate 11: (1RS,2SR,5SR)-2-((tert-Butoxycarbonyl)amino)-5-phenylcyclopentanecarboxylic acid

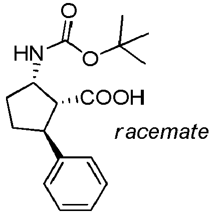

**Step 1. (1*RS*,2*SR*,5*SR*)-Methyl 2-amino-5-phenylcyclopentanecarboxylate:** A mixture of racemic (1*RS*,2*SR*,5*SR*)-methyl 2-(dibenzylamino)-5-phenylcyclopentanecarboxylate (prepared according to Organic & Biomolecular Chemistry, 2005, 3(15), 2762-2775; 269 mg, 0.67 mmol) and palladium hydroxide on carbon (27 mg, 20 wt% on carbon, wet) in MeOH (4 mL) was was stirred under 5 bar of H₂ at r.t. overnight. The catalyst was then filtered through a pad of Celite and it was washed with MeOH. The filtrate was evaporated to dryness to give the title compound (as a racemate, relative configuration as drawn) (140 mg, 95% yield).
**Step 2. (1*RS*,2*SR*,5*SR*)-2-Amino-5-phenylcyclopentane-1-carboxylic acid hydrochloride:** A solution of the product obtained in Step 1 (0.3 g, 1.37 mmol) in 20% wt aq. HCI (20 mL) was heated at 100 °C overnight. After cooling down to r.t., the aqueous phase was washed with EtOAc and it was concentrated to dryness to afford the title compound (as a racemate, relative configuration as drawn) (0.244 g, 74 % yield).
**Step 3. Title compound.** Following the experimental procedure described in Step 2 of Intermediate 2A, starting from the product obtained in Step 2, the title compound was obtained (533 mg, 58% yield), as a racemate, relative configuration as drawn.

### Intermediate 12A: 3-((tert-Butoxycarbonyl)amino)-2-(2,3-dihydro-1H-inden-1-yl)propanoic acid

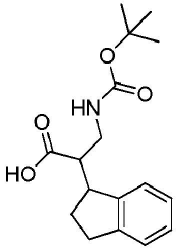

**Step 1. Methyl 2-cyano-2-(2,3-dihydro-1*H*-inden-1-yl)acetate:** To a solution of 1-chloro-2,3-dihydro-1*H*-indene (1 g, 6.55 mmol) in DMF (5.4 mL), cooled at 0 °C, NaH (0.314 g, 7.86 mmol, 60 wt% dispersion in mineral oil) was added portionwise and the mixture was stirred for 30 min at 0 °C. Then, methyl cyanoacetate (0.580 mL, 6.55 mmol) was added and the reaction mixture was stirred at r.t. overnight. The solvent was concentrated and the residue was diluted with DCM and water. The phases were separated and the aqueous phase was extracted with DCM. The combined organic extracts were washed with brine, dried over MgSO₄, filtered and concentrated to dryness. The residue was purified by flash chromatography, silica gel, gradient CH to EtOAc, to give the title compound (0.448 g, 32% yield).
**Step 2. Methyl 3-amino-2-(2,3-dihydro-1*H*-inden-1-yl)propanoate:** A mixture of the product obtained in Step 1 (0.448 g, 2.081 mmol) and Raney-Nickel (slurry in water, 0.122 g) in MeOH (25 mL) was stirred under 3 bar of H₂ at r.t. overnight. The reaction mixture was filtered through a pad of Celite that was washed with MeOH, and the solvent was evaporated. The residue was partitioned between DCM and NaHCO₃ sat. sol. and the aqueous phase was extracted with DCM. The combined organic extracts were washed with brine, dried over MgSO₄, filtered and concentrated to dryness to afford the title compound (0.397 g, 87% yield).
**Step 3. Methyl 3-((*tert*-butoxycarbonyl)amino)-2-(2,3-dihydro-1*H*-inden-1-yl)propanoate.** To a solution of the product obtained in Step 2 (0.397 g, 1.81 mmol) and NaOH (0.290 g, 7.24 mmol) in a mixture of 1,4-dioxane (12 mL) and water (6 mL), di-*tert*-butyl dicarbonate (0.593 g, 2.72 mmol) was added and the mixture was stirred at r.t. overnight. Citric acid (10% aq. sol.) was added to adjust the pH to 3 and it was extracted with EtOAc. The combined organic extracts were dried over MgSO₄, filtered and concentrated to dryness. The residue was purified by flash chromatography, silica gel, gradient CH to EtOAc, to give the title compound (0.155 g, 27% yield).
**Step 4. Title compound:** To a solution of the compound obtained in Step 3 (0.155 g, 0.485 mmol) in a mixture of THF (2.5 mL) and water (2.5 mL), lithium hydroxide monohydrate (0.204 g, 4.85 mmol) was added. The reaction was stirred at r.t. for 3 days. EtOAc was added and the pH was adjusted to 3 with citric acid (10% aq. sol.). The layers were separated and the aqueous phase was extracted with EtOAc. The combined organic extracts were dried over MgSO₄, filtered and evaporated to dryness to obtain the title compound (0.174 g, overweight, quant. yield assumed), as a mixture of diastereomers that was used as such without purification.

This method was used for the preparation of Intermediates 12B-12C using suitable starting materials:

| **INT** | **Structure** | **Chemical name** |
|---|---|---|
| **12B** | 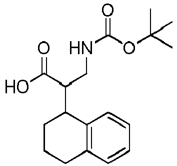 | 3-((*tert*-butoxycarbonyl)amino)-2-(1,2,3,4-tetrahydronaphthalen-1-yl)propanoic acid |
| **12C** | 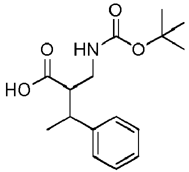 | 2-(((*tert-*butoxycarbonyl)amino)methyl)-3-phenylbutanoic acid |

### Intermediate 13A: 2-(tert-Butoxycarbonyl)-2,3,4,5-tetrahydro-1H-benzo[c]azepine-4-carboxylic acid

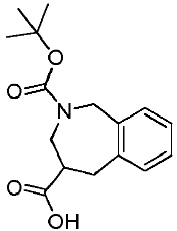

**Step 1. Ethyl 3-amino-2-benzylpropanoate:** A solution of 3-amino-2-benzylpropanoic acid hydrochloride (0.5 g, 2.3 mmol) in a mixture of EtOH (50 mL) and sulfuric acid (0.2 mL) was heated at reflux overnight. The solvent was evaporated and the residue was dissolved in DCM. The organic phase was washed with 1 M NaOH, dried over MgSO₄, filtered and concentrated to dryness to afford the title compound (0.352 g, 73% yield), which was used without further purification.
**Step 2. Ethyl 2-benzyl-3-((methoxycarbonyl)amino)propanoate:** To a solution of the product obtained in Step 1 (0.351 g, 1.7 mmol) in DCM (10 mL), cooled at 0 °C, pyridine (0.54 mL, 6.7 mmol) and methyl chloroformate (0.16 mL, 2 mmol) were added dropwise. The reaction was left to reach r.t. and stirred overnight. Then, cold water was added and it was extracted with DCM. The combined organic extracts were washed with 1 M HCI, water and brine, and finally dried over MgSO₄, filtered and concentrated to dryness to afford the title compound (0.369 g, 81% yield), which was used without further purification.
**Step 3. 4-Ethyl 2-methyl 1,3,4,5-tetrahydro-2*H*-benzo[c]azepine-2,4-dicarboxylate.** To a solution of the product obtained in Step 2 (0.369 g, 1.40 mmol) in a mixture of acetic acid (3.1 mL) and sulfuric acid (1.5 mL), formaldehyde (0.42 mL, 5.56 mmol, 37 wt% aq. sol.) was added and the reaction mixture was stirred at r.t. overnight. Cold water was added and it was extracted with EtOAc. The combined organic extracts were washed with water, dried over MgSO₄, filtered and concentrated to dryness to afford the title compound (0.579 g, overweight, quant. yield assumed), which was used without further purification.
**Step 4. 2,3,4,5-Tetrahydro-1*H*-benzo[c]azepine-4-carboxylic** acid hydrobromide: A solution of the compound obtained in Step 3 (0.265 g, 1.0 mmol) in a mixture of acetic acid/HBr 47 wt% aq. sol (2:1 v/v, 15 mL) was heated at 100 °C for 1.5 h. The solvent was concentrated in vacuo and the crude product was used without further purification (286 mg, quant yield).
**Step 5. Title compound:** Following the experimental procedure described in Step 2 of Intermediate 2A, starting from the product obtained in Step 4, the title compound was obtained (0.2 g, 65% yield).

### Intermediate 13B: 2-((Benzyloxy)carbonyl)-2,3,4,5-tetrahydro-1H-benzo[c]azepine-4-carboxylic acid

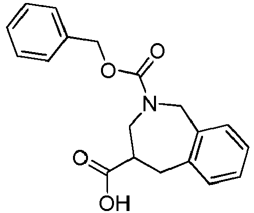

**Step 1. Methyl 2-benzyl-3-(((benzyloxy)carbonyl)amino)propanoate:** Following the experimental procedure described in Step 1 of Intermediate 6A, starting from methyl 3-(((benzyloxy)carbonyl)amino)propanoate (0.85 g, 3.6 mmol) and benzyl bromide, the title compound was obtained (0.54 g, 46% yield).
**Step 2. 2-Benzyl 4-methyl 4,5-dihydro-1*H*-benzo[*c*]azepine-2,4(3*H*)-dicarboxylate:** To a solution of the product obtained in Step 1 (0.54 g, 1.7 mmol) in TFA (0.5 M in DCM, 3.3 mL), cooled at 0 °C, paraformaldehyde (149 mg, 4.95 mmol) was added. The reaction mixture was stirred at 0 °C for 1 h. Then, NaHCO₃ sat.sol. was added and it was extracted with DCM. The combined organic extracts were dried over MgSO₄, filtered and concentrated to dryness to afford the title compound (0.529 g, 94% yield) as a crude product which was used without purification.
**Step 3. Title compound:** Following the experimental procedure described in Step 2 of Intermediate 6A, starting from the product obtained in Step 2 (0.639 g, 1.88 mmol), the desired product was obtained. The crude product was purified by flash chromatography, C₁₈, gradient NH₄HCO₃ pH 8 to ACN, to give the title compound (164 mg, 26% yield).

### Intermediate 14A: (2R,3R)-2-Benzyl-3-((tert-butoxycarbonyl)amino)butanoic acid

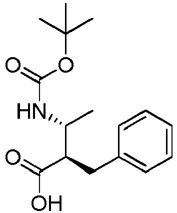

**Step 1. (*R*)-Methyl 3-(2-phenylacetamido)butanoate:** To a solution of (R)-3-aminobutanoic acid (1 g, 9.7 mmol) and NaOH (0.776 g, 19.4 mmol) in water (30 mL), cooled at 0 °C, a solution of 2-phenylacetyl chloride (1.8 g, 11.6 mmol) in acetone (10 mL) was added. The reaction mixture was stirred at r.t. for 1 h. Then, the solvent was evaporated. The aqueous layer was washed with EtOAc (that was discarded) and then it was acidified to pH 1 with 1 M HCl and extracted with EtOAc. The combined organic extracts were dried over MgSO₄, filtered and concentrated to dryness to afford (R)-3-(2-phenylacetamido)butanoic acid (1.75 g, 81% yield). A solution of thionyl chloride (1.4 mL, 19.4 mmol) in MeOH (10 mL) was stirred at -15 °C for 2 h. To this solution, (R)-3-(2-phenylacetamido)butanoic acid was added in one portion. The reaction mixture was left to reach r.t. and stirred for 5 h. The solvent was evaporated and the residue was purified by flash chromatography, silica gel, gradient CH to EtOAc, to give the title compound (1.39 g, 61% yield).
**Step 2. (2*R*,3*R*)-Methyl 2-benzyl-3-(2-phenylacetamido)butanoate:** To a solution of the product obtained in Step 1 (0.86 g, 3.66 mmol) in anh. THF (16 mL), cooled at -78 °C under a nitrogen atmosphere, LDA (5.4 mL, 8 mmol, 1.5 M in THF/Ethylbenzene/Heptane) was added dropwise and the mixture was stirred at -78 °C for 30 min. Benzyl bromide (0.52 mL, 4.4 mmol) was added and the reaction mixture was left to reach r.t. and stirred for further 30 min. NH₄Cl sat. sol. was added and it was extracted with EtOAc. The combined organic phases were washed with brine, dried over Na₂SO₄, filtered and concentrated to dryness. The residue was purified by flash chromatography, silica gel, gradient CH to EtOAc, to give the title compound (0.675 g, 56% yield).
**Step 3. (2*R*,3*R*)-3-Amino-2-benzylbutanoic acid hydrochloride.** In a sealed tube, a solution of the product obtained in Step 2 (0.655 g, 2 mmol) in 6 M HCI (6 mL) was heated at reflux for 2 days. The aqueous phase was washed twice with EtOAc and then it was concentrated to dryness to afford the crude product (0.4 g, 87% yield), which was used without further purification.
**Step 5. Title compound:** Following the experimental procedure described in Step 2 of Intermediate 2A, starting from the product obtained in Step 4, the title compound was obtained (0.356 g, 70% yield).

This method was used for the preparation of Intermediate 14B using suitable starting materials:

| **INT** | **Structure** | **Chemical name** |
|---|---|---|
| **14B** | 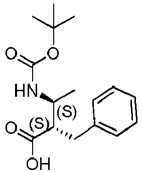 | (2*S*,3*S*)-2-benzyl-3-((*tert-*butoxycarbonyl)amino)butanoic acid |

### Synthesis of Examples

### General Deprotection Methods

**Method 1. Cbz deprotection with Pd(OH)₂.** To a solution of the *N*-Cbz protected compound (1 mmol) in MeOH (20 mL), under a N₂ atmosphere, palladium hydroxide on carbon (20% wt, wet; 20% w/w) was added and the reaction mixture was purged with H₂ and stirred under a H₂ atmosphere (balloon) at r.t. until full conversion. The catalyst was then filtered through a plug of Celite, that was washed thoroughly with MeOH. The filtrate was evaporated to dryness. The crude product was purified by flash chromatography, silica gel, gradient DCM to MeOH:DCM (1:4) to give the title compound.
**Method 2. Boc deprotection with HCl.** To a solution of the *N*-Boc protected compound (1 mmol) in 1,4-dioxane (10 mL), HCl (4 N solution in 1,4-dioxane, 10 mmol) was added under a N₂ atmosphere. The reaction was stirred at r.t. overnight. The solvent was evaporated to render the target compound as the hydrochloride salt. Optionally, the crude product was purified by eluting through an acidic ion exchange resin cartridge (SCX), to give the desired compound as the free base, which could be further purified by flash chromatography, silica gel, gradient DCM to MeOH:DCM (1:4).

### Synthesis of Examples

### Example 1. (2S,3R)-1-(4-(Dimethylamino)-4-phenylpiperidin-1-yl)-2-((methylamino)methyl)-3-phenylbutan-1-one dihydrochloride

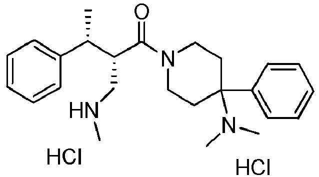

**Step 1. Benzyl ((2*S*,3*R*)-2-(4-(dimethylamino)-4-phenylpiperidine-1-carbonyl)-3-phenylbutyl)(methyl)carbamate:** To a solution of intermediate 3A (0.116 g, 0.38 mmol) and *N*,*N*-dimethyl-4-phenylpiperidin-4-amine dihydrochloride (0.105 g, 0.38 mmol) in anh. DMF (2.70 mL), DIPEA (0.26 mL, 1.51 mmol) and HATU (0.143 g, 0.38 mmol) were added and the reaction mixture was stirred at r.t. overnight. NaHCO₃ sat. sol. was added and it was extracted with EtOAc. The combined organic phases were washed with water and brine, dried over MgSO₄, filtered and concentrated to dryness. The crude product was purified by flash chromatography, silica gel, gradient DCM to MeOH:DCM (1:4) to give the title compound (112 mg, 60% yield).
**Step 2. Title compound:** Starting from the compound obtained in Step 1 (112 mg, 0.23 mmol) and following General Deprotection Method 2, the title compound was obtained (107 mg, quant. yield).
HPLC retention time (method A): 4.24 min; MS: 394.2 (M+H).

Optical purity by chiral HPLC (Chiralpak IC): 98.4% ee
This method was used for the preparation of Examples 2-70 using suitable starting materials:

| **EX** | **Structure** | **Chemical name** | **HPLC Method** | **Ret time (min)** | **MS (M+H)** | **Depro t. Method** |
|---|---|---|---|---|---|---|
| **2** | 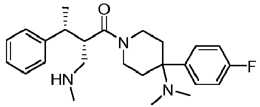 | (2*S*,3*R*)-1-(4-(dimethylamino)-4-(4-fluorophenyl)piperidin-1-yl)-2-((methylamino)methyl)-3-phenylbutan-1-one | A | 4.43 | 412.2 | 1 |
| **3** | 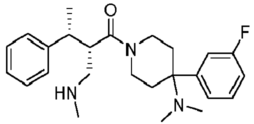 | (2*S*,3*R*)-1-(4-(dimethylamino)-4-(3-fluorophenyl)piperidin-1-yl)-2-((methylamino)methyl)-3-phenylbutan-1-one | A | 4.45 | 412.3 | 1 |
| **4** | 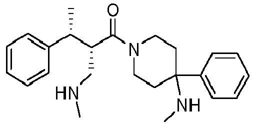 | (2*S*,3*R*)-1-(4-(methylamino)-4-phenylpiperidin-1-yl)-2-((methylamino)methyl)-3-phenylbutan-1-one | A | 4.09 | 380.2 | 1 |
| **5** | 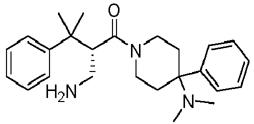 | (*S*)-2-(aminomethyl)-1-(4-(dimethylamino)-4-phenylpiperidin-1-yl)-3-methyl-3-phenylbutan-1-one | A | 4.97 | 394.2 | 1 |
| **6** | 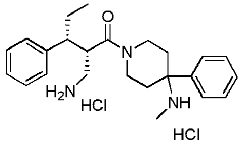 | (2S,3R)-2-(aminomethyl)-1-(4-(methylamino)-4-phenylpiperidin-1-yl)-3-phenylpentan-1-one dihydrochloride | A | 4.29 | 380.2 | 2 |
| **7** | 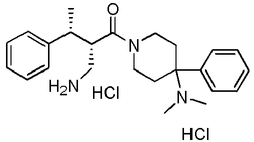 | (2*S*,3*R*)-2-(aminomethyl)-1-(4-(dimethylamino)-4-phenylpiperidin-1-yl)-3-phenylbutan-1-one dihydrochloride | A | 4.07 | 380.2 | 2 |
| **8** | 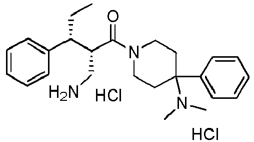 | (2*S*,3*R*)-2-(aminomethyl)-1-(4-(dimethylamino)-4-phenylpiperidin-1-yl)-3-phenylpentan-1-one dihydrochloride | A | 4.31 | 394.2 | 2 |
| **9** | 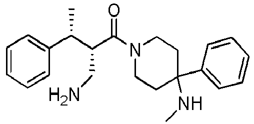 | (2*S*,3*R*)-2-(aminomethyl)-1-(4-(methylamino)-4-phenylpiperidin-1-yl)-3-phenylbutan-1-one | A | 4.15 | 366.2 | 2 |
| **10** | 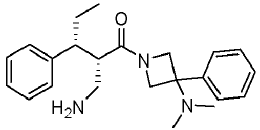 | (2*S*,3*R*)-2-(aminomethyl)-1-(3-(dimethylamino)-3-phenylazetidin-1-yl)-3-phenylpentan-1-one | A | 4.32 | 366.2 | 2 |
| **11** | 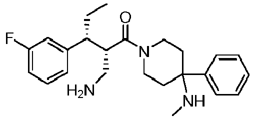 | (2*S*,3*R*)-2-(aminomethyl)-3-(3-fluorophenyl)-1-(4-(methylamino)-4-phenylpiperidin-1-yl)pentan-1-one | A | 4.45 | 398.2 | 2 |
| **12** | 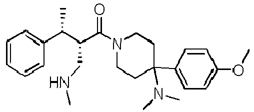 | (2S,3R)-1-(4-(dimethylamino)-4-(4-methoxyphenyl)piperidin-1-yl)-2-((methylamino)methyl)-3-phenylbutan-1-one | A | 4.36 | 424.3 | 1 |
| **13** | 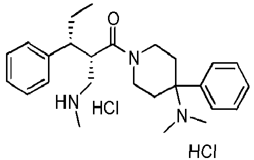 | (2*S*,3*R*)-1-(4-(dimethylamino)-4-phenylpiperidin-1-yl)-2-((methylamino)methyl)-3-phenylpentan-1-one dihydrochloride | A | 4.62 | 408.2 | 2 |
| **14** | 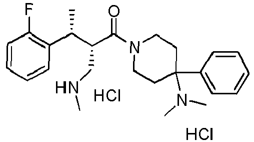 | (2*S*,3*R*)-1-(4-(dimethylamino)-4-phenylpiperidin-1-yl)-3-(2-fluorophenyl)-2-((methylamino)methyl)butan-1-one dihydrochloride | A | 4.58 | 412.2 | 2 |
| **15** | 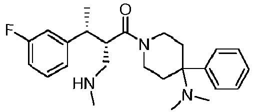 | (2*S*,3*R*)-1-(4-(dimethylamino)-4-phenylpiperidin-1-yl)-3-(3-fluorophenyl)-2-((methylamino)methyl)butan-1-one | A | 4.54 | 412.2 | 2 |
| **16** | 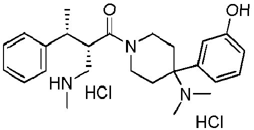 | (2*S*,3*R*)-1-(4-(dimethylamino)-4-(3-hydroxyphenyl)piperidin-1-yl)-2-((methylamino)methyl)-3-phenylbutan-1-one dihydrochloride | A | 3.76 | 410.2 | 2 |
| **17** | 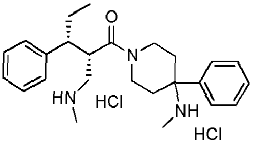 | (2*S*,3*R*)-1-(4-(methylamino)-4-phenylpiperidin-1-yl)-2-((methylamino)methyl)-3-phenylpentan-1-one dihydrochloride | A | 4.58 | 394.2 | 2 |
| **18** | 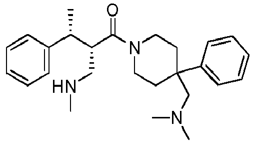 | (2*S*,3*R*)-1-(4-((dimethylamino)methyl)-4-phenylpiperidin-1-yl)-2-((methylamino)methyl)-3-phenylbutan-1-one | A | 4.88 | 408.2 | 2 |
| **19** | 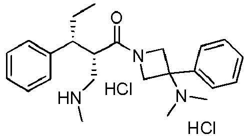 | (2*S*,3*R*)-1-(3-(dimethylamino)-3-phenylazetidin-1-yl)-2-((methylamino)methyl)-3-phenylpentan-1-one dihydrochloride | A | 4.57 | 380.2 | 2 |
| **20** | 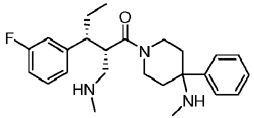 | (2*S*,3*R*)-3-(3-fluorophenyl)-1-(4-(methylamino)-4-phenylpiperidin-1-yl)-2-((methylamino)methyl)pentan-1-one | A | 4.81 | 412.2 | 2 |
| **21** | 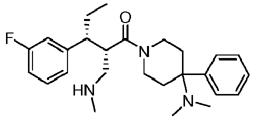 | (2*S*,3*R*)-1-(4-(dimethylamino)-4-phenylpiperidin-1-yl)-3-(3-fluorophenyl)-2-((methylamino)methyl)pentan-1-one | A | 5.06 | 426.2 | 2 |
| **22** | 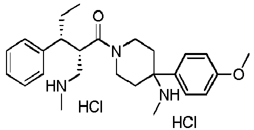 | (2*S*,3*R*)-1-(4-(4-methoxyphenyl)-4-(methylamino)piperidin-1-yl)-2-((methylamino)methyl)-3-phenylpentan-1-one dihydrochloride | A | 4.28 | 424.2 | 2 |
| **23** | 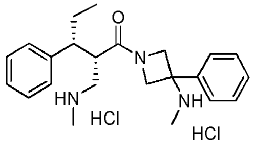 | (2*S*,3*R*)-1-(3-(methylamino)-3-phenylazetidin-1-yl)-2-((methylamino)methyl)-3-phenylpentan-1-one dihydrochloride | A | 4.07 | 366.2 | 2 |
| **24** | 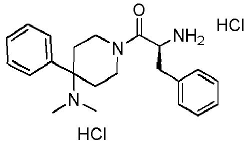 | (*S*)-2-amino-1-(4-(dimethylamino)-4-phenylpiperidin-1-yl)-3-phenylpropan-1-one dihydrochloride | A | 4.05 | 352.2 | 2 |
| **25** | 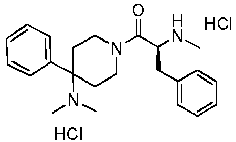 | (*S*)-1-(4-(dimethylamino)-4-phenylpiperidin-1-yl)-2-(methylamino)-3-phenylpropan-1-one dihydrochloride | A | 4.21 | 366.2 | 2 |
| **26** | 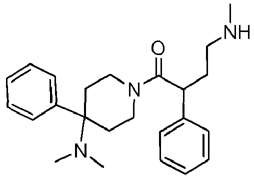 | 1-(4-(dimethylamino)-4-phenylpiperidin-1-yl)-4-(methylamino)-2-phenylbutan-1-one | A | 4.48 | 380.2 | 2 |
| **27** | 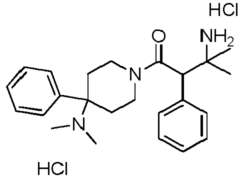 | 3-amino-1-(4-(dimethylamino)-4-phenylpiperidin-1-yl)-3-methyl-2-phenylbutan-1-one dihydrochloride | A | 4.61 | 380.2 | 2 |
| **28** | 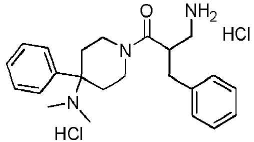 | 3-amino-2-benzyl-1-(4-(dimethylamino)-4-phenylpiperidin-1-yl)propan-1-one dihydrochloride | A | 4.29 | 366.2 | 2 |
| **29** | 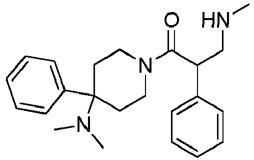 | 1-(4-(dimethylamino)-4-phenylpiperidin-1-yl)-3-(methylamino)-2-phenylpropan-1-one | A | 4.43 | 366.2 | 2 |
| **30** | 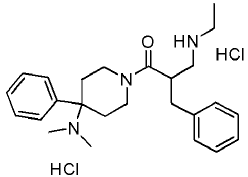 | 2-benzyl-1-(4-(dimethylamino)-4-phenylpiperidin-1-yl)-3-(ethylamino)propan-1-one dihydrochloride | A | 4.69 | 394.2 | 2 |
| **31** | 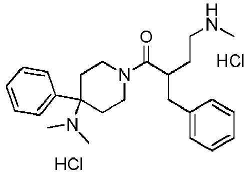 | 2-benzyl-1-(4-(dimethylamino)-4-phenylpiperidin-1-yl)-4-(methylamino)butan-1-one dihydrochloride | A | 4.87 | 394.2 | 2 |
| **32** | 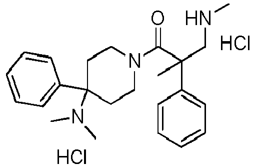 | 1-(4-(dimethylamino)-4-phenylpiperidin-1-yl)-2-methyl-3-(methylamino)-2-phenylpropan-1-one dihydrochloride | A | 4.67 | 380.2 | 2 |
| **33** | 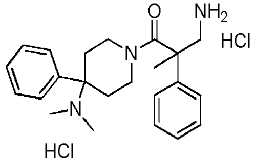 | 3-amino-1-(4-(dimethylamino)-4-phenylpiperidin-1-yl)-2-methyl-2-phenylpropan-1-one dihydrochloride | A | 4.40 | 366.2 | 2 |
| **34** | 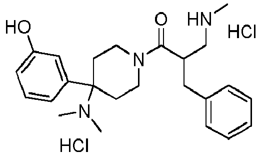 | 2-benzyl-1-(4-(dimethylamino)-4-(3-hydroxyphenyl)piperidin-1-yl)-3-(methylamino)propan-1-one dihydrochloride | A | 4.20 | 396.2 | 2 |
| **35** | 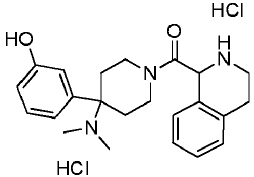 | (4-(dimethylamino)-4-(3-hydroxyphenyl)piperidin-1-yl)(1,2,3,4-tetrahydroisoquinolin-1-yl)methanone dihydrochloride | A | 3.54 | 380.2 | 2 |
| **36** | 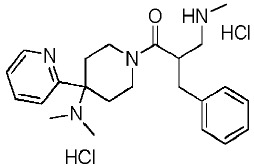 | 2-benzyl-1-(4-(dimethylamino)-4-(pyridin-2-yl)piperidin-1-yl)-3-(methylamino)propan-1-one dihydrochloride | A | 3.53 | 381.2 | 2 |
| **37** | 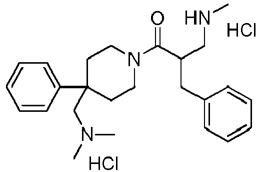 | 2-benzyl-1-(4-((dimethylamino)methyl)-4-phenylpiperidin-1-yl)-3-(methylamino)propan-1-one dihydrochloride | A | 4.39 | 394.2 | 2 |
| **38** | 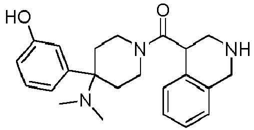 | (4-(dimethylamino)-4-(3-hydroxyphenyl)piperidin-1-yl)(1,2,3,4-tetrahydroisoquinolin-4-yl)methanone | A | 3.31 | 380.2 | 2 |
| **39** | 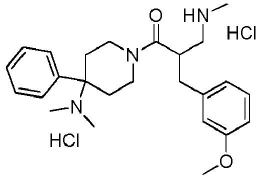 | 1-(4-(dimethylamino)-4-phenylpiperidin-1-yl)-2-(3-methoxybenzyl)-3-(methylamino)propan-1-one dihydrochloride | A | 4.97 | 410.0 | 2 |
| **40** | 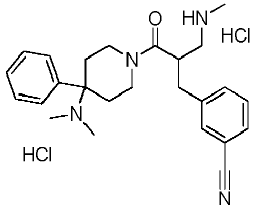 | 3-(3-(4-(dimethylamino)-4-phenylpiperidin-1-yl)-2-((methylamino)methyl)-3-oxopropyl)benzonitrile dihydrochloride | A | 4.79 | 405.0 | 2 |
| **41** | 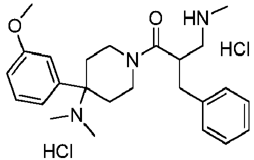 | 2-benzyl-1-(4-(dimethylamino)-4-(3-methoxyphenyl)piperidin-1-yl)-3-(methylamino)propan-1-one dihydrochloride | A | 4.95 | 410.0 | 2 |
| **42** | 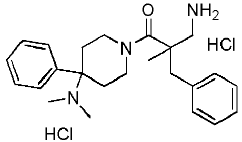 | 3-amino-2-benzyl-1-(4-(dimethylamino)-4-phenylpiperidin-1-yl)-2-methylpropan-1-one dihydrochloride | A | 4.21 | 380.0 | 2 |
| **43** | 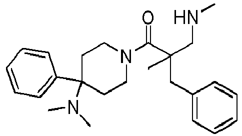 | 2-benzyl-1-(4-(dimethylamino)-4-phenylpiperidin-1-yl)-2-methyl-3-(methylamino)propan-1-one | A | 4.37 | 394.0 | 2 |
| **44** | 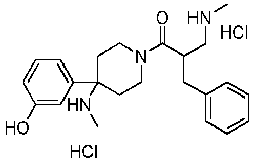 | 2-benzyl-1-(4-(3-hydroxyphenyl)-4-(methylamino)piperidin-1-yl)-3-(methylamino)propan-1-one dihydrochloride | A | 3.38 | 382.0 | 2 |
| **45** | 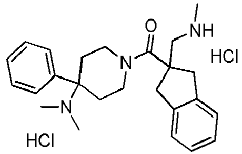 | (4-(dimethylamino)-4-phenylpiperidin-1-yl)(2-((methylamino)methyl)-2,3-dihydro-1*H*-inden-2-yl)methanone dihydrochloride | A | 4.46 | 392.0 | 2 |
| **46** | 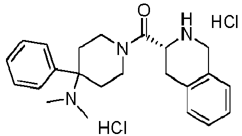 | (*R*)-(4-(dimethylamino)-4-phenylpiperidin-1-yl)(1,2,3,4-tetrahydroisoquinolin-3-yl)methanone dihydrochloride | A | 4.21 | 364.0 | 2 |
| **47** | 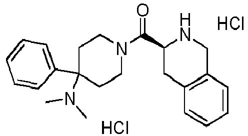 | (*S*)-(4-(dimethylamino)-4-phenylpiperidin-1-yl)(1,2,3,4-tetrahydroisoquinolin-3-yl)methanone dihydrochloride | A | 4.20 | 364.0 | 2 |
| **48** | 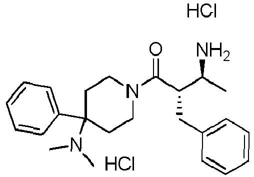 | (2*S*,3*S*)-3-amino-2-benzyl-1-(4-(dimethylamino)-4-phenylpiperidin-1-yl)butan-1-one dihydrochloride | A | 4.30 | 380.0 | 2 |
| **49** | 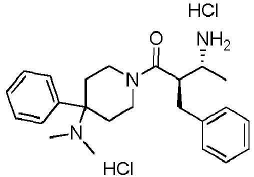 | (2*R*,3*R*)-3-amino-2-benzyl-1-(4-(dimethylamino)-4-phenylpiperidin-1-yl)butan-1-one dihydrochloride | A | 4.27 | 380.0 | 2 |
| **50** | 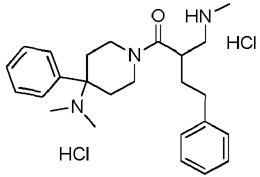 | 1-(4-(dimethylamino)-4-phenylpiperidin-1-yl)-2-((methylamino)methyl)-4-phenylbutan-1-one dihydrochloride | A | 4.43 | 394.0 | 2 |
| **51** | 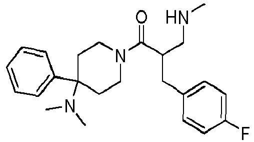 | 1-(4-(dimethylamino)-4-phenylpiperidin-1-yl)-2-(4-fluorobenzyl)-3-(methylamino) propan-1-one | A | 4.36 | 398.0 | 2 |
| **52** | 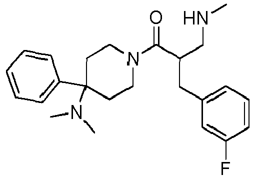 | 1-(4-(dimethylamino)-4-phenylpiperidin-1-yl)-2-(3-fluorobenzyl)-3-(methylamino) propan-1-one | A | 4.22 | 398.0 | 2 |
| **53** | 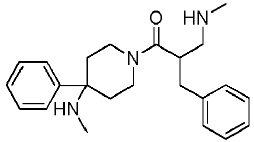 | 2-benzyl-3-(methylamino)-1-(4-(methylamino)-4-phenylpiperidin-1-yl)propan-1-one | A | 4.18 | 366.0 | 2 |
| **54** | 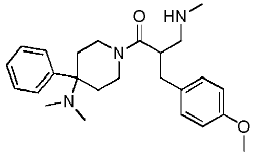 | 1-(4-(dimethylamino)-4-phenylpiperidin-1-yl)-2-(4-methoxybenzyl)-3-(methylamino)propan-1-one | A | 4.40 | 410.2 | 2 |
| **55** | 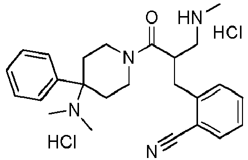 | 2-(3-(4-(dimethylamino)-4-phenylpiperidin-1-yl)-2-((methylamino)methyl)-3-oxopropyl)benzonitrile dihydrochloride | A | 4.19 | 405.2 | 2 |
| **56** | 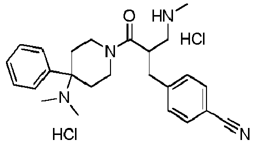 | 4-(3-(4-(dimethylamino)-4-phenylpiperidin-1-yl)-2-((methylamino)methyl)-3-oxopropyl)benzonitrile dihydrochloride | A | 4.13 | 405.2 | 2 |
| **57** | 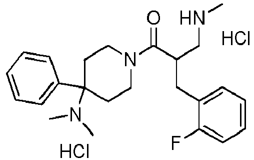 | 1-(4-(dimethylamino)-4-phenylpiperidin-1-yl)-2-(2-fluorobenzyl)-3-(methylamino)propan-1-one dihydrochloride | A | 4.45 | 398.2 | 2 |
| **58** | 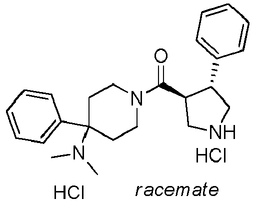 | *trans*-(4-(dimethylamino)-4-phenylpiperidin-1-yl)(4-phenylpyrrolidin-3-yl)methanone dihydrochloride | A | 4.57 | 378.2 | 2 |
| **59** | 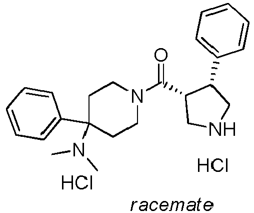 | *cis*-(4-(dimethylamino)-4-phenylpiperidin-1-yl)(4-phenylpyrrolidin-3-yl)methanone dihydrochloride | A | 4.00 | 378.2 | 2 |
| **60** | 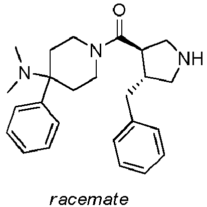 | *trans*-(4-benzylpyrrolidin-3-yl)(4-(dimethylamino)-4-phenylpiperidin-1-yl)methanone | A | 4.27 | 392.3 | 2 |
| **61** | 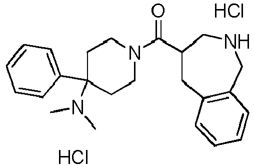 | (4-(dimethylamino)-4-phenylpiperidin-1-yl)(2,3,4,5-tetrahydro-1 *H-*benzo[*c*]azepin-4-yl)methanone dihydrochloride | A | 3.94 | 378.2 | 2 |
| **62** | 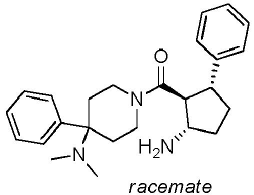 | *trans*/*trans*-(2-amino-5-phenylcyclopentyl)(4-(dimethylamino)-4-phenylpiperidin-1-yl)methanone | A | 4.37 | 392.2 | 2 |
| **63** | 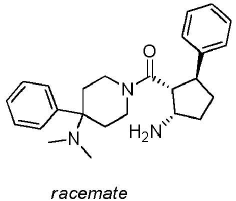 | ((1 *RS*,2*SR*,5*SR*)-2-amino-5-phenylcyclopentyl)(4-(dimethylamino)-4-phenylpiperidin-1-yl)methanone | A | 4.56 | 392.2 | 2 |
| **64** | 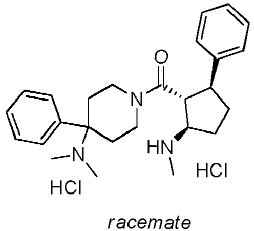 | *trans*/*trans*-(4-(dimethylamino)-4-phenylpiperidin-1-yl)(2-(methylamino)-5-phenylcyclopentyl)methanone dihydrochloride | A | 4.53 | 406.2 | 2 |
| **65** | 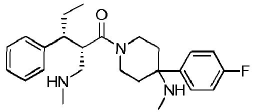 | (2*S*,3*R*)-1-(4-(4-fluorophenyl)-4-(methylamino)piperidin-1-yl)-2-((methylamino)methyl)-3-phenylpentan-1-one | A | 4.57 | 412.3 | 2 |
| **66** | 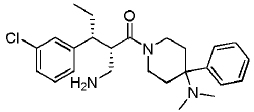 | (2*S*,3*R*)-2-(aminomethyl)-3-(3-chlorophenyl)-1-(4-(dimethylamino)-4-phenylpiperidin-1-yl)pentan-1-one | A | 4.85 | 428.2 | 2 |
| **67** | 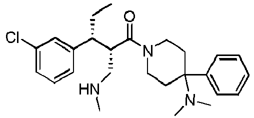 | (2*S*,3*R*)-3-(3-chlorophenyl)-1-(4-(dimethylamino)-4-phenylpiperidin-1-yl)-2-((methylamino)methyl)pentan-1-one | A | 5.26 | 442.2 | 2 |
| **68** | 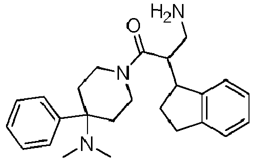 | 3-amino-2-(2,3-dihydro-1 *H-*inden-1-yl)-1-(4-(dimethylamino)-4-phenylpiperidin-1-yl)propan-1-one | A | 4.45 | 392.2 | 2 |
| **69** | 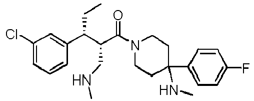 | (2*S*,3*R*)-3-(3-chlorophenyl)-1-(4-(4-fluorophenyl)-4-(methylamino)piperidin-1-yl)-2-((methylamino)methyl)pentan-1-one | A | 5.08 | 446.2 | 2 |
| **70** | 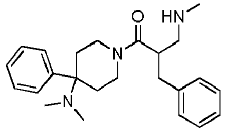 | 2-benzyl-1-(4-(dimethylamino)-4-phenylpiperidin-1-yl)-3-(methylamino)propan-1-one | B | 2.44 | 380.2 | 2 |

### Example 71. 2-Benzyl-1-(4-(dimethylamino)-4-phenylpiperidin-1-yl)-3-(phenethylamino)propan-1-one

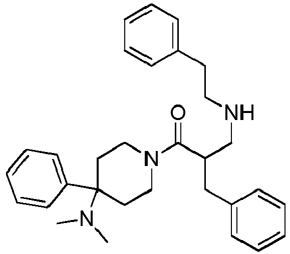

A solution of Example 28 (182 mg, 0.36 mmol) and 2-phenylacetaldehyde (43 mg, 0.36 mmol) in DCE (1 mL) was stirred at r.t. for 30 min. Then, sodium cyanoborohydride (45 mg, 0.72 mmol) was added in one portion and the reaction mixture was stirred at r.t. overnight. NaHCO₃ sat. sol. was added and the phases were separated. The aq. phase was extracted with DCM. The combined organic extracts were washed with brine, dried over MgSO₄, filtered and concentrated to dryness. The crude product was purified by flash chromatography, silica gel, gradient DCM to MeOH:DCM (1:4) and then repurified by flash chromatography, C₁₈, gradient NH₄HCO₃ pH 8 to ACN, to give the title compound (11 mg, 6% yield).
HPLC retention time (method A): 5.52 min; MS: 470.3 (M+H).

This method was used for the preparation of Examples 72-73 using suitable starting materials:

| **EX** | **Structure** | **Chemical name** | **HPLC Method** | **Ret time (min)** | **MS (M+H)** |
|---|---|---|---|---|---|
| **72** | 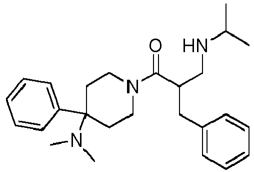 | 2-benzyl-1-(4-(dimethylamino)-4-phenylpiperidin-1-yl)-3-(isopropylamino)propan-1-one | A | 4.56 | 408.3 |
| **73** | 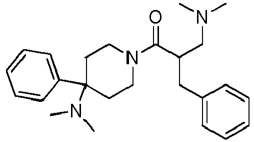 | 2-benzyl-3-(dimethylamino)-1-(4-(dimethylamino)-4-phenylpiperidin-1-yl)propan-1-one | B | 2.57 | 394.2 |

### Example 74. trans-(1,4-Dibenzylpyrrolidin-3-yl)(4-(dimethylamino)-4-phenylpiperidin-1-yl)methanone

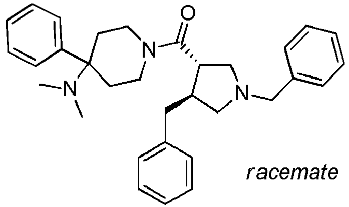

Following the experimental procedure described in Step 1 of Example 1, starting from *trans*-1,4-dibenzylpyrrolidine-3-carboxylic acid (50 mg, 0.17 mmol), the title compound was obtained (31 mg, 38% yield).
HPLC retention time (method A): 5.98 min; MS: 482.3 (M+H).

### Example 75. 3-Amino-1-(4-(dimethylamino)-4-phenylpiperidin-1-yl)-2-(2-methylbenzyl)propan-1-one dihydrochloride

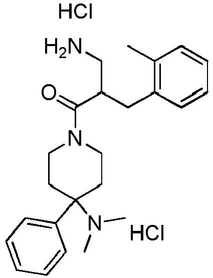

**Step 1. Benzyl 4-(4-(dimethylamino)-4-phenylpiperidine-1-carbonyl)-4,5-dihydro-1*H*-benzo[c]azepine-2(3*H*)-carboxylate:** Following the experimental procedure described for the preparation of Example 1 Step 1, starting from Intermediate 13B (117 mg, 0.36 mmol), the title compound was obtained (56 mg, 30% yield).
**Step 2. Title compound:** To a solution of the product obtained in Step 1 (56 mg, 0.11 mmol) and HCI (1 M in Et₂O, 0.22 mL, 0.22 mmol) in MeOH (3.0 mL), under a N₂ atmosphere, palladium on carbon (10 wt%, wet, 6 mg) was added and the reaction mixture was purged with H₂ and stirred under a H₂ atmosphere (balloon) at r.t. overnight. The catalyst was then filtered through a plug of Celite that was washed thoroughly with MeOH. The filtrate was evaporated to dryness to give the title compound (49 mg, 99% yield).
HPLC retention time (method A): 4.19 min; MS: 380.0 (M+H).

### Example 76. 2-Benzyl-3-(benzylamino)-1-(4-(dimethylamino)-4-phenylpiperidin-1-yl)propan-1-one dihydrochloride:

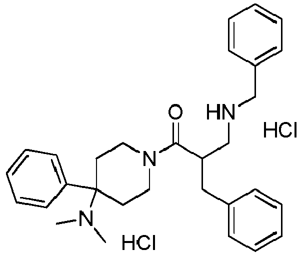

**Step 1. *tert*-Butyl benzyl(2-benzyl-3-(4-(dimethylamino)-4-phenylpiperidin-1-yl)-3-oxopropyl)carbamate:** To a solution of the product obtained in Step 1 of Example 28 (78 mg, 0.17 mmol) in THF (3 mL), cooled at 0 °C under a N₂ atmosphere, NaH (10 mg, 0.25 mmol, 60 wt% dispersion in mineral oil) was added. The suspension was stirred at this temperature for 30 min and then benzyl bromide (18 µL, 0.15 mmol) was added. The reaction mixture was allowed to warm to r.t. overnight. Then, NH₄Cl sat. sol. was added and the aq. phase was extracted with EtOAc. The combined organic extracts were washed with brine, dried over MgSO₄, filtered and concentrated to dryness. The residue was purified by flash chromatography, silica gel, gradient DCM to MeOH:DCM (1:4) to give the title compound (69 mg, 74% yield).
**Step 2. Title compound:** Starting from the compound obtained in Step 1 (69 mg, 0.12 mmol) and following General Deprotection Method 2, the title compound was obtained.
HPLC retention time (method A): 5.30 min; MS: 456.3 (M+H).

### Examples 77 and 78. (RS)-3-Amino-1-(4-(dimethylamino)-4-phenylpiperidin-1-yl)-2-((SR)-1,2,3,4-tetrahydronaphthalen-1-yl)propan-1-one and (RS)-3-amino-1-(4-(dimethylamino)-4-phenylpiperidin-1-yl)-2-((RS)-1,2,3,4-tetrahydronaphthalen-1-yl)propan-1-one (racemates)

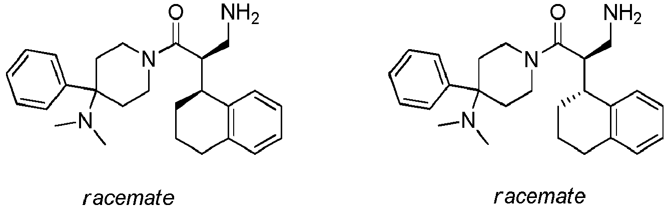

**Step 1. 3-Amino-1-(4-(dimethylamino)-4-phenylpiperidin-1-yl)-2-(1,2,3,4-tetrahydronaphthalen-1-yl)propan-1-one:** Following the experimental procedure described in Step 1 of Example 1, starting from Intermediate 12B (73 mg, 0.23 mmol), the title compound was obtained (79 mg, 68% yield) as 1:1 mixture of diastereomers (each one as a racemate).
**Step 2. Title compounds:** Starting from the diastereomeric mixture of compounds obtained in Step 1 (79 mg, 0.156 mmol) and following General Deprotection Method 2, the title compounds were obtained as a 1:1 mixture of diastereomers (each one as a racemate). The mixture was dissolved in EtOAc and it was washed with 1 M NaOH. The organic phase was dried over MgSO₄, filtered and concentrated to dryness. The residue was purified by silica gel, isocratic CHCl₃/ 5% EtOH / 1% NH₃ to give the title compounds as pure diastereomers (each one as a racemate).
   Example 77 (7.5 mg, 12% yield)
      HPLC retention time (method A): 4.78 min; MS: 406.2 (M+H).
   Example 78 (7.5 mg, 12% yield)
      HPLC retention time (method A): 4.88 min; MS: 406.2 (M+H).

### Examples 79 and 80. (S)-2-Benzyl-1-(4-(dimethylamino)-4-phenylpiperidin-1-yl)-3-(methylamino)propan-1-one dihydrochloride and (R)-2-benzyl-1-(4-(dimethylamino)-4-phenylpiperidin-1-yl)-3-(methylamino)propan-1-one dihydrochloride:

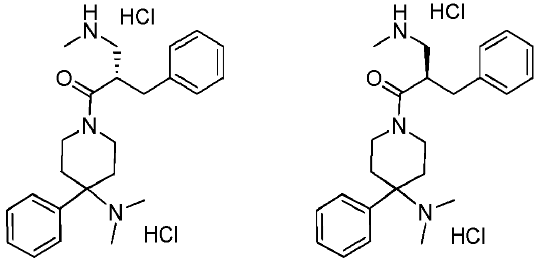

**Step 1. (*S*)-*tert*-Butyl (2-benzyl-3-(4-(dimethylamino)-4-phenylpiperidin-1-yl)-3-oxopropyl)(methyl)carbamate and (*R*)-*tert*-butyl (2-benzyl-3-(4-(dimethylamino)-4-phenylpiperidin-1-yl)-3-oxopropyl)(methyl)carbamate:** Starting from Step 1 of Example 70, a chiral preparative HPLC separation (column: Chiralpak IC; temperature: ambient; flow: 11 mL/min; eluent: n-Heptane/(EtOH + 0.33% DEA) 70/30 v/v) was carried out to give the title compounds.
**Step 2. Title compounds:** Starting from the enantiopure compounds obtained in Step 1 and following General Deprotection Method 2, the title compounds were obtained.
   Example 79: HPLC retention time (method A): 4.03 min; MS: 380.2 (M+H).
   Example 80: HPLC retention time (method A): 4.13 min; MS: 380.2 (M+H).

### Examples 81, 82, 83 and 84. (R)-3-amino-2-((S)-2,3-dihydro-1H-inden-1-yl)-1-(4-(dimethylamino)-4-phenylpiperidin-1-yl)propan-1-one, (S)-3-amino-2-((R)-2,3-dihydro-1H-inden-1-yl)-1-(4-(dimethylamino)-4-phenylpiperidin-1-yl)propan-1-one, (R)-3-amino-2-((R)-2,3-dihydro-1H-inden-1-yl)-1-(4-(dimethylamino)-4-phenylpiperidin-1-yl)propan-1-one and (S)-3-amino-2-((S)-2,3-dihydro-1H-inden-1-yl)-1-(4-(dimethylamino)-4-phenylpiperidin-1-yl)propan-1-one:

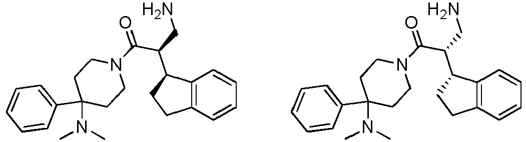

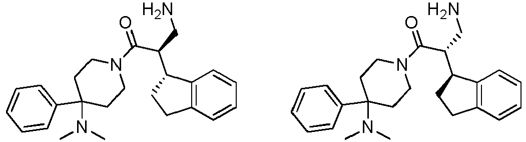

Starting from Example 68, a chiral preparative HPLC separation (column: Chiralpak IC; temperature: ambient; flow: 14 mL/min; eluent: n-Heptane/(EtOH + 0.33% DEA) 80/20 v/v) was carried out to give the title compounds.
Example 81: HPLC retention time (method B):1.80 min; MS: 392.3 (M+H).
Example 82: HPLC retention time (method B):1.79 min; MS: 392.3 (M+H).
Example 83: HPLC retention time (method B): 1.81 min; MS: 392.3 (M+H).
Example 84: HPLC retention time (method B): 1.81 min; MS: 392.3 (M+H).

### Examples 85, 86 and 87. (2S,3S)-1-(4-(Dimethylamino)-4-phenylpiperidin-1-yl)-2-((methylamino)methyl)-3-phenylbutan-1-one, (2R,3R)-1-(4-(dimethylamino)-4-phenylpiperidin-1-yl)-2-((methylamino)methyl)-3-phenylbutan-1-one and (2R,3S)-1-(4-(dimethylamino)-4-phenylpiperidin-1-yl)-2-((methylamino)methyl)-3-phenylbutan-1-one:

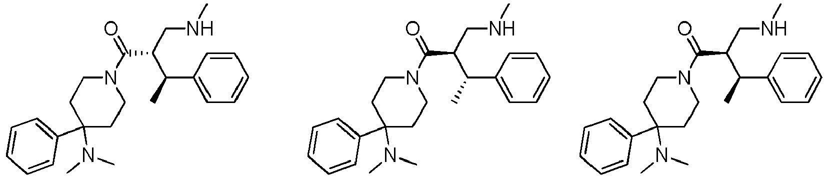

**Step 1. *tert*-Butyl (2-(4-(dimethylamino)-4-phenylpiperidine-1-carbonyl)-3-phenylbutyl)(methyl)carbamate:** Following the experimental procedure described in Step 1 of Example 1, starting from Intermediate 3P (420 mg, 1.16 mmol), the title compound was obtained (368 mg, 64% yield) as a mixture of diastereomers.
**Step 2. 1-(4-(Dimethylamino)-4-phenylpiperidin-1-yl)-2-((methylamino)methyl)-3-phenylbutan-1-one:** Starting from the diastereomeric mixture obtained in Step 1 and following General Deprotection Method 2, the title compound was obtained (235 mg, 80% yield) as a mixture of diastereomers.
**Step 3. Title compounds:** Starting from the diastereomeric mixture obtained in Step 2, a chiral preparative HPLC separation (column: Chiralpak IC; temperature: ambient; flow: 12 mL/min; eluent: n-Heptane/(EtOH + 0.33% DEA) 90/10 v/v) was carried out, followed by a second chiral preparative HPLC purification (column: Chiralcel ADH; temperature: ambient; flow: 14 mL/min; eluent: n-Heptane/(IPA + 0.33% DEA) 95/5 v/v), to give the title compounds together with Example 1.
   Example 85: HPLC retention time (method B): 1.78 min; MS: 394.3 (M+H).
   Example 86: HPLC retention time (method B): 1.78 min; MS: 394.3 (M+H).
   Example 87: HPLC retention time (method B): 1.75 min; MS: 394.3 (M+H).

### Examples of biological activity

This invention is aimed at providing a series of compounds which show pharmacological activity towards the subunit α2δ of voltage-gated calcium channels (VGCC), especially the α2δ-1 subunit of voltage-gated calcium channels or dual activity towards the subunit α2δ of voltage-gated calcium channels (VGCC), especially the α2δ-1 subunit of voltage-gated calcium channels, and the µ-opiod receptor (MOR or mu-opioid receptor) and especially compounds which have a binding expressed as Kᵢ responding to the following scales:
Kᵢ(α₂δ-1) is preferably < 10000 nM, more preferably < 5000 nM, or even more preferably < 500 nM.
Kᵢ(µ) is preferably < 1000 nM, more preferably < 500 nM, even more preferably < 100 nM.

### Binding assay to human α2δ-1 subunit of Cav2.2 calcium channel.

Human α2δ-1 enriched membranes (2.5 µg) were incubated with 15 nM of radiolabeled [3H]-Gabapentin in assay buffer containing Hepes-KOH 10 mM, pH 7.4.

NSB (non specific binding) was measured by adding 10 µM pregabalin. The binding of the test compound was measured at either one concentration (% inhibition at 1 or 10 µM) or five different concentrations to determine affinity values (Ki). After 60 min incubation at 27 °C, binding reaction was terminated by filtering through Multiscreen GF/C (Millipore) presoaked in 0.5 % polyethyleneimine in Vacuum Manifold Station, followed by 3 washes with ice-cold filtration buffer containing 50 mM Tris-HCI, pH 7.4.

Filter plates were dried at 60 °C for 1 h and 30µl of scintillation cocktail were added to each well before radioactivity reading.

Readings were performed in a Trilux 1450 Microbeta radioactive counter (Perkin Elmer).

### Binding assay to human µ-opioid receptor

Transfected CHO-K1 cell membranes (20 µg) were incubated with [³H]-DAMGO (1 nM) in assay buffer containing Tris-HCl 50 mM, MgCl₂ 5 mM at pH 7.4.

NBS (non-specific binding) was measured by adding 10 µM naloxone. The binding of the test compound was measured at either one concentration (% inhibition at 1 or 10 µM) or five different concentrations to determine affinity values (Ki). Plates were incubated at 27 °C for 60 min. After the incubation period, the reaction mixture was then transferred to MultiScreen HTS, FC plates (Millipore), filtered and plates were washed 3 times with ice-cold 10 mM Tris-HCl (pH 7.4).

Filters were dried and counted at approximately 40% efficiency in a MicroBeta scintillation counter (Perkin-Elmer) using EcoScint liquid scintillation cocktail.

### Results:

The following scale has been adopted for representing the binding to the α₂δ-1 subunit of voltage-gated calcium channels expressed as Kᵢ:
+ Kᵢ(α₂δ-1) >= 5000 nM
++ 500nM <= Kᵢ(α₂δ-1) <5000 nM
+++ Kᵢ(α₂δ-1) <500 nM

Preferably, when Kᵢ(α₂δ-1) > 5000 nM, the following scale has been adopted for representing the binding to the α₂δ-1 subunit of voltage-gated calcium channels:
+ Kᵢ(α₂δ-1) > 5000 nM or inhibition ranges between 1 % and 50 %

The following scale has been adopted for representing the binding to µ-opioid receptor expressed as Kᵢ:
+ Kᵢ (µ) >= 500 nM
++ 100 nM <= Kᵢ(µ) < 500 nM
+++ Kᵢ(µ) < 100 nM

Preferably, when Kᵢ (µ) > 500 nM, the following scale has been adopted for representing the binding to the µ -receptor:
+ Kᵢ (µ) > 500 nM or inhibition ranges between 1% and 50 %.

α₂δ-1 binding results of the compounds prepared in the present invention are shown in Table 1:

**Table 1**

| **Example** | **Binding α2δ-1** |
|---|---|
| 1 | +++ |
| 2 | +++ |
| 3 | +++ |
| 4 | +++ |
| 5 | ++ |
| 6 | +++ |
| 7 | +++ |
| 8 | +++ |
| 9 | +++ |
| 10 | +++ |
| 11 | +++ |
| 12 | +++ |
| 13 | +++ |
| 14 | +++ |
| 15 | +++ |
| 16 | +++ |
| 17 | +++ |
| 18 | +++ |
| 19 | ++ |
| 20 | +++ |
| 21 | +++ |
| 22 | +++ |
| 23 | +++ |
| 28 | ++ |
| 30 | ++ |
| 31 | ++ |
| 34 | ++ |
| 35 | + |
| 41 | ++ |
| 44 | ++ |
| 48 | + |
| 49 | + |
| 52 | ++ |
| 55 | ++ |
| 57 | ++ |
| 65 | +++ |
| 66 | +++ |
| 67 | +++ |
| 69 | +++ |
| 70 | ++ |
| 75 | ++ |
| 76 | ++ |
| 77 | ++ |
| 78 | ++ |
| 79 | ++ |
| 82 | ++ |
| 85 | + |
| 86 | +++ |
| 87 | + |

Further, the results of the binding for the α₂δ-1 and the µ-opioid receptor are shown in Table 2:

**Table 2**

| **Example** | **Binding α2δ-1** | **Binding µ** |
|---|---|---|
| 1 | +++ | +++ |
| 2 | +++ | +++ |
| 3 | +++ | +++ |
| 4 | +++ | ++ |
| 5 | ++ | +++ |
| 6 | +++ | +++ |
| 7 | +++ | +++ |
| 8 | +++ | +++ |
| 9 | +++ | +++ |
| 10 | +++ | + |
| 11 | +++ | +++ |
| 12 | +++ | +++ |
| 13 | +++ | +++ |
| 14 | +++ | ++ |
| 15 | +++ | +++ |
| 16 | +++ | +++ |
| 17 | +++ | +++ |
| 19 | ++ | + |
| 20 | +++ | +++ |
| 21 | +++ | +++ |
| 22 | +++ | +++ |
| 28 | ++ | +++ |
| 30 | ++ | +++ |
| 31 | ++ | ++ |
| 34 | ++ | +++ |
| 35 | + | +++ |
| 41 | ++ | +++ |
| 44 | ++ | +++ |
| 48 | + | +++ |
| 49 | + | ++ |
| 52 | ++ | +++ |
| 55 | ++ | ++ |
| 57 | ++ | +++ |
| 65 | +++ | +++ |
| 66 | +++ | +++ |
| 67 | +++ | +++ |
| 69 | +++ | +++ |
| 70 | ++ | ++ |
| 75 | ++ | ++ |
| 76 | ++ | +++ |
| 77 | ++ | +++ |
| 78 | ++ | +++ |
| 79 | ++ | +++ |
| 85 | + | ++ |
| 86 | +++ | +++ |
| 87 | + | ++ |

## Claims

1. A compound of formula (I): 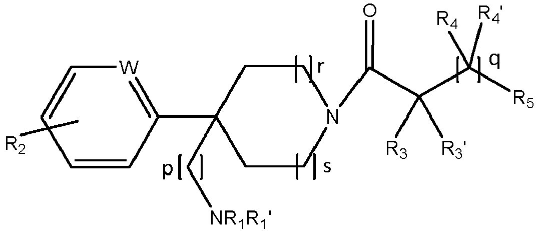 wherein:
W is N or CH;
R₁ and R₁' are independently selected from the group consisting of hydrogen, substituted or unsubstituted C₁₋₆ alkyl, substituted or unsubstituted C₂₋₆alkenyl, and substituted or unsubstituted C₂₋₆ alkynyl;
R₂ is absent or is selected from the group consisting of halogen, substituted or unsubstituted C₁₋₆ alkyl, substituted or unsubstituted C₂₋₆ alkenyl, substituted or unsubstituted C₂₋₆ alkynyl, OR₂', -SR₂', -SOR₂', -SO₂R₂', -CN, -COR₂', -COOR₂',-NR₂'R₂", -CONR₂'R₂" and haloalkoxy;
wherein R₂'and R₂" are independently selected from the group consisting of hydrogen and substituted or unsubstituted C₁₋₆ alkyl;
R₃, R₃', R₄ and R₄' are independently selected from the group consisting of hydrogen, substituted or unsubstituted C₁₋₆ alkyl, substituted or unsubstituted C₂₋₆ alkenyl, substituted or unsubstituted C₂₋₆ alkynyl and (CR₆R₆')ₐ-NR₇R₇'; wherein one of R₃ or R₄ is (CR₆R₆')ₐ-NR₇R₇';
wherein
R₆ and R₆' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl;
R₇ and R₇' are independently selected from the group consisting of hydrogen, substituted or unsubstituted C₁₋₆ alkyl, substituted or unsubstituted C₂₋₆ alkenyl, and substituted or unsubstituted C₂₋₆ alkynyl, substituted or unsubstituted aryl, and substituted or unsubstituted C₁₋₆ alkylaryl; and
a is 0, 1 or 2;
alternatively,
R₃', R₄ or R₇ forms a cycle with R₅; or
R₇ forms a cycle with R₄; or
R₆ forms a cycle with R₄, wherein said cycle is a substituted cycloalkyl being substituted by -NR₇R₇';
R₅ is selected from the group consisting of substituted or unsubstituted aryl, substituted or unsubstituted heterocyclyl and substituted or unsubstituted C₄₋₁₀-cycloalkyl;
p is 0, 1 or 2;
q is 0, 1 or 2;
r is 0, 1 or 2; and
s is 0, 1 or 2;
wherein the compound of formula (I) is optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Compound according to claim 1, wherein R₂ is absent or selected from the group consisting of halogen and OR₂' and wherein R₂' is selected from the group consisting of hydrogen and unsubstituted C₁₋₆ alkyl.

3. Compound according to any one of claims 1 to 2, wherein R₁ and R₁' are selected from the group consisting of hydrogen and unsubstituted C₁₋₆ alkyl.

4. Compound according to any one of claims 1 to 3, wherein R₃', R₄ or R₇ forms a cycle with R₅.

5. Compound according to any one of claims 1 to 3, wherein R₇ forms a cycle with R₄.

6. Compound according to any one of claims 1 to 3, wherein R₆ forms a cycle with R₄, wherein said cycle is a substituted cycloalkyl being substituted by -NR₇R₇'.

7. Compound according to any one of claims 1 to 6, wherein R₅ is substituted or unsubstituted aryl.

8. Compound according to claim 7, wherein when R₅ is substituted aryl the substituents are selected from the group consisting of halogen, unsubstituted C₁₋₆ alkyl, -OR', -SR', -SOR', -SO₂R', -CN, -COR', -COOR', -NR'R", -CONR'R", haloalkyl, and haloalkoxy, wherein each of R' and R" is independently selected from the group consisting of hydrogen, and unsubstituted C₁₋₆ alkyl.

9. Compound according to any one of claims 1 to 8 wherein said compound is selected from the group consisting of:
| | |
|---|---|
| [1] | (2*S*,3*R*)-1-(4-(dimethylamino)-4-phenylpiperidin-1-yl)-2-((methylamino)methyl)-3-phenylbutan-1-one dihydrochloride; |
| [2] | (2*S*,3*R*)-1-(4-(dimethylamino)-4-(4-fluorophenyl)piperidin-1-yl)-2-((methylamino)methyl)-3-phenylbutan-1-one; |
| [3] | (2*S*,3*R*)-1-(4-(dimethylamino)-4-(3-fluorophenyl)piperidin-1-yl)-2-((methylamino)methyl)-3-phenylbutan-1-one; |
| [4] | (2*S*,3*R*)-1-(4-(methylamino)-4-phenylpiperidin-1-yl)-2-((methylamino)methyl)-3-phenylbutan-1-one; |
| [5] | (*S*)-2-(aminomethyl)-1-(4-(dimethylamino)-4-phenylpiperidin-1-yl)-3-methyl-3-phenylbutan-1-one; |
| [6] | (2S,3R)-2-(aminomethyl)-1-(4-(methylamino)-4-phenylpiperidin-1-yl)-3-phenylpentan-1-one dihydrochloride; |
| [7] | (2*S*,3*R*)-2-(aminomethyl)-1-(4-(dimethylamino)-4-phenylpiperidin-1-yl)-3-phenylbutan-1-one dihydrochloride; |
| [8] | (2*S*,3*R*)-2-(aminomethyl)-1-(4-(dimethylamino)-4-phenylpiperidin-1-yl)-3-phenylpentan-1-one dihydrochloride; |
| [9] | (2*S*,3*R*)-2-(aminomethyl)-1-(4-(methylamino)-4-phenylpiperidin-1-yl)-3-phenylbutan-1-one; |
| [10] | (2S,3R)-2-(aminomethyl)-1-(3-(dimethylamino)-3-phenylazetidin-1-yl)-3-phenylpentan-1-one; |
| [11] | (2*S*,3*R*)-2-(aminomethyl)-3-(3-fluorophenyl)-1-(4-(methylamino)-4-phenylpiperidin-1-yl)pentan-1-one; |
| [12] | (2*S*,3*R*)-1-(4-(dimethylamino)-4-(4-methoxyphenyl)piperidin-1-yl)-2-((methylamino)methyl)-3-phenylbutan-1-one; |
| [13] | (2*S*,3*R*)-1-(4-(dimethylamino)-4-phenylpiperidin-1-yl)-2-((methylamino)methyl)-3-phenylpentan-1-one dihydrochloride; |
| [14] | (2*S*,3*R*)-1-(4-(dimethylamino)-4-phenylpiperidin-1-yl)-3-(2-fluorophenyl)-2-((methylamino)methyl)butan-1-one dihydrochloride; |
| [15] | (2S,3R)-1-(4-(dimethylamino)-4-phenylpiperidin-1-yl)-3-(3-fluorophenyl)-2-((methylamino)methyl)butan-1-one; |
| [16] | (2*S*,3*R*)-1-(4-(dimethylamino)-4-(3-hydroxyphenyl)piperidin-1-yl)-2-((methylamino)methyl)-3-phenylbutan-1-one dihydrochloride; |
| [17] | (2*S*,3*R*)-1-(4-(methylamino)-4-phenylpiperidin-1-yl)-2-((methylamino)methyl)-3-phenylpentan-1-one dihydrochloride; |
| [18] | (2*S*,3*R*)-1-(4-((dimethylamino)methyl)-4-phenylpiperidin-1-yl)-2-((methylamino)methyl)-3-phenylbutan-1-one; |
| [19] | (2*S*,3*R*)-1-(3-(dimethylamino)-3-phenylazetidin-1-yl)-2-((methylamino)methyl)-3-phenylpentan-1-one dihydrochloride; |
| [20] | (2*S*,3*R*)-3-(3-fluorophenyl)-1-(4-(methylamino)-4-phenylpiperidin-1-yl)-2-((methylamino)methyl)pentan-1-one; |
| [21] | (2*S*,3*R*)-1-(4-(dimethylamino)-4-phenylpiperidin-1-yl)-3-(3-fluorophenyl)-2-((methylamino)methyl)pentan-1-one; |
| [22] | (2*S*,3*R*)-1-(4-(4-methoxyphenyl)-4-(methylamino)piperidin-1-yl)-2-((methylamino)methyl)-3-phenylpentan-1-one dihydrochloride; |
| [23] | (2*S*,3*R*)-1-(3-(methylamino)-3-phenylazetidin-1-yl)-2-((methylamino)methyl)-3-phenylpentan-1-one dihydrochloride; |
| [24] | (*S*)-2-amino-1-(4-(dimethylamino)-4-phenylpiperidin-1-yl)-3-phenylpropan-1-one dihydrochloride; |
| [25] | (*S*)-1-(4-(dimethylamino)-4-phenylpiperidin-1-yl)-2-(methylamino)-3-phenylpropan-1-one dihydrochloride; |
| [26] | 1-(4-(dimethylamino)-4-phenylpiperidin-1-yl)-4-(methylamino)-2-phenylbutan-1-one; |
| [27] | 3-amino-1-(4-(dimethylamino)-4-phenylpiperidin-1-yl)-3-methyl-2-phenylbutan-1-one dihydrochloride; |
| [28] | 3-amino-2-benzyl-1-(4-(dimethylamino)-4-phenylpiperidin-1-yl)propan-1-one dihydrochloride; |
| [29] | 1-(4-(dimethylamino)-4-phenylpiperidin-1-yl)-3-(methylamino)-2-phenylpropan-1-one; |
| [30] | 2-benzyl-1-(4-(dimethylamino)-4-phenylpiperidin-1-yl)-3-(ethylamino)propan-1-one dihydrochloride; |
| [31] | 2-benzyl-1-(4-(dimethylamino)-4-phenylpiperidin-1-yl)-4-(methylamino)butan-1-one dihydrochloride; |
| [32] | 1-(4-(dimethylamino)-4-phenylpiperidin-1-yl)-2-methyl-3-(methylamino)-2-phenylpropan-1-one dihydrochloride; |
| [33] | 3-amino-1-(4-(dimethylamino)-4-phenylpiperidin-1-yl)-2-methyl-2-phenylpropan-1-one dihydrochloride; |
| [34] | 2-benzyl-1-(4-(dimethylamino)-4-(3-hydroxyphenyl)piperidin-1-yl)-3-(methylamino)propan-1-one dihydrochloride; |
| [35] | (4-(dimethylamino)-4-(3-hydroxyphenyl)piperidin-1-yl)(1,2,3,4-tetrahydroisoquinolin-1-yl)methanone dihydrochloride; |
| [36] | 2-benzyl-1-(4-(dimethylamino)-4-(pyridin-2-yl)piperidin-1-yl)-3-(methylamino)propan-1-one dihydrochloride; |
| [37] | 2-benzyl-1-(4-((dimethylamino)methyl)-4-phenylpiperidin-1-yl)-3-(methylamino)propan-1-one dihydrochloride; |
| [38] | (4-(dimethylamino)-4-(3-hydroxyphenyl)piperidin-1-yl)(1,2,3,4-tetrahydroisoquinolin-4-yl)methanone; |
| [39] | 1-(4-(dimethylamino)-4-phenylpiperidin-1-yl)-2-(3-methoxybenzyl)-3-(methylamino)propan-1-one dihydrochloride; |
| [40] | 3-(3-(4-(dimethylamino)-4-phenylpiperidin-1-yl)-2-((methylamino)methyl)-3-oxopropyl)benzonitrile dihydrochloride; |
| [41] | 2-benzyl-1-(4-(dimethylamino)-4-(3-methoxyphenyl)piperidin-1-yl)-3-(methylamino)propan-1-one dihydrochloride; |
| [42] | 3-amino-2-benzyl-1-(4-(dimethylamino)-4-phenylpiperidin-1-yl)-2-methylpropan-1-one dihydrochloride; |
| [43] | 2-benzyl-1-(4-(dimethylamino)-4-phenylpiperidin-1-yl)-2-methyl-3-(methylamino)propan-1-one; |
| [44] | 2-benzyl-1-(4-(3-hydroxyphenyl)-4-(methylamino)piperidin-1-yl)-3-(methylamino)propan-1-one dihydrochloride; |
| [45] | (4-(dimethylamino)-4-phenylpiperidin-1-yl)(2-((methylamino)methyl)-2,3-dihydro-1*H*-inden-2-yl)methanone dihydrochloride; |
| [46] | (*R*)-(4-(dimethylamino)-4-phenylpiperidin-1-yl)(1,2,3,4-tetrahydroisoquinolin-3-yl)methanone dihydrochloride; |
| [47] | (*S*)-(4-(dimethylamino)-4-phenylpiperidin-1-yl)(1,2,3,4-tetrahydroisoquinolin-3-yl)methanone dihydrochloride; |
| [48] | (2*S*,3*S*)-3-amino-2-benzyl-1-(4-(dimethylamino)-4-phenylpiperidin-1-yl)butan-1-one dihydrochloride; |
| [49] | (2*R*,3*R*)-3-amino-2-benzyl-1-(4-(dimethylamino)-4-phenylpiperidin-1-yl)butan-1-one dihydrochloride; |
| [50] | 1-(4-(dimethylamino)-4-phenylpiperidin-1-yl)-2-((methylamino)methyl)-4-phenylbutan-1-one dihydrochloride; |
| [51] | 1-(4-(dimethylamino)-4-phenylpiperidin-1-yl)-2-(4-fluorobenzyl)-3-(methylamino)propan-1-one; |
| [52] | 1-(4-(dimethylamino)-4-phenylpiperidin-1-yl)-2-(3-fluorobenzyl)-3-(methylamino)propan-1-one; |
| [53] | 2-benzyl-3-(methylamino)-1-(4-(methylamino)-4-phenylpiperidin-1-yl)propan-1-one; |
| [54] | 1-(4-(dimethylamino)-4-phenylpiperidin-1-yl)-2-(4-methoxybenzyl)-3-(methylamino)propan-1-one; |
| [55] | 2-(3-(4-(dimethylamino)-4-phenylpiperidin-1-yl)-2-((methylamino)methyl)-3-oxopropyl)benzonitrile dihydrochloride; |
| [56] | 4-(3-(4-(dimethylamino)-4-phenylpiperidin-1-yl)-2-((methylamino)methyl)-3-oxopropyl)benzonitrile dihydrochloride; |
| [57] | 1-(4-(dimethylamino)-4-phenylpiperidin-1-yl)-2-(2-fluorobenzyl)-3-(methylamino)propan-1-one dihydrochloride; |
| [58] | *trans*-(4-(dimethylamino)-4-phenylpiperidin-1-yl)(4-phenylpyrrolidin-3-yl)methanone dihydrochloride; |
| [59] | *cis*-(4-(dimethylamino)-4-phenylpiperidin-1-yl)(4-phenylpyrrolidin-3-yl)methanone dihydrochloride; |
| [60] | *trans*-(4-benzylpyrrolidin-3-yl)(4-(dimethylamino)-4-phenylpiperidin-1-yl)methanone; |
| [61] | (4-(dimethylamino)-4-phenylpiperidin-1-yl)(2,3,4,5-tetrahydro-1*H-*benzo[c]azepin-4-yl)methanone dihydrochloride; |
| [62] | *trans*/*trans*-(2-amino-5-phenylcyclopentyl)(4-(dimethylamino)-4-phenylpiperidin-1-yl)methanone; |
| [63] | ((1*RS*,2*SR*,5*SR*)-2-amino-5-phenylcyclopentyl)(4-(dimethylamino)-4-phenylpiperidin-1-yl)methanone; |
| [64+ | *trans*/*trans*-(4-(dimethylamino)-4-phenylpiperidin-1-yl)(2-(methylamino)-5-phenylcyclopentyl)methanone dihydrochloride; |
| [65] | (2*S*,3*R*)-1-(4-(4-fluorophenyl)-4-(methylamino)piperidin-1-yl)-2-((methylamino)methyl)-3-phenylpentan-1-one; |
| [66] | (2*S*,3*R*)-2-(aminomethyl)-3-(3-chlorophenyl)-1-(4-(dimethylamino)-4-phenylpiperidin-1-yl)pentan-1-one; |
| [67] | (2*S*,3*R*)-3-(3-chlorophenyl)-1-(4-(dimethylamino)-4-phenylpiperidin-1-yl)-2-((methylamino)methyl)pentan-1-one; |
| [68] | 3-amino-2-(2,3-dihydro-1 *H*-inden-1-yl)-1-(4-(dimethylamino)-4-phenylpiperidin-1-yl)propan-1-one; |
| [69] | (2*S*,3*R*)-3-(3-chlorophenyl)-1-(4-(4-fluorophenyl)-4-(methylamino)piperidin-1-yl)-2-((methylamino)methyl)pentan-1-one; |
| [70] | 2-benzyl-1-(4-(dimethylamino)-4-phenylpiperidin-1-yl)-3-(methylamino)propan-1-one; |
| [71] | 2-Benzyl-1-(4-(dimethylamino)-4-phenylpiperidin-1-yl)-3-(phenethylamino)propan-1-one; |
| [72] | 2-benzyl-1-(4-(dimethylamino)-4-phenylpiperidin-1-yl)-3-(isopropylamino)propan-1-one; |
| [73] | 2-benzyl-3-(dimethylamino)-1-(4-(dimethylamino)-4-phenylpiperidin-1-yl)propan-1-one; |
| [74] | *trans*-(1,4-dibenzylpyrrolidin-3-yl)(4-(dimethylamino)-4-phenylpiperidin-1-yl)methanone; |
| [75] | 3-amino-1-(4-(dimethylamino)-4-phenylpiperidin-1-yl)-2-(2-methylbenzyl)propan-1-one dihydrochloride; |
| [76] | 2-benzyl-3-(benzylamino)-1-(4-(dimethylamino)-4-phenylpiperidin-1-yl)propan-1-one dihydrochloride; |
| [77] | (*RS*)-3-amino-1-(4-(dimethylamino)-4-phenylpiperidin-1-yl)-2-((SR)-1,2,3,4-tetrahydronaphthalen-1-yl)propan-1-one; |
| [78] | (*RS*)-3-amino-1-(4-(dimethylamino)-4-phenylpiperidin-1-yl)-2-((*RS*)-1,2,3,4-tetrahydronaphthalen-1-yl)propan-1-one; |
| [79] | (*S*)-2-benzyl-1-(4-(dimethylamino)-4-phenylpiperidin-1-yl)-3-(methylamino)propan-1-one dihydrochloride; |
| [80] | (*R*)-2-benzyl-1-(4-(dimethylamino)-4-phenylpiperidin-1-yl)-3-2(methylamino)propan-1-one dihydrochloride; |
| [81] | (*R*)-3-amino-2-((*S*)-2,3-dihydro-1 *H*-inden-1-yl)-1-(4-(dimethylamino)-4-phenylpiperidin-1-yl)propan-1-one; |
| [82] | (*S*)-3-amino-2-((*R*)-2,3-dihydro-1 *H*-inden-1-yl)-1-(4-(dimethylamino)-4-phenylpiperidin-1-yl)propan-1-one; |
| [83] | (*R*)-3-amino-2-((*R*)-2,3-dihydro-1 *H*-inden-1-yl)-1-(4-(dimethylamino)-4-phenylpiperidin-1-yl)propan-1-one; |
| [84] | (*S*)-3-amino-2-((*S*)-2,3-dihydro-1 *H*-inden-1-yl)-1-(4-(dimethylamino)-4-phenylpiperidin-1-yl)propan-1-one; |
| [85] | (2*S*,3*S*)-1-(4-(dimethylamino)-4-phenylpiperidin-1-yl)-2-((methylamino)methyl)-3-phenylbutan-1-one; |
| [86] | (2*R*,3*R*)-1-(4-(dimethylamino)-4-phenylpiperidin-1-yl)-2-((methylamino)methyl)-3-phenylbutan-1-one; and |
| [87] | (2*R*,3*S*)-1-(4-(dimethylamino)-4-phenylpiperidin-1-yl)-2-((methylamino)methyl)-3-phenylbutan-1-one. |

10. Process for the preparation of a compound of formula (I) according to claim 1: 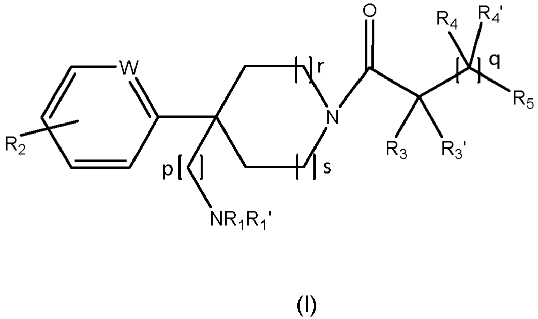 said process comprising:
treating a compound of formula (II): 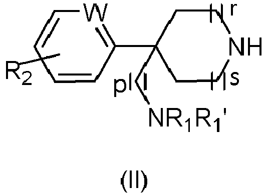 with a compound of formula (III): 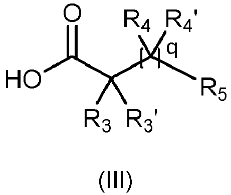 wherein R₁, R₁', R₂, R₃, R₃', R₄, R₄', R₅, W, p, q, r and s have the same meaning as indicated in any one of claims 1 to 8.

11. Use of a compound selected from 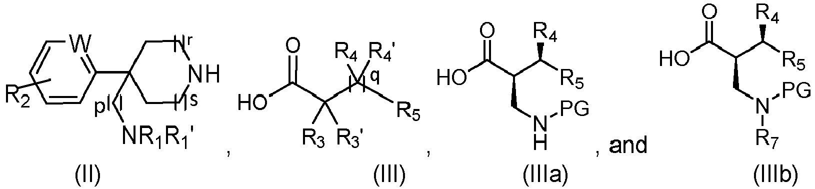 wherein R₁, R₁', R₂, R₃, R₃', R₄, R₄', R₅, W, p, q, r and s have the same meanings as indicated in any one of claims 1 to 8 and PG represents a protecting group, for the manufacture of a compound according to claim 1.

12. Compound according to any one of claims 1 to 9 for use as a medicament.

13. Compound according to any one of claims 1 to 9, for use in the treatment and/or prophylaxis of diseases and/or disorders mediated by the subunit α2δ, especially the α2δ-1 subunit of voltage-gated calcium channels and the µ-opioid receptor.

14. Compound for use according to claim 13, where the disease or disorder is pain, especially neuropathic pain, inflammatory pain, and chronic pain or other pain conditions involving allodynia and/or hyperalgesia, depression, anxiety and attention-deficit-/hyperactivity disorder.

15. A pharmaceutical composition comprising a compound of general formula (I) according to any one of claims 1 to 9 or a pharmaceutically acceptable salt, isomer, prodrug or solvate thereof, and at least a pharmaceutically acceptable carrier, additive, adjuvant or vehicle.
